# EUROPÄISCHE PATENTANMELDUNG

(11) **EP 1 721 522 A2**
(43) Veröffentlichungstag der Anmeldung: **15.11.2006**
(21) Anmeldenummer: 05026674.1
(22) Anmeldetag: 21.03.1997
(51) Int. Cl.: A01N 43/16, C07D 309/38, C07C 57/36, C07C 59/84, C07C 69/612, C07D 311/74, C07D 405/04, C07D 405/14, C07D 493/04

(54) **Neue substituierte Phenylketoenole**

(30) Priorität: 02.04.1996 DE 19613171; 29.11.1996 DE 19649665
(62) Teilanmeldung aus: 97915409.3
(71) Anmelder: Bayer CropScience AG, 40789 Monheim (DE)
(72) Erfinder: Lieb, Folker, 51375 Leverkusen (DE); Hagemann, Hermann, 51375 Leverkusen (DE); Widdig, Arno, 51519 Odenthal (DE); Ruther, Michael, 40764 Langenfeld (DE); Fischer, Reiner, 40789 Monheim (DE); Brechtschneider, Thomas, 53797 Lohmar (DE); Erdelen, Christoph, Deceased (DE); Wachedorff-Neumann, Ulrike, 56566 Neuwied (DE); Graff, Alan, 79539 Lörrach (DE); Schneider, Udo, 51373 Leverkusen (DE)

(57) **Zusammenfassung**

Die vorliegende Erfindung betrifft neue Verbindungen der Formel (I) 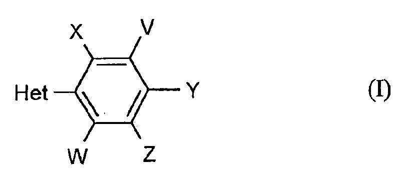 in welcher
Het
für eine der Gruppen 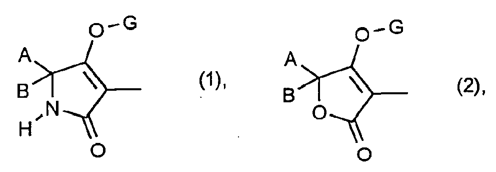 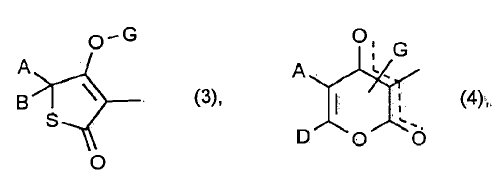 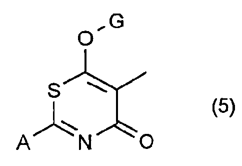 steht,
worin
A, B, D, G, V, W, X, Y und Z die in der Beschreibung angegebenen Bedeutungen haben,

Verfahren und Zwischenprodukte zu ihrer Herstellung und ihre Verwendung als Schädlingsbekämpfungsmittel und Herbizide.

## Beschreibung

Die Erfindung betrifft neue phenylsubstituierte cyclische Ketoenole, mehrere Verfahren und Zwischenprodukte zu ihrer Herstellung und ihre Verwendung als Schädlingsbekämpfungsmittel und Herbizide.

Es ist bereits bekannt geworden, daß bestimmte phenylsubstituierte cyclische Ketoenole als Insektizide, Akarizide und/oder Herbizide wirksam sind.

Von 3-Acyl-pyrrolidin-2,4-dionen sind pharmazeutische Eigenschaften vorbeschrieben (S. Suzuki et al. Chem. Pharm. Bull. 15 1120 (1967)). Weiterhin wurden N-Phenylpyrrolidin-2,4-dione von R. Schmierer und H. Mildenberger (Liebigs Ann. Chem. 1985, 1095) synthetisiert. Eine biologische Wirksamkeit dieser Verbindungen wurde nicht beschrieben.

In EP-A-0 262 399 und GB-A-2 266 888 werden ähnlich strukturierte Verbindungen (3-Aryl-pyrrolidin-2,4-dione) offenbart, von denen jedoch keine herbizide, insektizide oder akarizide Wirkung bekannt geworden ist. Bekannt mit herbizider, insektizider oder akarizider Wirkung sind unsubstituierte, bicyclische 3-Aryl-pyrrolidin-2,4-dion-Derivate (EP-A-355 599 und EP-A-415 211) sowie substituierte monocyclische 3-Aryl-pyrrolidin-2,4-dion-Derivate (EP-A-377 893 und EP-A-442 077).

Weiterhin bekannt sind polycyclische 3-Arylpyrrolidin-2,4-dion-Derivate (EP-A-442 073) sowie 1H-Arylpyrrolidin-dion-Derivate (EP-A-456 063, EP-A-521 334, EP-A-596 298, EP-A-613 884, EP-A-613 885, DE 44 40 594, WO 94/01 997, WO 95/01 358, WO 95/20 572, EP-A-668 267 und WO 95/26 954.

Es ist bekannt, daß bestimmte substituierte Δ³-Dihydrofuran-2-on-Derivate herbizide Eigenschaften besitzen (vgl. DE-A-4 014 420). Die Synthese der als Ausgangsverbindungen verwendeten Tetronsäurederivate (wie z.B. 3-(2-Methyl-phenyl)-4-hydroxy-5-(4-fluorphenyl)-Δ³-dihydrofuranon-(2)) ist ebenfalls in DE-A-4 014 420 beschrieben. Ähnlich strukturierte Verbindungen ohne Angabe einer insektiziden und/oder akariziden Wirksamkeit sind aus der Publikation Campbell et al., J. Chem. Soc., Perkin Trans. 1, 1985, (8) 1567-76 bekannt. Weiterhin sind 3-Aryl-Δ³-dihydrofuranon-Derivate mit herbiziden, akariziden und insektiziden Eigenschaften aus EP-A-528 156 und EP-A-0 647 637 bekannt. Auch 3-Aryl-Δ³-dihydrothiophen-on-Derivate sind bekannt (WO 95/26 345).

Bestimmte, im Phenylring unsubstituierte Phenyl-pyron-Derivate sind bereits bekannt geworden (vgl. A.M. Chirazi, T. Kappe und E. Ziegler, Arch. Pharm. 309, 558 (1976) und K.-H. Boltze und K. Heidenbluth, Chem. Ber. 91, 2849), wobei für diese Verbindungen eine mögliche Verwendbarkeit als Schädlingsbekämpfungsmittel nicht angegeben wird. Im Phenylring substituierte Phenyl-pyron-Derivate mit herbiziden, akariziden und insektiziden Eigenschaften sind in EP-A-588 137 beschrieben.

Bestimmte, im Phenylring unsubstituierte 5-Phenyl-1,3-thiazin-Derivate sind bereits bekannt geworden (vgl. E. Ziegler und E. Steiner, Monatsh. 95, 147 (1964), R. Ketcham, T. Kappe und E. Ziegler, J. Heterocycl. Chem. 10, 223 (1973)), wobei für diese Verbindungen eine mögliche Anwendung als Schädlingsbekämpfungsmittel nicht angegeben wird. Im Phenylring substituierte 5-Phenyl-1,3-thiazin-Derivate mit herbizider, akarizider und insektizider Wirkung sind in WO 94/14 785 beschrieben.

Die akarizide und insektizide Wirksamkeit und/oder Wirkungsbreite, und/oder die Pflanzenverträglichkeit dieser Verbindungen, insbesondere gegenüber Kulturpflanzen, ist jedoch nicht immer ausreichend.

Es wurden nun neue Verbindungen der Formel (I) 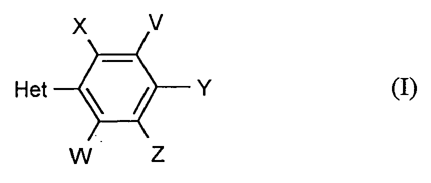 gefunden,
in welcher
- V: für Wasserstoff, Halogen, Alkyl oder Alkoxy steht,
- W: für Cyano, Nitro, Halogen, Alkyl, Alkenyl, Alkinyl, Alkoxy, Halogenalkyl, Halogenalkoxy, jeweils gegebenenfalls substituiertes Phenyl, Phenoxy, Phenylthio, Phenylalkoxy oder Phenylalkylthio steht,
- X: für Wasserstoff, Halogen, Alkyl, Alkenyl, Alkinyl, Alkoxy, Halogenalkyl, Halogenalkoxy, Cyano, Nitro oder für jeweils gegebenenfalls substituiertes Phenyl, Phenoxy, Phenylthio, Phenylalkyloxy oder Phenylalkylthio steht,
- Y: für Wasserstoff, Halogen, Alkyl, Alkoxy, Halogenalkyl, Halogenalkoxy, Cyano oder Nitro steht,
- Z: für Halogen, Alkyl, Alkoxy, Halogenalkyl, Halogenalkoxy, Hydroxy, Cyano, Nitro oder jeweils gegebenenfalls substituiertes Phenoxy, Phenylthio, 5- bis 6-gliedriges Hetaryloxy, 5- bis 6-gliedriges Hetarylthio, Phenylalkyloxy oder Phenylalkylthio steht oder
- Y und Z: gemeinsam mit den Kohlenstoffatomen, an die sie gebunden sind, für einen gegebenenfalls substituierten und gegebenenfalls durch ein oder mehrere Heteroatome unterbrochenen Cyclus stehen, wobei V, X und W eine der obengenannten Bedeutungen haben oder
- W und Z: gemeinsam mit den Kohlenstoffatomen, an die sie gebunden sind, einen gegebenenfalls substituierten und gegebenenfalls durch ein oder mehrere Heteroatome unterbrochenen Cyclus stehen, wobei V, X und Y eine der obengenannten Bedeutungen haben,
- Het: für eine der Gruppen 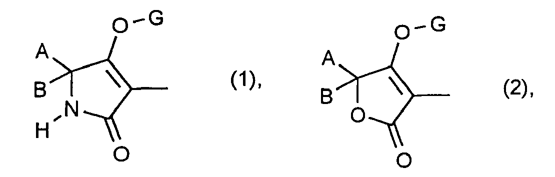 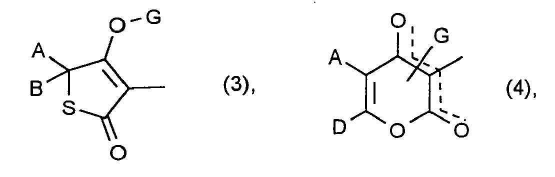 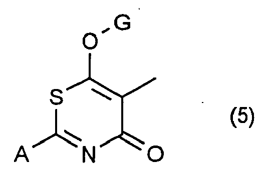 steht,
worin
- A: für Wasserstoff, für jeweils gegebenenfalls durch Halogen substituiertes Alkyl, Alkenyl, Alkoxyalkyl, Polyalkoxyalkyl oder Alkylthioalkyl, für jeweils gesättigtes oder ungesättigtes und gegebenenfalls substituiertes Cycloalkyl oder Heterocyclyl oder für jeweils gegebenenfalls durch Halogen, Alkyl, Halogenalkyl, Alkoxy, Halogenalkoxy, Cyano oder Nitro substituiertes Aryl, Arylalkyl oder Hetaryl steht,
- B: für Wasserstoff, Alkyl oder Alkoxyalkyl steht, oder
- A und B: gemeinsam mit dem Kohlenstoffatom an das sie gebunden sind, für einen gesättigten oder ungesättigten, gegebenenfalls substituierten Carbocyclus oder Heterocyclus stehen,
- D: für Wasserstoff oder für einen gegebenenfalls substituierten Rest aus der Reihe Alkyl, Alkenyl, Alkinyl, Alkoxyalkyl, Polyalkoxyalkyl, Alkylthioalkyl, gesättigtes oder ungesättigtes Cycloalkyl, gesättigtes oder ungesättigtes Heterocyclyl, Arylalkyl, Aryl, Hetarylalkyl oder Hetaryl steht oder
- A und D: gemeinsam mit den Atomen an die sie gebunden sind für einen jeweils gegebenenfalls substituierten Carbocyclus oder Heterocyclus stehen,
- G: für Wasserstoff (a) oder für eine der Gruppen 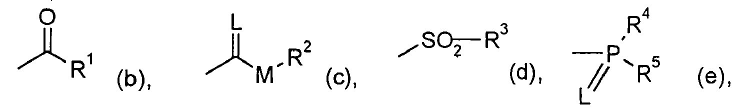 E (f) oder 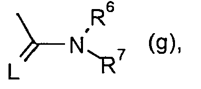 steht,
worin
E für ein Metallionäquivalent oder ein Ammoniumion steht,
L für Sauerstoff oder Schwefel steht,
M für Sauerstoff oder Schwefel steht,
- R¹: für jeweils gegebenenfalls durch Halogen substituiertes Alkyl, Alkenyl, Alkoxyalkyl, Alkylthioalkyl oder Polyalkoxyalkyl oder für jeweils gegebenenfalls durch Halogen, Alkyl oder Alkoxy substituiertes Cycloalkyl oder Heterocyclyl oder für jeweils gegebenenfalls substituiertes Phenyl, Phenylalkyl, Hetaryl, Phenoxyalkyl oder Hetaryloxyalkyl steht,
- R²: für jeweils gegebenenfalls durch Halogen substituiertes Alkyl, Alkenyl, Alkoxyalkyl oder Polyalkoxyalkyl oder für jeweils gegebenenfalls substituiertes Cycloalkyl, Phenyl oder Benzyl steht,
- R³, R⁴ und R⁵: unabhängig voneinander für jeweils gegebenenfalls durch Halogen substituiertes Alkyl, Alkoxy, Alkylamino, Dialkylamino, Alkylthio, Alkenylthio oder Cycloalkylthio oder für jeweils gegebenenfalls substituiertes Phenyl, Benzyl, Phenoxy oder Phenylthio stehen,
- R⁶ und R⁷: unabhängig voneinander für Wasserstoff, für jeweils gegebenenfalls durch Halogen substituiertes Alkyl, Cycloalkyl, Alkenyl, Alkoxy oder Alkoxyalkyl oder für jeweils gegebenenfalls substituiertes Phenyl oder Benzyl stehen, oder gemeinsam mit dem N-Atom, an das sie gebunden sind, einen gegebenenfalls Sauerstoff oder Schwefel enthaltenden und gegebenenfalls substituierten Cyclus bilden, mit Ausnahme folgender Verbindungen

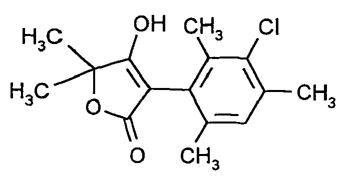

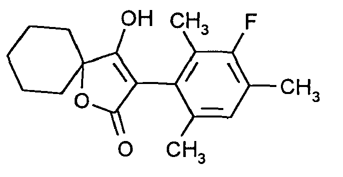

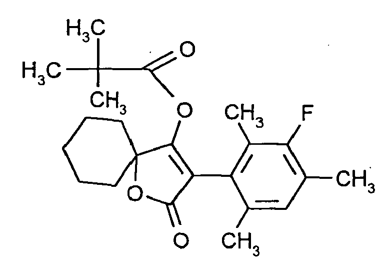

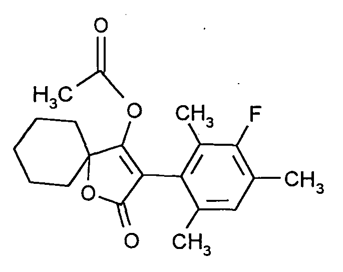

Die Verbindungen der Formel (I) können, auch in Abhängigkeit von der Art der Substituenten, als geometrische und/oder optische Isomere oder Isomerengemische, in unterschiedlicher Zusammensetzung vorliegen, die gegebenenfalls in üblicher Art und Weise getrennt werden können. Sowohl die reinen Isomeren als auch die Isomerengemische, deren Herstellung und Verwendung sowie diese enthaltende Mittel sind Gegenstand der vorliegenden Erfindung. Im folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sind.

Unter Einbeziehung der Bedeutungen (1) bis (5) der Gruppe Het ergeben sich folgende hauptsächliche Strukturen (I-1) bis (I-5): 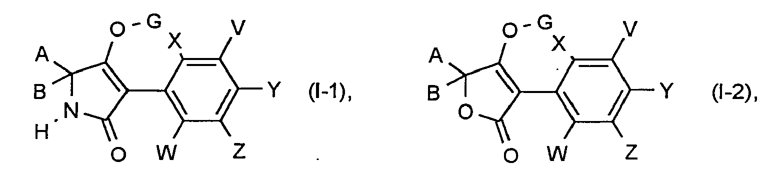 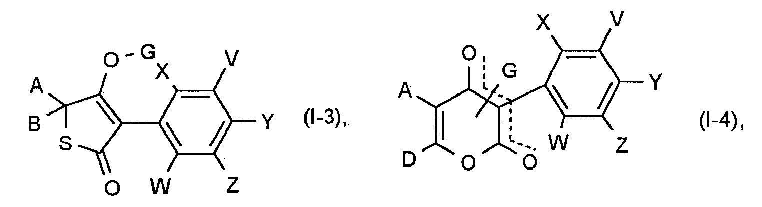 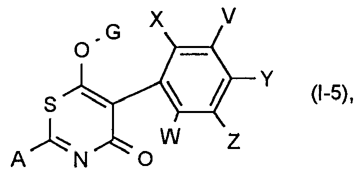 worin
A, B, D, G, V, W, X, Y und Z die oben angegebene Bedeutung haben.

Unter Einbeziehung der verschiedenen Bedeutungen (a), (b), (c), (d), (e), (f) und (g) der Gruppe G ergeben sich folgende hauptsächliche Strukturen (I-1-a) bis (I-1-g), wenn Het für die Gruppe (1) steht, 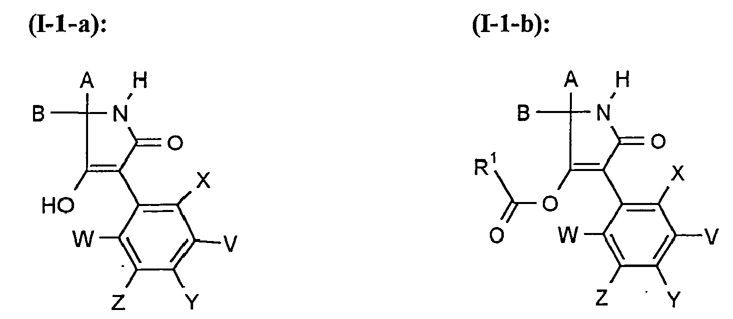 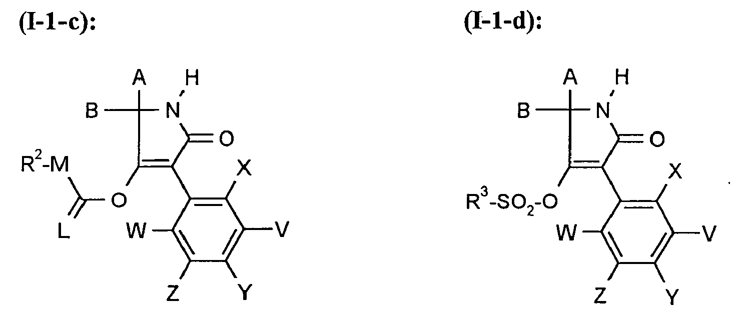 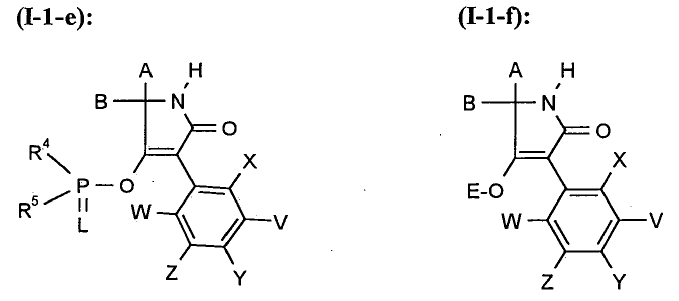 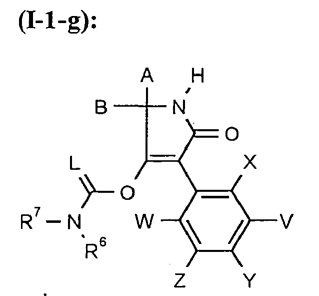 worin
A, B, E, L, M, V, W, X, Y, Z, R¹, R², R³, R⁴, R⁵, R⁶ und R⁷ die oben angegebenen Bedeutungen besitzen.

Unter Einbeziehung der verschiedenen Bedeutungen (a), (b), (c), (d), (e), (f) und (g) der Gruppe G ergeben sich folgende hauptsächliche Strukturen (I-2-a) bis (I-2-g), wenn Het für die Gruppe (2) steht, 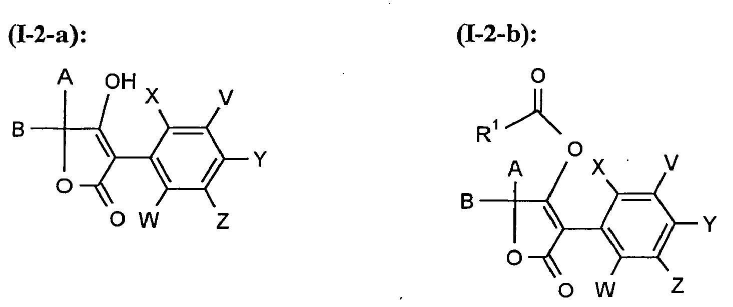 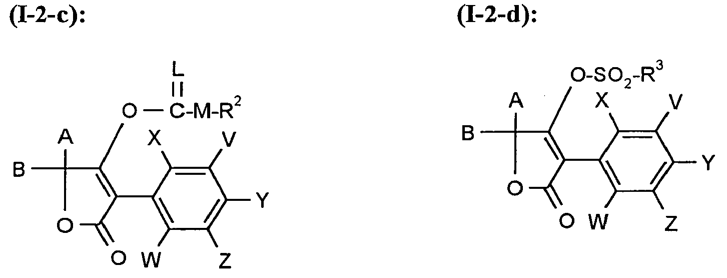 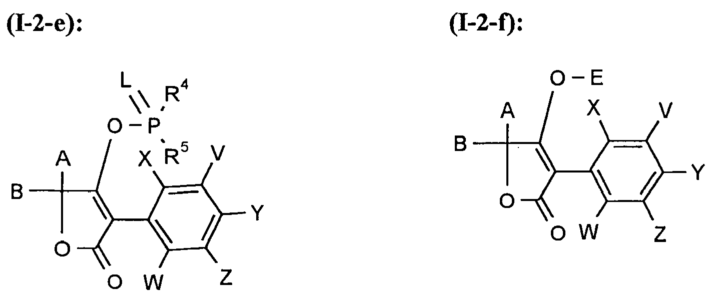 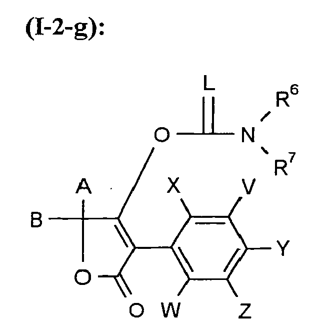 worin
A, B, E, L, M, V, W, X, Y, Z, R¹, R², R³, R⁴, R⁵, R⁶ und R⁷ die oben angegebene Bedeutung haben.

Unter Einbeziehung der verschiedenen Bedeutungen (a), (b), (c), (d), (e), (f) und (g) der Gruppe G ergeben sich folgende hauptsächliche Strukturen (I-3-a) bis (I-3-g), wenn Het für die Gruppe (3) steht, 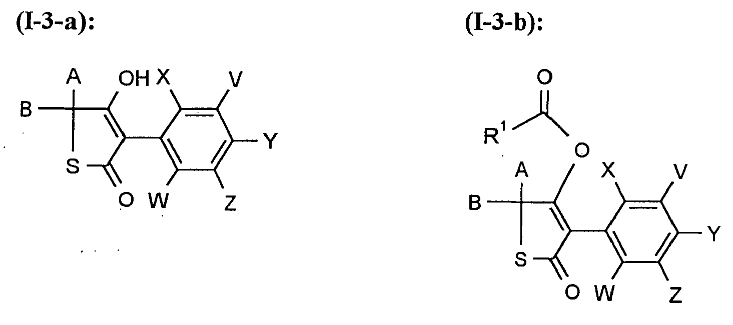 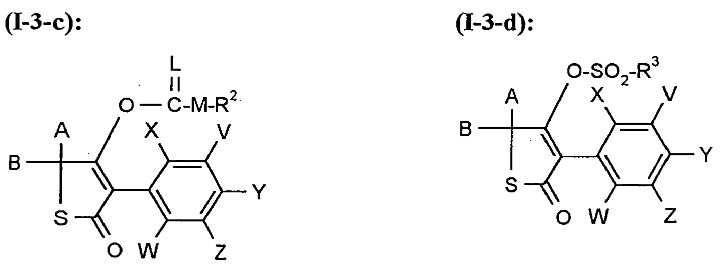 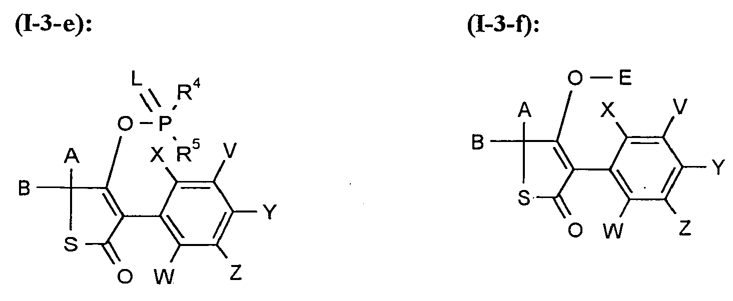 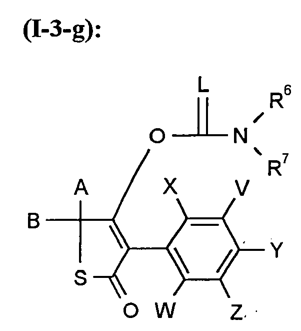 worin
A, B, E, L, M, V, W, X, Y, Z, R¹, R², R³, R⁴, R⁵, R⁶ und R⁷ die oben angegebenen Bedeutung besitzen.

Die Verbindungen der Formel (I-4) können in Abhängigkeit von der Stellung des Substituenten G in den zwei isomeren Formen der Formeln (I-4)ₐ und (I-4)_{b} vorliegen, 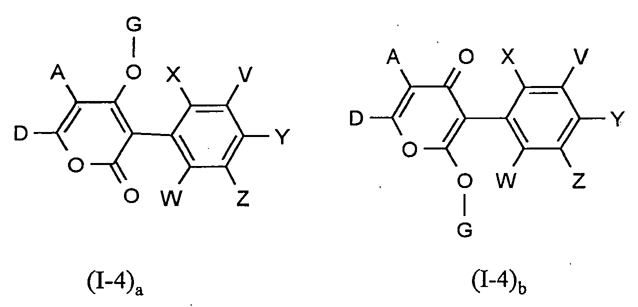 was durch die gestrichelte Linie in der Formel (I-4) zum Ausdruck gebracht werden soll.

Die Verbindungen der Formeln (I-4)ₐ und (1-4)_{b} können sowohl als Gemische als auch in Form ihrer reinen Isomeren vorliegen. Gemische der Verbindungen der Formeln (I-4)ₐ und (I-4)_{b} lassen sich gegebenenfalls in an sich bekannter Weise durch physikalische Methoden trennen, beispielsweise durch chromatographische Methoden.

Aus Gründen der besseren Übersichtlichkeit wird im folgenden jeweils nur eines der möglichen Isomeren aufgeführt. Das schließt nicht aus, daß die Verbindungen gegebenenfalls in Form der Isomerengemische oder in der jeweils anderen isomeren Form vorliegen können.

Unter Einbeziehung der verschiedenen Bedeutungen (a), (b), (c), (d), (e), (f) und (g) der Gruppe G ergeben sich folgende hauptsächliche Strukturen (I-4-a) bis (I-4-g), wenn Het für die Gruppe (4) steht, 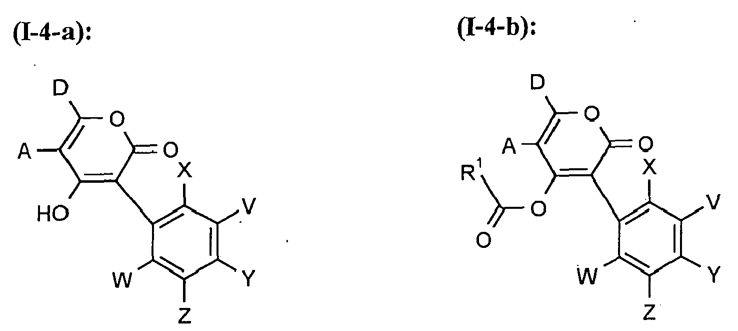 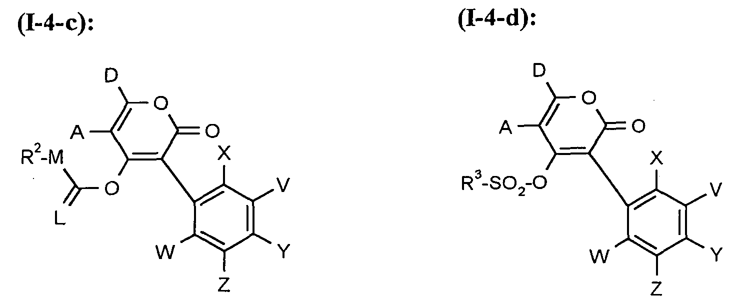 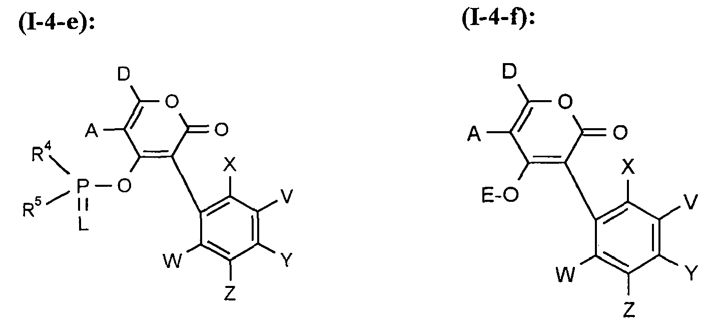 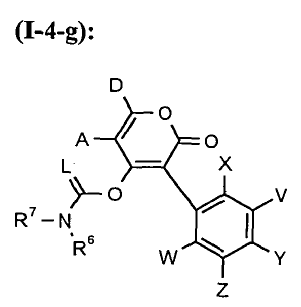 worin
A, D, E, L, M, V, W, X, Y, Z, R¹, R², R³, R⁴, R⁵, R⁶ und R⁷ die oben angegebenen Bedeutungen besitzen.

Unter Einbeziehung der verschiedenen Bedeutungen (a), (b), (c), (d), (e), (f) und (g) der Gruppe G ergeben sich folgende hauptsächliche Strukturen (I-5-a) bis (I-5-g), wenn Het für die Gruppe (5) steht, 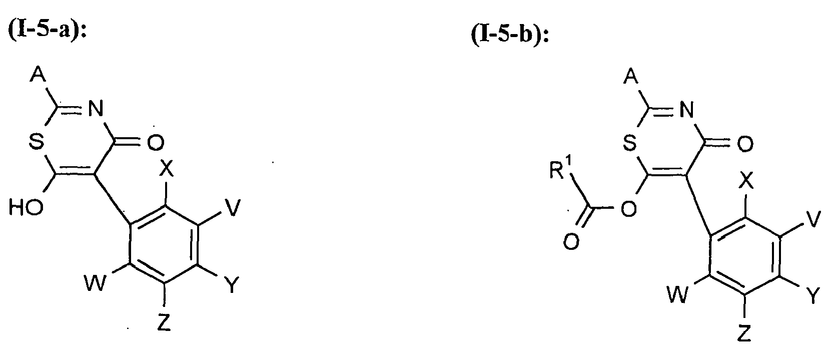 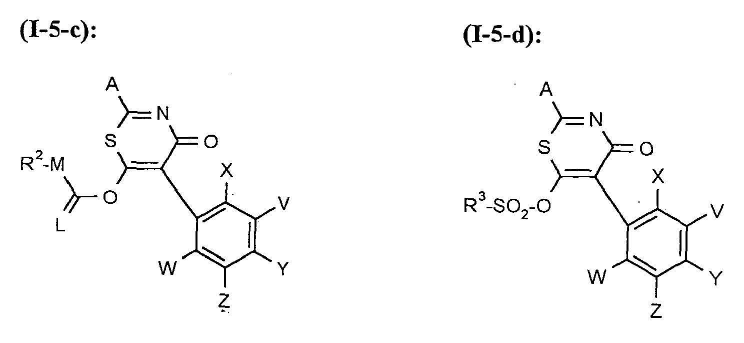 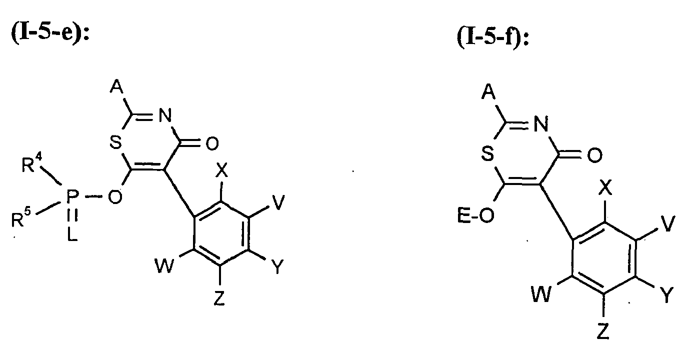 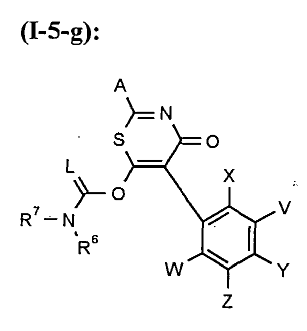 worin
A, E, L, M, V, W, X, Y, Z, R¹, R², R³, R⁴, R⁵, R⁶ und R⁷ die oben angegebenen Bedeutungen besitzen.

Weiterhin wurde gefunden, daß man die neuen Verbindungen der Formel (I) nach einem der im folgenden beschriebenen Verfahren erhält:
(A) Man erhält Verbindungen der Formel (I-1-a) 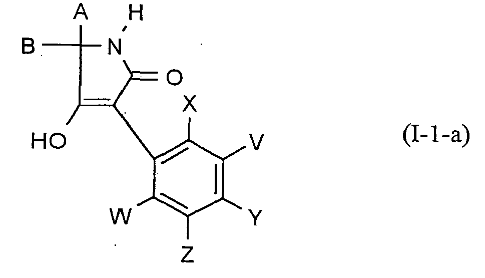 in welcher
   A, B, V, W, X, Y und Z die oben angegebenen Bedeutungen haben,
   wenn man
   Verbindungen der Formel (II) 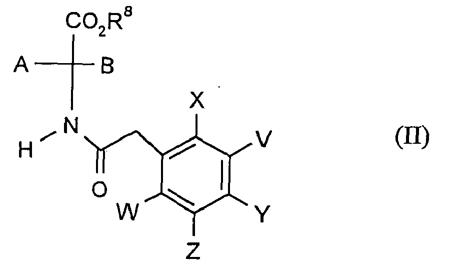 in welcher
   A, B, V, W, X, Y und Z die oben angegebenen Bedeutungen haben,
   und
   - R⁸: für Alkyl (bevorzugt C₁-C₆-Alkyl) steht,
   in Gegenwart eines Verdünnungsmittels und in Gegenwart einer Base intramolekular kondensiert.
(B) Außerdem wurde gefunden, daß man Verbindungen der Formel (I-2-a) 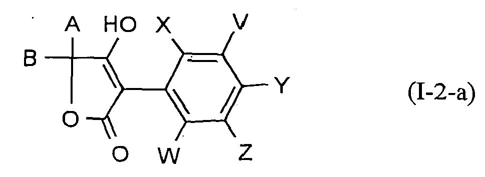 in welcher
   A, B, V, W, X, Y und Z die oben angegebenen Bedeutungen haben,
   erhält, wenn man
   Verbindungen der Formel (III) 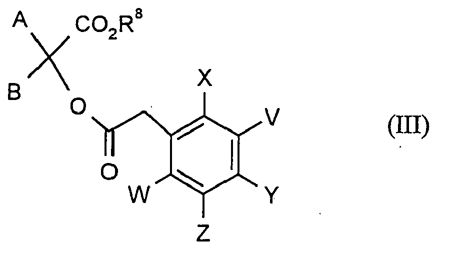 in welcher
   A, B, V, W, X, Y, Z und R⁸ die oben angegebenen Bedeutungen haben,
   in Gegenwart eines Verdünnungsmittels und in Gegenwart einer Base intramolekular kondensiert.
(C) Weiterhin wurde gefunden, daß man Verbindungen der Formel (I-3-a) 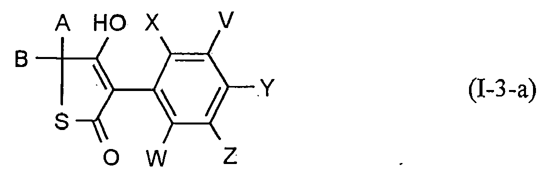 in welcher
   A, B, V, W, X, Y und Z die oben angegebenen Bedeutungen haben,
   erhält, wenn man
   Verbindungen der Formel (IV) 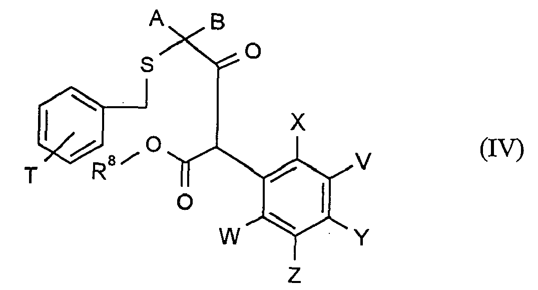 in welcher
   A, B, V, W, X, Y, Z und R⁸ die oben angegebenen Bedeutungen haben und
   - T: für Wasserstoff, Halogen, Alkyl (bevorzugt C₁-C₆-Alkyl) oder Alkoxy (bevorzugt C₁-C₈-Alkoxy) steht,
   gegebenenfalls in Gegenwart eines Verdünnungsmittels und in Gegenwart einer Säure intramolekular cyclisiert.
(D) Weiterhin wurde gefunden, daß man die Verbindungen der Formel (I-4-a) 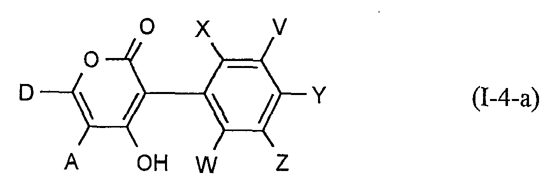 in welcher
   A, D, V, W, X, Y und Z die oben angegebenen Bedeutungen haben,
   erhält, wenn man
   Verbindungen der Formel (V) 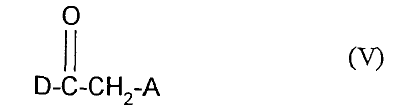 in welcher
   - A und D: die oben angegebenen Bedeutungen haben,
   oder deren Silylenolether der Formel (Va) 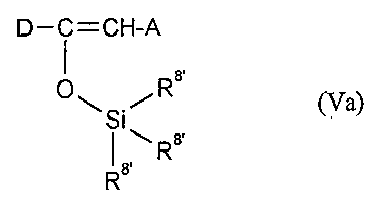 in welcher
   A und D die obengenannte Bedeutung haben und
   R^{8'} für Alkyl (bevorzugt Methyl) steht, mit Verbindungen der Formel (VI) 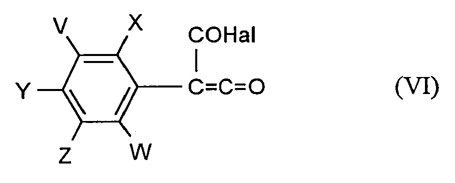 in welcher
   V, W, X, Y und Z die oben angegebenen Bedeutungen haben und
   Hal für Halogen (vorzugsweise für Chlor oder Brom) steht,
   gegebenenfalls in Gegenwart eines Verdünnungsmittels und gegebenenfalls in Gegenwart eines Säureakzeptors umsetzt.
(E) Weiterhin wurde gefunden, daß man die Verbindungen der Formel (I-5-a) 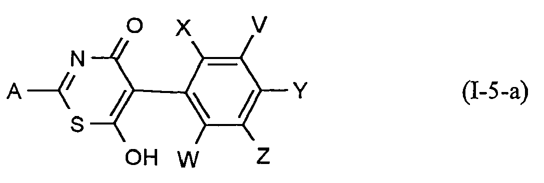 in welcher
   - A, V, W, X, Y und Z: die oben angegebenen Bedeutungen haben,
   erhält, wenn man Verbindungen der Formel (VII) 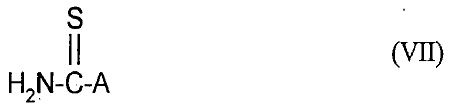 in welcher
   - A: die oben angegebene Bedeutung hat,
   mit Verbindungen der Formel (VI) 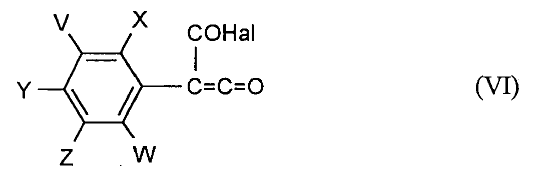 in welcher
   - Hal, V, W, X, Y und Z: die oben angegebenen Bedeutungen haben,
   gegebenenfalls in Gegenwart eines Verdünnungsmittels und gegebenenfalls in Gegenwart eines Säureakzeptors umsetzt.
   Außerdem wurde gefunden,
(F) daß man die Verbindungen der oben gezeigten Formeln (I-1-b) bis (I-5-b), in welchen A, B, D, R¹, V, W, X, Y und Z die oben angebenen Bedeutungen haben, erhält, wenn man Verbindungen der oben gezeigten Formeln (I-1-a) bis (I-5-a), in welchen A, B, D, V, W, X, Y und Z die oben angegebenen Bedeutungen haben,
   α) mit Säurehalogeniden der Formel (VIII) 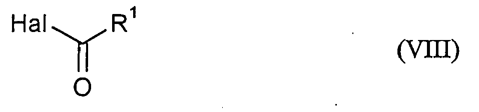 in welcher
      - R¹: die oben angegebene Bedeutung hat und
      - Hal: für Halogen (insbesondere Chlor oder Brom) steht
      oder
   β) mit Carbonsäureanhydriden der Formel (IX)

      R¹-CO-O-CO-R¹ (IX)

      in welcher
      - R¹: die oben angegebene Bedeutung hat,
      gegebenenfalls in Gegenwart eines Verdünnungsmittels und gegebenenfalls in Gegenwart eines Säurebindemittels umsetzt;
(G) daß man die Verbindungen der oben gezeigten Formeln (I-1-c) bis (1-5-c), in welchen A, B, D, R², V, W, M, X, Y und Z die oben angegebenen Bedeutungen haben und L für Sauerstoff steht, erhält, wenn man Verbindungen der oben gezeigten Formeln (I-1-a) bis (I-5-a), in welchen A, B, D, V, W, X, Y und Z die oben angegebenen Bedeutungen haben, jeweils
   mit Chlorameisensäureestern oder Chlorameisensäurethioestern der Formel (X)

   R²-M-CO-Cl (X)

   in welcher
   - R² und M: die oben angegebenen Bedeutungen haben,
   gegebenenfalls in Gegenwart eines Verdünnungsmittels und gegebenenfalls in Gegenwart eines Säurebindemittels umsetzt;
(H) daß man Verbindungen der oben gezeigten Formeln (I-1-c) bis (I-5-c), in welchen A, B, D, R², V, W, M, X, Y und Z die oben angegebenen Bedeutungen haben und L für Schwefel steht, erhält, wenn man Verbindungen der oben gezeigten Formeln (I-1-a) bis (I-5-a), in welchen A, B, D, V, W, X, Y und Z die oben angegebenen Bedeutungen haben, jeweils
   α) mit Chlormonothioameisensäureestern oder Chlordithioameisensäureestern der Formel (XI) 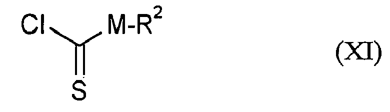 in welcher
      - M und R²: die oben angegebenen Bedeutungen haben,
      gegebenenfalls in Gegenwart eines Verdünnungsmittels und gegebenenfalls in Gegenwart eines Säurebindemittels umsetzt
      oder
   β) mit Schwefelkohlenstoff und anschließend mit Verbindungen der Formel (XII)

      R²-Hal (XII)

      in welcher
      - R²: die oben angegebene Bedeutung hat und
      - Hal: für Chlor, Brom oder Iod steht,
      gegebenenfalls in Gegenwart eines Verdünnungsmittels und gegebenenfalls in Gegenwart einer Base umsetzt,
(I) daß man Verbindungen der oben gezeigten Formeln (I-1-d) bis (I-5-d), in welchen A, B, D, R³, V, W, X, Y und Z die oben angegebenen Bedeutungen haben, erhält, wenn man Verbindungen der oben gezeigten Formeln (I-1-a) bis (I-5-a), in welchen A, B, D, V, W, X, Y und Z die oben angegebenen Bedeutungen haben, jeweils
   mit Sulfonsäurechloriden der Formel (XIII)

   R³-SO₂-Cl (XIII)

   in welcher
   - R³: die oben angegebene Bedeutung hat,
   gegebenenfalls in Gegenwart eines Verdünnungsmittels und gegebenenfalls in Gegenwart eines Säurebindemittels umsetzt,
(J) daß man Verbindungen der oben gezeigten Formeln (I-1-e) bis (I-5-e), in welchen A, B, D, L, R⁴, R⁵, V, W, X, Y und Z die oben angegebenen Bedeutungen haben, erhält, wenn man Verbindungen der oben gezeigten Formeln (I-1-a) bis (I-4-a), in welchen A, B, D, V, W, X, Y und Z die oben angegebenen Bedeutungen haben, jeweils
   mit Phosphorverbindungen der Formel (XIV) 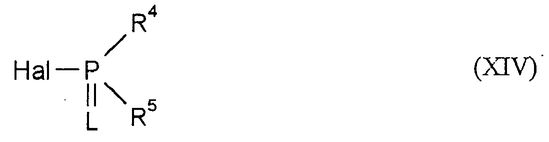 in welcher
   - L, R⁴ und R⁵: die oben angegebenen Bedeutungen haben und
   - Hal: für Halogen (insbesondere Chlor oder Brom) steht,
   gegebenenfalls in Gegenwart eines Verdünnungsmittels und gegebenenfalls in Gegenwart eines Säurebindemittels umsetzt,
(K) daß man Verbindungen der oben gezeigten Formeln (I-1-f) bis (I-5-f), in welchen A, B, D, E, V, W, X, Y und Z die oben angegebenen Bedeutungen haben, erhält, wenn man Verbindungen der Formeln (I-1-a) bis (1-5-a), in welchen A, B, D, V, W, X, Y und Z die oben angegebenen Bedeutungen haben, jeweils
   mit Metallverbindungen oder Aminen der Formeln (XV) oder (XVI) 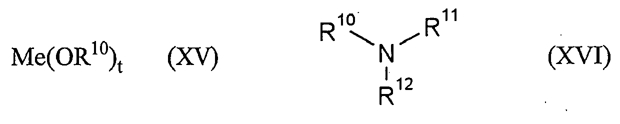 in welchen
   - Me: für ein ein- oder zweiwertiges Metall (bevorzugt ein Alkali- oder Erdalkalimetall wie Lithium, Natrium, Kalium, Magnesium oder Calcium),
   - t: für die Zahl 1 oder 2 und
   - R¹⁰, R¹¹, R¹²: unabhängig voneinander für Wasserstoff oder Alkyl (bevorzugt C₁-C₈-Alkyl) stehen,
   gegebenenfalls in Gegenwart eines Verdünnungsmittels umsetzt,
(L) daß man Verbindungen der oben gezeigten Formeln (I-1-g) bis (I-5-g), in welchen A, B, D, L, R⁶, R⁷, V, W, X, Y und Z die oben angegebenen Bedeutungen haben, erhält, wenn man Verbindungen der oben gezeigten Formeln (I-1-a) bis (I-5-a), in welchen A, B, D, V, W, X, Y und Z die oben angegebenen Bedeutungen haben, jeweils
   α) mit Isocyanaten oder Isothiocyanaten der Formel (XVII)

      R ⁶-N=C=L (XVII)

      in welcher
      - R⁶ und L: die oben angegebenen Bedeutungen haben,
      gegebenenfalls in Gegenwart eines Verdünnungsmittels und gegebenenfalls in Gegenwart eines Katalysators umsetzt oder
   β) mit Carbamidsäurechloriden oder Thiocarbamidsäurechloriden der Formel (XVIII) 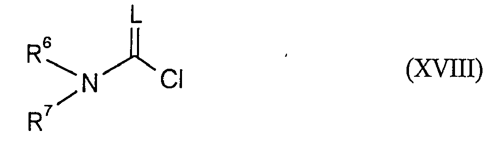 in welcher
      - L, R⁶ und R⁷: die oben angegebenen Bedeutungen haben,
      gegebenenfalls in Gegenwart eines Verdünnungsmittels und gegebenenfalls in Gegenwart eines Säurebindemittels, umsetzt.

Weiterhin wurde überraschend gefunden, daß die neuen Verbindungen der Formel (I) eine sehr gute Wirksamkeit als Schädlingsbekämpfungsmittel, vorzugsweise als Insektizide, Akarizide und als Herbizide aufweisen und darüber hinaus sehr gut pflanzenverträglich, insbesondere gegenüber Kulturpflanzen, sind.

Die erfindungsgemäßen Verbindungen sind durch die Formel (I) allgemein definiert. Bevorzugte Substituenten bzw. Bereiche der in der oben und nachstehend erwähnten Formeln aufgeführten Reste werden im folgenden erläutert:
- V: steht bevorzugt für Wasserstoff, Halogen, C₁-C₆-Alkyl oder C₁-C₆-Alkoxy.
- W: steht bevorzugt für Cyano, Nitro, Halogen, C₁-C₆-Alkyl, C₂-C₆-Alkenyl, C₂-C₆-Alkinyl, C₁-C₆-Alkoxy, C₁-C₄-Halogenalkyl, C₁-C₄-Halogenalkoxy oder jeweils gegebenenfalls durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl, Phenoxy, Phenylthio, Phenyl-C₁-C₄-alkoxy oder Phenyl-C₁-C₄-alkylthio,
- X: steht bevorzugt für Wasserstoff, Halogen, C₁-C₆-Alkyl, C₂-C₆-Alkenyl, C₂-C₆-Alkinyl, C₁-C₆-Alkoxy, C₁-C₄-Halogenalkyl, C₁-C₄-Halogenalkoxy, Cyano, Nitro oder jeweils gegebenenfalls durch Halogen, C₁-C₆-Alkyl, C₁₋C₆-Alkoxy, C₁-C₄-Halogenalkyl, C₁-C₄-Halogenalkoxy, Nitro oder Cyano substituiertes Phenyl, Phenoxy, Phenylthio, Phenyl-C₁-C₄-alkoxy oder Phenyl-C₁-C₄-alkylthio,
- Y: steht bevorzugt für Wasserstoff, Halogen, C₁-C₆-Alkyl, C₁-C₆-Alkoxy, C₁₋C₄-Halogenalkyl, C₁-C₄-Halogenalkoxy, Cyano oder Nitro,
- Z: steht bevorzugt für Halogen, C₁-C₆-Alkyl, C₁-C₆-Alkoxy, C₁-C₄-Halogenalkyl, C₁-C₄-Halogenalkoxy, Hydroxy, Cyano, Nitro oder jeweils gegebenenfalls durch Halogen, C₁-C₄-Alkyl, C₁-C₄-Alkoxy, C₁-C₄-Halogenalkyl, C₁-C₄-Halogenalkoxy, Nitro oder Cyano substituiertes Phenoxy, Phenylthio, Thiazolyloxy, Pyridinyloxy, Pyrimidyloxy, Pyrazolyloxy, Phenyl-C₁-C₄-alkyloxy oder Phenyl-C₁-C-alkylthio oder
- Y und Z: stehen bevorzugt gemeinsam für jeweils gegebenenfalls durch Halogen, C₁-C₆-Alkyl, C₁-C₆-Alkoxy oder C₁-C₄-Halogenalkyl substituiertes C₃-C₅₋Alkandiyl oder C₃-C₅-Alkendiyl, in welchen gegebenenfalls ein bis drei Glieder durch Sauerstoff, Schwefel, Stickstoff oder eine Carbonylgruppe unabhängig voneinander ersetzt sein können, oder
- W und Z: stehen gemeinsam für jeweils gegebenenfalls durch Halogen, C₁-C₆₋Alkyl, C₁-C₆-Alkoxy oder C₁-C₄-Halogenalkyl substituiertes C₃-C₅₋Alkandiyl oder C₃-C₅-Alkendiyl, in welchen gegebenenfalls ein bis drei Glieder durch Sauerstoff, Schwefel, Stickstoff oder eine Carbonylgruppe unabhängig voneinander ersetzt sein können.
- Het: steht bevorzugt für eine der Gruppen 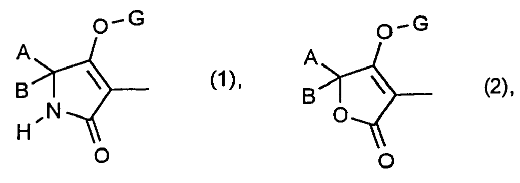 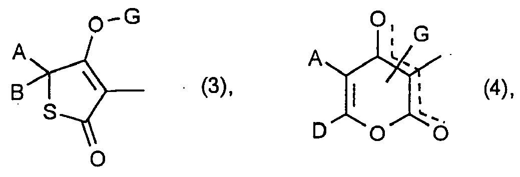 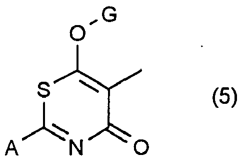
- A: steht bevorzugt für Wasserstoff, für jeweils gegebenenfalls durch Halogen substituiertes C₁-C₁₂-Alkyl, C₂-C₈-Alkenyl, C₁-C₁₀-Alkoxy-C₁-C₈-alkyl, Poly-C₁-C₈-alkoxy-C₁-C₈-alkyl oder C₁-C₁₀-Alkylthio-C₁-C₆-alkyl, für gegebenenfalls durch Halogen, C₁-C₆-Alkyl oder C₁-C₆-Alkoxy substituiertes C₃-C₈-Cycloalkyl, in welchem gegebenenfalls eine oder zwei nicht direkt benachbarte Methylengruppen durch Sauerstoff und/oder Schwefel ersetzt sind oder für jeweils gegebenenfalls durch Halogen, C₁-C₆-Alkyl, C₁-C₆₋Halogenalkyl, C₁-C₆-Alkoxy, C₁-C₆-Halogenalkoxy, Cyano oder Nitro substituiertes Phenyl, Naphthyl, Phenyl-C₁-C₆-alkyl, Naphthyl-C₁-C₆-alkyl oder Hetaryl mit 5 oder 6 Ringatomen und ein bis drei Heteroatomen aus der Reihe Sauerstoff, Schwefel und Stickstoff.
- B: steht bevorzugt für Wasserstoff, C₁-C₁₂-Alkyl oder C₁-C₈-Alkoxy-C₁-C₆₋alkyl oder
- A, B: und das Kohlenstoffatom an das sie gebunden sind, stehen bevorzugt für C₃-C₁₀-Cycloalkyl oder C₅-C₁₀-Cycloalkenyl, worin jeweils gegebenenfalls eine Methylengruppe durch Sauerstoff oder Schwefel ersetzt ist und welche gegebenenfalls durch C₁-C₈-Alkyl, C₃-C₁₀-Cycloalkyl, C₁-C₈-Halogenalkyl, C₁-C₈-Alkoxy, C₁-C₈-Alkylthio, Halogen oder Phenyl substituiert sind oder
- A, B: und das Kohlenstoffatom, an das sie gebunden sind, stehen bevorzugt für C₅-C₆-Cycloalkyl, welches durch eine gegebenenfalls ein oder zwei Sauerstoff- und/oder Schwefelatome enthaltende Alkylendiyl-, oder durch eine Alkylendioxy- oder durch eine Alkylendithioyl-Gruppe substituiert ist, die mit dem Kohlenstoffatom, an das sie gebunden ist, einen weiteren fünf- bis achtgliedrigen Ring bildet oder
- A, B: und das Kohlenstoffatom, an das sie gebunden sind stehen bevorzugt für C₃-C₈-Cycloalkyl oder C₅-C₈-Cycloalkenyl, in dem zwei Kohlenstoffatome durch jeweils gegebenenfalls durch C₁-C₆-Alkyl, C₁-C₆-Alkoxy oder Halogen substituiertes C₃-C₆-Alkandiyl, C₃-C₆-Alkendiyl oder C₄-C₆-Alkandiendiyl miteinander verbunden sind, worin jeweils gegebenenfalls eine Methylengruppe durch Sauerstoff oder Schwefel ersetzt ist.
- D: steht bevorzugt für Wasserstoff, für jeweils gegebenenfalls durch Halogen substituiertes C₁-C₁₂-Alkyl, C₃-C₈-Alkenyl, C₃-C₈-Alkinyl, C₁-C₁₀-Alkoxy-C₂-C₈-alkyl, Poly-C₁-C₈-alkoxy-C₂-C₈-alkyl oder C₁-C₁₀-Alkylthio-C₂-C₈₋alkyl, für gegebenenfalls durch Halogen, C₁-C₄-Alkyl, C₁-C₄-Alkoxy oder C₁-C₄-Halogenalkyl substituiertes C₃-C₈-Cycloalkyl, in welchem gegebenenfalls eine oder zwei nicht direkt benachbarte Methylengruppen durch Sauerstoff und/oder Schwefel ersetzt sind oder für jeweils gegebenenfalls durch Halogen, C₁-C₆-Alkyl, C₁-C₆-Halogenalkyl, C₁-C₆-Alkoxy, C₁-C₆₋Halogenalkoxy, Cyano oder Nitro substituiertes Phenyl, Hetaryl mit 5 bis 6 Ringatomen und ein oder zwei Heteroatomen aus der Reihe Sauerstoff, Schwefel und Stickstoff, Phenyl-C₁-C₆-alkyl oder Hetaryl-C₁-C₆-alkyl mit 5 bis 6 Ringatomen und ein oder zwei Heteroatomen aus der Reihe Sauerstoff, Schwefel und Stickstoff oder
- A und D: stehen gemeinsam bevorzugt für eine C₃-C₆-Alkandiyl-, C₃-C₆-Alkendiyl- oder C₄-C₆-Alkadiendiylgruppe, in welchen jeweils gegebenenfalls eine Methylengruppe durch Sauerstoff oder Schwefel ersetzt ist und welche jeweils gegebenenfalls substituiert sind durch Halogen oder durch jeweils gegebenenfalls durch Halogen substituiertes C₁-C₁₀-Alkyl, C₁-C₆-Alkoxy, C₁-C₆-Alkylthio, C₃-C₇-Cycloalkyl, Phenyl oder Benzyloxy oder durch eine weitere, einen ankondensierten Ring bildende C₃-C₆-Alkandiyl-, C₃-C₆₋Alkendiyl- oder C₄-C₆-Alkadiendiylgruppe, in welchen gegebenenfalls jeweils eine Methylengruppe durch Sauerstoff oder Schwefel ersetzt ist und welche gegebenenfalls durch C₁-C₆-Alkyl substituiert sind, oder
- A und D: stehen gemeinsam für eine C₃-C₆-Alkandiyl- oder C₃-C₆-Alkendiylgruppe, worin jeweils gegebenenfalls eine der folgenden Gruppen 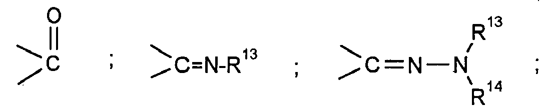 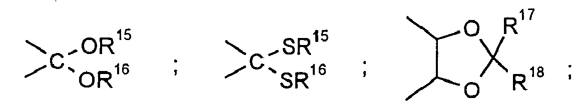 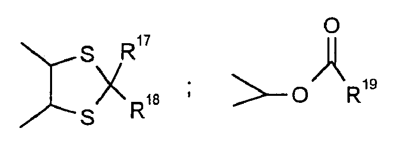 oder 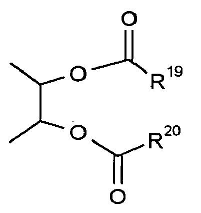 enthalten ist.
- G: steht bevorzugt für Wasserstoff (a) oder für eine der Gruppen 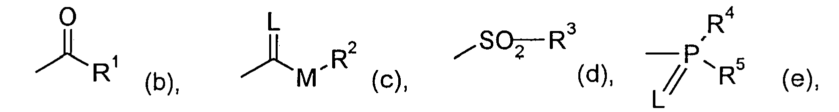 E (f) oder 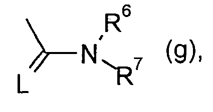 in welchen
E für ein Metallionäquivalent oder ein Ammoniumion steht,
L für Sauerstoff oder Schwefel steht und
M für Sauerstoff oder Schwefel steht.
- R¹: steht bevorzugt für jeweils gegebenenfalls durch Halogen substituiertes C₁₋C₂₀-Alkyl, C₂-C₂₀-Alkenyl, C₁-C₈-Alkoxy-C₁-C₈-alkyl, C₁-C₈-Alkylthio-C₁-C₈-alkyl oder Poly-C₁-C₈-alkoxy-C₁-C₈-alkyl oder für gegebenenfalls durch Halogen, C₁-C₆-Alkyl oder C₁-C₆-Alkoxy substituiertes C₃-C₈-Cycloalkyl, in welchem gegebenenfalls eine oder zwei nicht direkt benachbarte Methylengruppen durch Sauerstoff und/oder Schwefel ersetzt sind,
für gegebenenfalls durch Halogen, Cyano, Nitro, C₁-C₆-Alkyl, C₁-C₆₋Alkoxy, C₁-C₆-Halogenalkyl, C₁-C₆-Halogenalkoxy, C₁-C₆-Alkylthio oder C₁-C₆-Alkylsulfonyl substituiertes Phenyl,
für gegebenenfalls durch Halogen, Nitro, Cyano, C₁-C₆-Alkyl, C₁-C₆₋Alkoxy, C₁-C₆-Halogenalkyl oder C₁-C₆-Halogenalkoxy substituiertes Phenyl-C₁-C₆-alkyl,
für gegebenenfalls durch Halogen oder C₁-C₆-Alkyl substituiertes 5- oder 6-gliedriges Hetaryl mit ein oder zwei Heteroatomen aus der Reihe Sauerstoff, Schwefel und Stickstoff,
für gegebenenfalls durch Halogen oder C₁-C₆-Alkyl substituiertes Phenoxy-C₁-C₆-alkyl oder
für gegebenenfalls durch Halogen, Amino oder C₁-C₆-Alkyl substituiertes 5- oder 6-gliedriges Hetaryloxy-C₁-C₆-alkyl mit ein oder zwei Heteroatomen aus der Reihe Sauerstoff, Schwefel und Stickstoff.
- R²: steht bevorzugt für jeweils gegebenenfalls durch Halogen substituiertes C₁₋C₂₀-Alkyl, C₂-C₂₀-Alkenyl, C₁-C₈-Alkoxy-C₂-C₈-alkyl oder Poly-C₁-C₈₋alkoxy-C₂-C₈-alkyl,
für gegebenenfalls durch Halogen, C₁-C₆-Alkyl oder C₁-C₆-Alkoxy substituiertes C₃-C₈-Cycloalkyl oder
für jeweils gegebenenfalls durch Halogen, Cyano, Nitro, C₁-C₆-Alkyl, C₁₋C₆-Alkoxy, C₁-C₆-Halogenalkyl oder C₁-C₆-Halogenalkoxy substituiertes Phenyl oder Benzyl.
- R³: steht bevorzugt für gegebenenfalls durch Halogen substituiertes C₁-C₈₋Alkyl oder jeweils gegebenenfalls durch Halogen, C₁-C₆-Alkyl, C₁-C₆-Alkoxy, C₁-C₄-Halogenalkyl, C₁-C₄-Halogenalkoxy, Cyano oder Nitro substituiertes Phenyl oder Benzyl.
- R⁴ und R⁵: stehen unabhängig voneinander bevorzugt für jeweils gegebenenfalls durch Halogen substituiertes C₁-C₈-Alkyl, C₁-C₈-Alkoxy, C₁-C₈-Alkylamino, Di-(C₁-C₈-alkyl)amino, C₁-C₈-Alkylthio oder C₃-C₈-Alkenylthio oder für jeweils gegebenenfalls durch Halogen, Nitro, Cyano, C₁-C₄₋Alkoxy, C₁-C₄-Halogenalkoxy, C₁-C₄-Alkylthio, C₁-C₄-Halogenalkylthio, C₁-C₄-Alkyl oder C₁-C₄-Halogenalkyl substituiertes Phenyl, Phenoxy oder Phenylthio.
- R⁶ und R⁷: stehen unabhängig voneinander bevorzugt für Wasserstoff, für jeweils gegebenenfalls durch Halogen substituiertes C₁-C₈-Alkyl, C₃-C₈-Cycloalkyl, C₁-C₈-Alkoxy, C₃-C₈-Alkenyl oder C₁-C₈-Alkoxy-C₂-C₈-alkyl, für jeweils gegebenenfalls durch Halogen, C₁-C₈-Alkyl, C₁-C₈-Halogenalkyl oder C₁-C₈-Alkoxy substituiertes Phenyl oder Benzyl oder zusammen für einen gegebenenfalls durch C₁-C₆-Alkyl substituierten C₃-C₆-Alkylenrest, in welchem gegebenenfalls eine Methylengruppe durch Sauerstoff oder Schwefel ersetzt ist.
- R¹³: steht bevorzugt für Wasserstoff oder jeweils gegebenenfalls durch Halogen substituiertes C₁-C₈-Alkyl oder C₁-C₈-Alkoxy, für gegebenenfalls durch Halogen, C₁-C₄-Alkyl oder C₁-C₄-Alkoxy substituiertes C₃-C₈-Cycloalkyl, in welchem gegebenenfalls eine Methylengruppe durch Sauerstoff oder Schwefel ersetzt ist, oder für jeweils gegebenenfalls durch Halogen, C₁-C₆₋Alkyl, C₁-C₆-Alkoxy, C₁-C₄-Halogenalkyl, C₁-C₄-Halogenalkoxy, Nitro oder Cyano substituiertes Phenyl, Phenyl-C₁-C₄-alkyl oder Phenyl-C₁-C₄₋alkoxy.
- R¹⁴: steht bevorzugt für Wasserstoff oder C₁-C₈-Alkyl oder
- R¹³ und R¹⁴: stehen gemeinsam bevorzugt für C₄-C₆-Alkandiyl.
- R¹⁵ und R¹⁶: sind gleich oder verschieden und stehen bevorzugt für C₁-C₆-Alkyl oder
- R¹⁵ und R¹⁶: stehen gemeinsam bevorzugt für einen C₂-C₄-Alkandiylrest, der gegebenenfalls durch C₁-C₆-Alkyl oder durch gegebenenfalls durch Halogen, C₁-C₄-Alkyl, C₁-C₄-Halogenalkyl, C₁-C₄-Alkoxy, C₁-C₄-Halogenalkoxy, Nitro oder Cyano substituiertes Phenyl substituiert ist.
- R¹⁷ und R¹⁸: stehen unabhängig voneinander bevorzugt für Wasserstoff, für gegebenenfalls durch Halogen substituiertes C₁-C₈-Alkyl oder für gegebenenfalls durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl oder
- R¹⁷ und R¹⁸: stehen gemeinsam mit dem Kohlenstoffatom, an das sie gebunden sind, für gegebenenfalls durch C₁-C₄-Alkyl substituiertes C₅-C₇-Cycloalkyl, in dem gegebenenfalls eine Methylengruppe durch Sauerstoff oder Schwefel ersetzt ist.
- R¹⁹ und R²⁰: stehen unabhängig voneinander bevorzugt für C₁-C₁₀-Alkyl, C₂-C₁₀₋Alkenyl, C₁-C₁₀-Alkoxy, C₁-C₁₀-Alkylamino, C₃-C₁₀-Alkenylamino, Di-(C₁-C₁₀-alkyl)amino oder Di-(C₃-C₁₀-alkenyl)amino.
- V: steht besonders bevorzugt für Wasserstoff, Fluor, Chlor, Brom, C₁-C₄₋Alkyl oder C₁-C₄-Alkoxy.
- W: steht besonders bevorzugt für Cyano, Nitro, Fluor, Chlor, Brom, C₁-C₄₋Alkyl, C₁-C₄-Alkoxy, C₁-C₂-Halogenalkyl, C₁-C₂-Halogenalkoxy oder jeweils gegebenenfalls durch Fluor, Chlor, Brom, C₁-C₄-Alkyl, C₁-C₄-Alkoxy, C₁-C₂-Halogenalkyl, C₁-C₂-Halogenalkoxy, Nitro oder Cyano substituiertes Phenyl, Phenoxy, Benzyl oder Benzyloxy.
- X: steht besonders bevorzugt für Wasserstoff, Fluor, Chlor, Brom, C₁-C₄₋Alkyl, C₁-C₄-Alkoxy, C₁-C₂-Halogenalkyl, C₁-C₂-Halogenalkoxy, Cyano, Nitro oder jeweils gegebenenfalls durch Fluor, Chlor, Brom, C₁-C₄-Alkyl, C₁-C₄-Alkoxy, C₁-C₂-Halogenalkyl, C₁-C₂-Halogenalkoxy, Nitro oder Cyano substituiertes Phenyl, Phenoxy, Benzyl oder Benzyloxy.
- Y: steht besonders bevorzugt für Wasserstoff, Fluor, Chlor, Brom, C₁-C₄₋Alkyl, C₁-C₄-Alkoxy, C₁-C₂-Halogenalkyl, C₁-C₂-Halogenalkoxy, Cyano oder Nitro:
- Z: steht besonders bevorzugt für Fluor, Chlor, Brom, C₁-C₄-Alkyl, C₁-C₄₋Alkoxy, C₁-C₂-Halogenalkyl, C₁-C₂-Halogenalkoxy, Hydroxy, Cyano, Nitro oder jeweils gegebenenfalls durch Fluor, Chlor, Brom, C₁-C₄-Alkyl, C₁-C₄₋Alkoxy, C₁-C₂-Halogenalkyl, C₁-C-Halogenalkoxy, Nitro oder Cyano substituiertes Phenoxy oder Benzyloxy, oder
- Y und Z: stehen besonders bevorzugt gemeinsam für jeweils gegebenenfalls durch Fluor, Chlor, Brom, C₁-C₄-Alkyl, C₁-C₄-Alkoxy oder C₁-C₂-Halogenalkyl substituiertes C₃-C₄-Alkandiyl oder C₃-C₄-Alkendiyl, worin gegebenenfalls ein oder zwei nicht direkt benachbarte Glieder durch Sauerstoff, Schwefel oder Stickstoff unabhängig voneinander ersetzt sind oder
- W und Z: stehen besonders bevorzugt gemeinsam für jeweils gegebenenfalls durch Fluor, Chlor, Brom, C₁-C₄-Alkyl, C₁-C₄-Alkoxy oder C₁-C₂-Halogenalkyl substituiertes C₃-C₄-Alkandiyl oder C₃-C₄-Alkenyldiyl, worin gegebenenfalls ein oder zwei nicht direkt benachbarte Glieder durch Sauerstoff, Schwefel oder Stickstoff unabhängig voneinander ersetzt sind.
- Het: steht besonders bevorzugt für eine der Gruppen 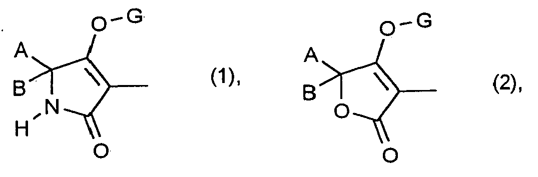 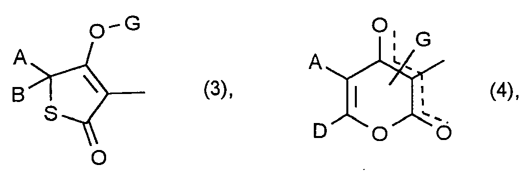 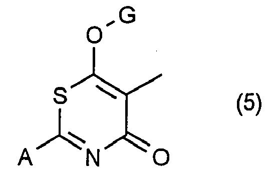
- A: steht besonders bevorzugt für Wasserstoff, für jeweils gegebenenfalls durch Fluor oder Chlor substituiertes C₁-C₁₀-Alkyl, C₂-C₆-Alkenyl, C₁-C₈₋Alkoxy-C₁-C₆-alkyl, Poly-C₁-C₆-alkoxy-C₁-C₆-alkyl oder C₁-C₈-Alkylthio-C₁-C₆-alkyl oder für gegebenenfalls durch Fluor, Chlor, C₁-C₄-Alkyl oder C₁-C₄-Alkoxy substituiertes C₃-C₇-Cycloalkyl, in welchem gegebenenfalls eine oder zwei nicht direkt benachbarte Methylengruppen durch Sauerstoff und/oder Schwefel ersetzt sind oder für jeweils gegebenenfalls durch Fluor, Chlor, Brom, C₁-C₄-Alkyl, C₁-C₄-Halogenalkyl, C₁-C₄-Alkoxy, C₁-C₄₋Halogenalkoxy, Cyano oder Nitro substituiertes Phenyl, Furanyl, Pyridyl, Imidazolyl, Triazolyl, Pyrazolyl, Indolyl, Thiazolyl, Thienyl oder Phenyl-C₁-C₄-alkyl.
- B: steht besonders bevorzugt für Wasserstoff, C₁-C₁₀-Alkyl oder C₁-C₆₋Alkoxy-C₁-C₄-alkyl oder
- A, B: und das Kohlenstoffatom an das sie gebunden sind, stehen besonders bevorzugt für C₃-C₈-Cycloalkyl oder C₅-C₈-Cycloalkenyl, worin jeweils gegebenenfalls eine Methylengruppe durch Sauerstoff oder Schwefel ersetzt ist und welche gegebenenfalls durch C₁-C₆-Alkyl, C₃-C₈-Cycloalkyl, C₁-C₃₋Halogenalkyl, C₁-C₆-Alkoxy, C₁-C₆-Alkylthio, Fluor, Chlor oder Phenyl substituiert sind oder
- A, B: und das Kohlenstoffatom, an das sie gebunden sind, stehen besonders bevorzugt für C₅-C₆-Cycloalkyl, welches durch eine gegebenenfalls ein oder zwei Sauerstoff- oder Schwefelatome enthaltende Alkylendiyl- oder durch eine Alkylendioxy- oder durch eine Alkylendithiol-Gruppe substituiert ist, die mit dem Kohlenstoffatom, an das sie gebunden ist, einen weiteren fünf- bis siebengliedrigen Ring bildet oder
- A, B: und das Kohlenstoffatom, an das sie gebunden sind, stehen besonders bevorzugt für C₃-C₆-Cycloalkyl oder C₅-C₆-Cycloalkenyl, worin zwei Kohlenstoffatome durch jeweils gegebenenfalls durch C₁-C₄-Alkyl, C₁-C₄₋Alkoxy, Fluor, Chlor oder Brom substituiertes C₃-C₅-Alkandiyl, C₃-C₅₋Alkendiyl oder Butadiendiyl miteinander verbunden sind, worin jeweils gegebenenfalls eine Methylengruppe durch Sauerstoff oder Schwefel ersetzt ist.
- D: steht besonders bevorzugt für Wasserstoff, für jeweils gegebenenfalls durch Fluor oder Chlor substituiertes C₁-C₁₀-Alkyl, C₃-C₆-Alkenyl, C₃-C₆₋Alkinyl, C₁-C₈-Alkoxy-C₂-C₆-alkyl, Poly-C₁-C₆-alkoxy-C₂-C₆-alkyl oder C₁-C₈-Alkylthio-C₂-C₆-alkyl, für gegebenenfalls durch Fluor, Chlor, C₁-C₄₋Alkyl, C₁-C₄-Alkoxy oder C₁-C₂-Halogenalkyl substituiertes C₃-C₇-Cycloalkyl, in welchem gegebenenfalls eine oder zwei nicht direkt benachbarte Methylengruppen durch Sauerstoff und/oder Schwefel ersetzt sind oder für jeweils gegebenenfalls durch Fluor, Chlor, Brom, C₁-C₄-Alkyl, C₁-C₄-Halogenalkyl, C₁-C₄-Alkoxy, C₁-C₄-Halogenalkoxy, Cyano oder Nitro substituiertes Phenyl, Furanyl, Imidazolyl, Pyridyl, Thiazolyl, Pyrazolyl, Pyrimidyl, Pyrrolyl, Thienyl, Triazolyl oder Phenyl-C₁-C₄-alkyl oder
- A und D: stehen gemeinsam besonders bevorzugt für eine C₃-C₅-Alkandiyl- oder C₃-C₅-Alkendiylgruppe, worin jeweils gegebenenfalls ein Kohlenstoffatom durch Sauerstoff oder Schwefel ersetzt ist und welche gegebenenfalls substituiert sind durch Fluor, Chlor oder durch jeweils gegebenenfalls durch Fluor oder Chlor substituiertes C₁-C₆-Alkyl, C₁-C₄-Alkoxy, C₁-C₄₋Alkylthio, C₃-C₆-Cycloalkyl, Phenyl oder Benzyloxy oder
worin jeweils gegebenenfalls eine der folgenden Gruppen: 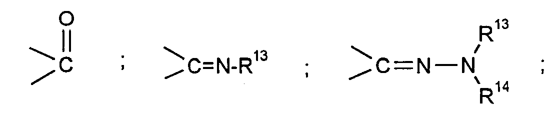 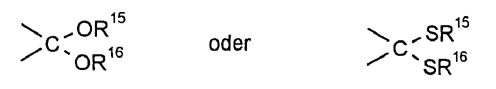 enthalten ist;
- G: steht besonders bevorzugt für Wasserstoff (a) oder für eine der Gruppen 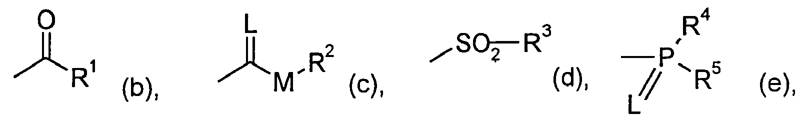 E (f) oder 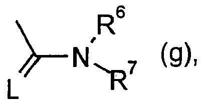 in welchen
E für ein Metallionäquivalent oder ein Ammoniumion steht,
L für Sauerstoff oder Schwefel steht und
M für Sauerstoff oder Schwefel steht.
- R¹: steht besonders bevorzugt für jeweils gegebenenfalls durch Fluor oder Chlor substituiertes C₁-C₁₆-Alkyl, C₂-C₁₆-Alkenyl, C₁-C₆-Alkoxy-C₁-C₆₋alkyl, C₁-C₆-Alkylthio-C₁-C₆-alkyl oder Poly-C₁-C₆-alkoxy-C₁-C₆-alkyl oder für gegebenenfalls durch Fluor, Chlor, C₁-C₅-Alkyl oder C₁-C₅₋Alkoxy substituiertes C₃-C₇-Cycloalkyl, in welchem gegebenenfalls eine oder zwei nicht direkt benachbarte Methylengruppen durch Sauerstoff und/oder Schwefel ersetzt sind,
für gegebenenfalls durch Fluor, Chlor, Brom, Cyano, Nitro, C₁-C₄-Alkyl, C₁-C₄-Alkoxy, C₁-C₃-Halogenalkyl, C₁-C₃-Halogenalkoxy, C₁-C₄-Alkylthio oder C₁-C₄-Alkylsulfonyl substituiertes Phenyl,
für gegebenenfalls durch Fluor, Chlor, Brom, C₁-C₄-Alkyl, C₁-C₄-Alkoxy, C₁-C₃-Halogenalkyl oder C₁-C₃-Halogenalkoxy substituiertes Phenyl-C₁₋C₄-alkyl,
für jeweils gegebenenfalls durch Fluor, Chlor, Brom oder C₁-C₄-Alkyl substituiertes Pyrazolyl, Thiazolyl, Pyridyl, Pyrimidyl, Furanyl oder Thienyl,
für gegebenenfalls durch Fluor, Chlor, Brom oder C₁-C₄-Alkyl substituiertes Phenoxy-C₁-C₅-alkyl oder
für jeweils gegebenenfalls durch Fluor, Chlor, Brom, Amino oder C₁-C₄₋Alkyl substituiertes Pyridyloxy-C₁-C₅-alkyl, Pyrimidyloxy-C₁-C₅-alkyl oder Thiazolyloxy-C₁-C₅-alkyl.
- R²: steht besonders bevorzugt für jeweils gegebenenfalls durch Fluor oder Chlor substituiertes C₁-C₁₆-Alkyl, C₂-C₁₆-Alkenyl, C₁-C₆-Alkoxy-C₂-C₆₋alkyl oder Poly-C₁-C₆-alkoxy-C₂-C₆-alkyl,
für gegebenenfalls durch Fluor, Chlor, C₁-C₄-Alkyl oder C₁-C₄-Alkoxy substituiertes C₃-C₇-Cycloalkyl oder
für jeweils gegebenenfalls durch Fluor, Chlor, Brom, Cyano, Nitro, C₁-C₄₋Alkyl, C₁-C₃-Alkoxy, C₁-C₃-Halogenalkyl oder C₁-C₃-Halogenalkoxy substituiertes Phenyl oder Benzyl.
- R³: steht besonders bevorzugt für gegebenenfalls durch Fluor oder Chlor substituiertes C₁-C₆-Alkyl oder jeweils gegebenenfalls durch Fluor, Chlor, Brom, C₁-C₄-Alkyl, C₁-C₄-Alkoxy, C₁-C₂-Halogenalkoxy, C₁-C₂-Halogenalkyl, Cyano oder Nitro substituiertes Phenyl oder Benzyl.
- R⁴ und R⁵: stehen unabhängig voneinander besonders bevorzugt für jeweils gegebenenfalls durch Fluor oder Chlor substituiertes C₁-C₆-Alkyl, C₁-C₆₋Alkoxy, C₁-C₆-Alkylamino, Di-(C₁-C₆-alkyl)amino, C₁-C₆-Alkylthio oder C₃-C₄-Alkenylthio oder für jeweils gegebenenfalls durch Fluor, Chlor, Brom, Nitro, Cyano, C₁-C₃-Alkoxy, C₁-C₃-Halogenalkoxy, C₁-C₃-Alkylthio, C₁-C₃-Halogenalkylthio, C₁-C₃-Alkyl oder C₁-C₃-Halogenalkyl substituiertes Phenyl, Phenoxy oder Phenylthio.
- R⁶ und R⁷: stehen unabhängig voneinander besonders bevorzugt für Wasserstoff, für jeweils gegebenenfalls durch Fluor oder Chlor substituiertes C₁-C₆₋Alkyl, C₃-C₆-Cycloalkyl, C₁-C₆-Alkoxy, C₃-C₆-Alkenyl oder C₁-C₆-Alkoxy-C₂-C₆-alkyl, für jeweils gegebenenfalls durch Fluor, Chlor, Brom, C₁₋C₅-Halogenalkyl, C₁-C₅-Alkyl oder C₁-C₅-Alkoxy substituiertes Phenyl oder Benzyl, oder zusammen für einen gegebenenfalls durch C₁-C₄-Alkyl substituierten C₃-C₆-Alkylenrest, in welchem gegebenenfalls eine Methylengruppe durch Sauerstoff oder Schwefel ersetzt ist.
- R¹³: steht besonders bevorzugt für Wasserstoff oder jeweils gegebenenfalls durch Fluor oder Chlor substituiertes C₁-C₆-Alkyl oder C₁-C₆-Alkoxy, für gegebenenfalls durch Fluor, C₁-C₂-Alkyl oder C₁-C₂-Alkoxy substituiertes C₃-C₇-Cycloalkyl, in welchem gegebenenfalls eine Methylengruppe durch Sauerstoff oder Schwefel ersetzt ist oder für jeweils gegebenenfalls durch Fluor, Chlor, Brom, C₁-C₅-Alkyl, C₁-C₅-Alkoxy, C₁-C₂-Halogenalkyl, C₁₋C₂-Halogenalkoxy, Nitro oder Cyano substituiertes Phenyl, Phenyl-C₁-C₃₋alkyl oder Phenyl-C₁-C₂-alkyloxy.
- R¹⁴: steht besonders bevorzugt für Wasserstoff oder C₁-C₆-alkyl oder
- R¹³ und R¹⁴: stehen gemeinsam besonders bevorzugt für C₄-C₆-Alkandiyl.
- R¹⁵ und R¹⁶: sind gleich oder verschieden und stehen besonders bevorzugt für C₁₋C₄-Alkyl oder
- R¹⁵ und R¹⁶: stehen zusammen besonders bevorzugt für einen C₂-C₃-Alkandiylrest, der gegebenenfalls durch C₁-C₄-Alkyl oder durch gegebenenfalls durch Fluor, Chlor, Brom, C₁-C₂-Alkyl, C₁-C₂-Halogenalkyl, C₁-C₂-Alkoxy, C₁₋C₂-Halogenalkoxy, Nitro oder Cyano substituiertes Phenyl substituiert ist.
- V: steht ganz besonders bevorzugt für Wasserstoff, Fluor, Chlor, Brom, Methyl, Ethyl, Propyl, iso-Propyl, tert.-Butyl, Methoxy, Ethoxy, Propoxy oder iso-Propoxy.
- W: steht ganz besonders bevorzugt für Cyano, Nitro, Fluor, Chlor, Brom, Methyl, Ethyl, Propyl, Butyl, iso-Propyl, iso-Butyl, tert.-Butyl, Methoxy, Ethoxy, Propoxy, iso-Propoxy, Trifluormethyl, Difluormethoxy, Trifluormethoxy, Phenyl oder Benzyloxy.
- X: steht ganz besonders bevorzugt für Wasserstoff, Fluor, Chlor, Brom, Methyl, Ethyl, Propyl, Butyl, iso-Butyl, iso-Propyl, tert.-Butyl, Methoxy, Ethoxy, Propoxy, iso-Propoxy, Trifluormethyl, Trifluormethoxy, Difluormethoxy, Cyano, Nitro, Phenyl oder Benzyloxy.
- Y: steht ganz besonders bevorzugt für Wasserstoff, Fluor, Chlor, Brom, Methyl, Ethyl, Propyl, iso-Propyl, Butyl, iso-Butyl, tert.-Butyl, Methoxy, Ethoxy, Propoxy, iso-Propoxy, Trifluormethyl, Trifluormethoxy, Difluormethoxy, Cyano oder Nitro.
- Z: steht ganz besonders bevorzugt für Fluor, Chlor, Brom, Methyl, Ethyl, Propyl, Butyl, iso-Butyl, iso-Propyl, tert.-Butyl, Methoxy, Ethoxy, Propoxy, iso-Propoxy, Trifluormethyl, Trifluormethoxy, Difluormethoxy, Cyano oder Nitro, oder
- Y und Z: stehen ganz besonders bevorzugt gemeinsam für gegebenenfalls durch Fluor, Chlor, Methyl, Ethyl, Propyl, iso-Propyl, Methoxy, Ethoxy, Propoxy, iso-Propoxy oder Trifluormethyl substituiertes C₃-C₄-Alkandiyl, in welchem gegebenenfalls zwei nicht direkt benachbarte Glieder durch Sauerstoff ersetzt sind, oder
- W und Z: stehen ganz besonders bevorzugt gemeinsam für gegebenenfalls durch Fluor, Chlor, Methyl, Ethyl, Propyl, iso-Propyl, Methoxy, Ethoxy, Propoxy, iso-Propoxy oder Trifluormethyl substituiertes C₃-C₄-Alkandiyl, in welchem gegebenenfalls zwei nicht direkt benachbarte Glieder durch Sauerstoff ersetzt sind.
- Het: steht ganz besonders bevorzugt für eine der Gruppen 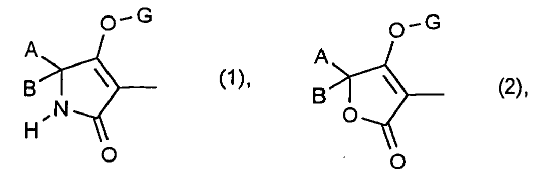 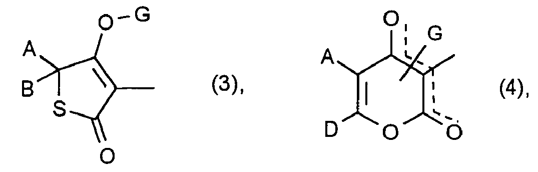 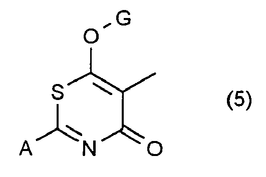
- A: steht ganz besonders bevorzugt für Wasserstoff, für jeweils gegebenenfalls durch Fluor oder Chlor substituiertes C₁-C₈-Alkyl, C₂-C₄-Alkenyl, C₁-C₆₋Alkoxy-C₁-C₄-alkyl, Poly-C₁-C₄-alkoxy-C₁-C₄-alkyl oder C₁-C₆-Alkylthio-C₁-C₄-alkyl oder für gegebenenfalls durch Fluor, Chlor, Methyl oder Methoxy substituiertes C₃-C₆-Cycloalkyl, in welchem gegebenenfalls eine oder zwei nicht direkt benachbarte Methylengruppen durch Sauerstoff und/oder Schwefel ersetzt sind oder für jeweils gegebenenfalls durch Fluor, Chlor, Brom, Methyl, Ethyl, n-Propyl, iso-Propyl, Methoxy, Ethoxy, Trifluormethyl, Trifluormethoxy, Cyano oder Nitro substituiertes Phenyl, Pyridyl oder Benzyl.
- B: steht ganz besonders bevorzugt für Wasserstoff, C₁-C₈-Alkyl oder C₁-C₄₋Alkoxy-C₁-C₂-alkyl oder
- A, B: und das Kohlenstoffatom an das sie gebunden sind, stehen ganz besonders bevorzugt für C₃-C₈-Cycloalkyl oder C₅-C₈-Cycloalkenyl, worin jeweils gegebenenfalls eine Methylengruppe durch Sauerstoff oder Schwefel ersetzt ist und welche gegebenenfalls durch Methyl, Ethyl, n-Propyl, iso-Ppropyl, Butyl, iso-Butyl, sec.-Butyl, tert.-Butyl, Cyclohexyl, Trifluormethyl, Methoxy, Ethoxy, n-Propoxy, iso-Propoxy, Butoxy, iso-Butoxy, sek.-Butoxy, tert.-Butoxy, Methylthio, Ethylthio, Fluor, Chlor oder Phenyl substituiert sind oder
- A, B: und das Kohlenstoffatom, an das sie gebunden sind, stehen ganz besonders bevorzugt für C₅-C₆-Cycloalkyl, welches durch eine gegebenenfalls ein Sauerstoff- oder Schwefelatom enthaltende Alkylendiyl- oder durch eine Alkylendioxy-Gruppe substituiert ist, die mit dem Kohlenstoffatom, an das sie gebunden ist, einen weiteren fünf- oder sechsgliedrigen Ring bildet oder
- A, B: und das Kohlenstoffatom, an das sie gebunden sind, stehen ganz besonders bevorzugt für C₃-C₆-Cycloalkyl oder C₅-C₆-Cycloalkenyl, worin zwei Kohlenstoffatome durch C₃-C₄-Alkandiyl, C₃-C₄-Alkendiyl oder Butadiendiyl miteinander verbunden sind.
- D: steht ganz besonders bevorzugt für Wasserstoff, für jeweils gegebenenfalls durch Fluor oder Chlor substituiertes C₁-C₈-Alkyl, C₃-C₄-Alkenyl, C₃-C₄₋Alkinyl, C₁-C₆-Alkoxy-C₂-C₄-alkyl, Poly-C₁-C₄-alkoxy-C₂-C₄-alkyl, C₁-C₄₋Alkylthio-C₂-C₄-alkyl oder C₃-C₆-Cycloalkyl, in welchem gegebenenfalls eine oder zwei nicht direkt benachbarte Methylengruppen durch Sauerstoff und/oder Schwefel ersetzt sind oder für jeweils gegebenenfalls durch Fluor, Chlor, Brom, Methyl, Ethyl, n-Propyl, iso-Propyl, Methoxy, Ethoxy, Trifluormethyl, Trifluormethoxy, Cyano oder Nitro substituiertes Phenyl, Furanyl, Pyridyl, Thienyl oder Benzyl,
oder
- A und D: stehen gemeinsam ganz besonders bevorzugt für eine C₃-C₅-Alkandiyl-oder C₃-C₅-Alkendiylgruppe, worin jeweils gegebenenfalls eine Methylengruppe durch Sauerstoff oder Schwefel ersetzt ist und welche gegebenenfalls durch Fluor, Chlor oder durch jeweils gegebenenfalls durch Fluor oder Chlor substituiertes C₁-C₆-Alkyl oder C₁-C₄-Alkoxy substituiert sind.
- G: G steht ganz besonders bevorzugt für Wasserstoff (a) oder für eine der Gruppen 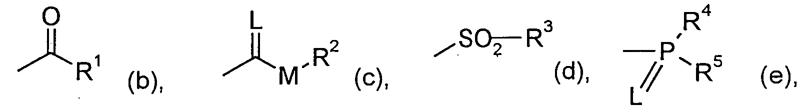 E (f) oder 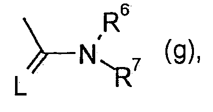 in welchen
E für ein Metallionäquivalent oder ein Ammoniumion steht,
L für Sauerstoff oder Schwefel steht und
M für Sauerstoff oder Schwefel steht.
- R¹: steht ganz besonders bevorzugt für jeweils gegebenenfalls durch Fluor oder Chlor substituiertes C₁-C₁₄-Alkyl, C₂-C₁₄-Alkenyl, C₁-C₄-Alkoxy-C₁-C₆₋alkyl, C₁-C₄-Alkylthio-C₁-C₆-alkyl, Poly-C₁-C₄-alkoxy-C₁-C₄-alkyl oder für gegebenenfalls durch Fluor, Chlor, Methyl, Ethyl, n-Propyl, i-Propyl, n-Butyl, i-Butyl, tert.-Butyl, Methoxy, Ethoxy, n-Propoxy oder iso-Propoxy substituiertes C₃-C₆-Cycloalkyl, in welchem gegebenenfalls eine oder zwei nicht direkt benachbarte Methylengruppen durch Sauerstoff und/oder Schwefel ersetzt sind,
für gegebenenfalls durch Fluor, Chlor, Brom, Cyano, Nitro, Methyl, Ethyl, n-Propyl, i-Propyl, Methoxy, Ethoxy, Trifluormethyl, Trifluormethoxy, Methylthio, Ethylthio, Methylsulfonyl oder Ethylsulfonyl substituiertes Phenyl,
für gegebenenfalls durch Fluor, Chlor, Brom, Methyl, Ethyl, n-Propyl, i-Propyl, Methoxy, Ethoxy, Trifluormethyl oder Trifluormethoxy substituiertes Benzyl,
für jeweils gegebenenfalls durch Fluor, Chlor, Brom, Methyl oder Ethyl substituiertes Furanyl, Thienyl oder Pyridyl,
für gegebenenfalls durch Fluor, Chlor, Methyl oder Ethyl substituiertes Phenoxy-C₁-C₄-alkyl oder
für jeweils gegebenenfalls durch Fluor, Chlor, Amino, Methyl oder Ethyl substituiertes Pyridyloxy-C₁-C₄-alkyl, Pyrimidyloxy-C₁-C₄-alkyl oder Thiazolyloxy-C₁-C₄-alkyl.
- R²: steht ganz besonders bevorzugt für jeweils gegebenenfalls durch Fluor oder Chlor substituiertes C₁-C₁₄-Alkyl, C₂-C₁₄-Alkenyl, C₁-C₄-Alkoxy-C₂-C₆₋alkyl oder Poly-C₁-C₄-alkoxy-C₂-C₆-alkyl,
für gegebenenfalls durch Fluor, Chlor, Methyl, Ethyl, n-Propyl, iso-Propyl oder Methoxy substituiertes C₃-C₆-Cycloalkyl,
oder für jeweils gegebenenfalls durch Fluor, Chlor, Cyano, Nitro, Methyl, Ethyl, n-Propyl, i-Propyl, Methoxy, Ethoxy, Trifluormethyl oder Trifluormethoxy substituiertes Phenyl oder Benzyl.
- R³: steht ganz besonders bevorzugt für gegebenenfalls durch Fluor oder Chlor substituiertes Methyl, Ethyl, Propyl, iso-Propyl, Butyl, tert.-Butyl, oder jeweils gegebenenfalls durch Fluor, Chlor, Brom, Methyl, Ethyl, iso-Propyl, tert.-Butyl, Methoxy, Ethoxy, iso-Propoxy, Trifluormethyl, Trifluormethoxy, Cyano oder Nitro substituiertes Phenyl oder Benzyl.
- R⁴ und R⁵: stehen unabhängig voneinander ganz besonders bevorzugt für jeweils gegebenenfalls durch Fluor oder Chlor substituiertes C₁-C₄-Alkyl, C₁-C₄₋Alkoxy, C₁-C₄-Alkylamino, Di-(C₁-C₄-alkyl)amino oder C₁-C₄-Alkylthio oder für jeweils gegebenenfalls durch Fluor, Chlor, Brom, Nitro, Cyano, Methyl, Methoxy, Trifluormethyl oder Trifluormethoxy substituiertes Phenyl, Phenoxy oder Phenylthio.
- R⁶ und R⁷: stehen unabhängig voneinander ganz besonders bevorzugt für Wasserstoff, für jeweils gegebenenfalls durch Fluor oder Chlor substituiertes C₁₋C₄-Alkyl, C₃-C₆-Cycloalkyl, C₁-C₄-Alkoxy, C₃-C₄-Alkenyl oder C₁-C₄₋Alkoxy-C₂-C₄-alkyl, für jeweils gegebenenfalls durch Fluor, Chlor, Brom, Methyl, Methoxy oder Trifluormethyl substituiertes Phenyl oder Benzyl, oder zusammen für einen gegebenenfalls durch Methyl oder Ethyl substituierten C₅-C₆-Alkylenrest, in welchem gegebenenfalls eine Methylengruppe durch Sauerstoff oder Schwefel ersetzt ist.

Ausgenommen sind jeweils folgende Verbindungen (bekannt aus EP-0 528 156):

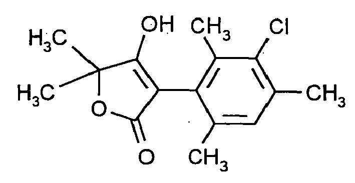

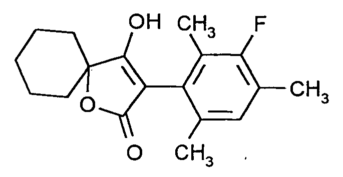

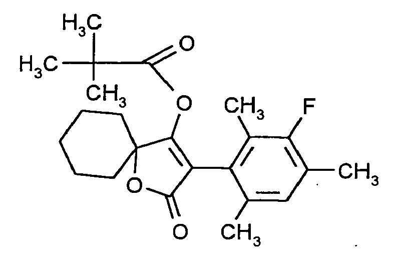

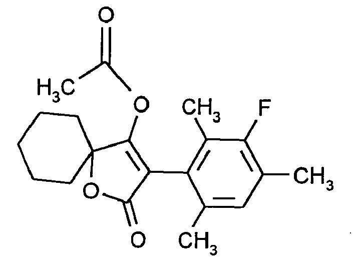

Wenn in Verbindungen der Formel (I-2) von den Substituenten X, V, Y, Z und W nur der Substituent V für Wasserstoff steht und X, Y, Z und W eine von Wasserstoff verschiedene Bedeutung haben, sind solche Verbindungen der Formel (I-2) bevorzugt, in welchen Z nicht für Halogen steht.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

Erfindungsgemäß ganz besonders bevorzugt werden die Verbindungen der Formel (I), in welchen eine Kombination der vorstehend als ganz besonders bevorzugt aufgeführten Bedeutungen vorliegt.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können einfach oder mehrfach substituiert sein, wobei bei Mehrfachsubstitutionen die Substituenten gleich oder verschieden sein können.

Insbesondere bevorzugt sind Verbindungen der Formel I-1 bis I-3, in welchen
a) A, B und das Kohlenstoffatom, an das sie gebunden sind, die als ganz besonders bevorzugt genannten Bedeutungen haben und V für Wasserstoff steht,
b) A, B und das Kohlenstoffatom, an das sie gebunden sind, die als ganz besonders bevorzugt genannten Bedeutungen haben und V und Y für Wasserstoff stehen.

Im einzelnen seien außer den bei den Herstellungsbeispielen genannten Verbindungen die folgenden Verbindungen der Formel (I-1-a) genannt:

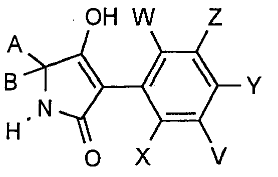

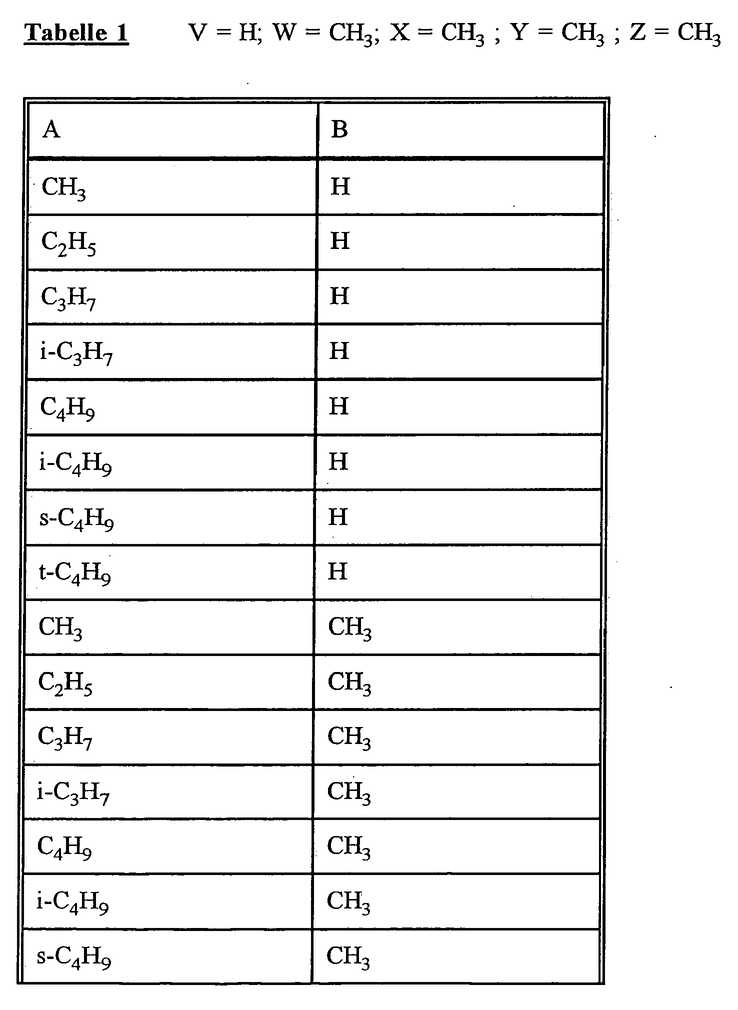

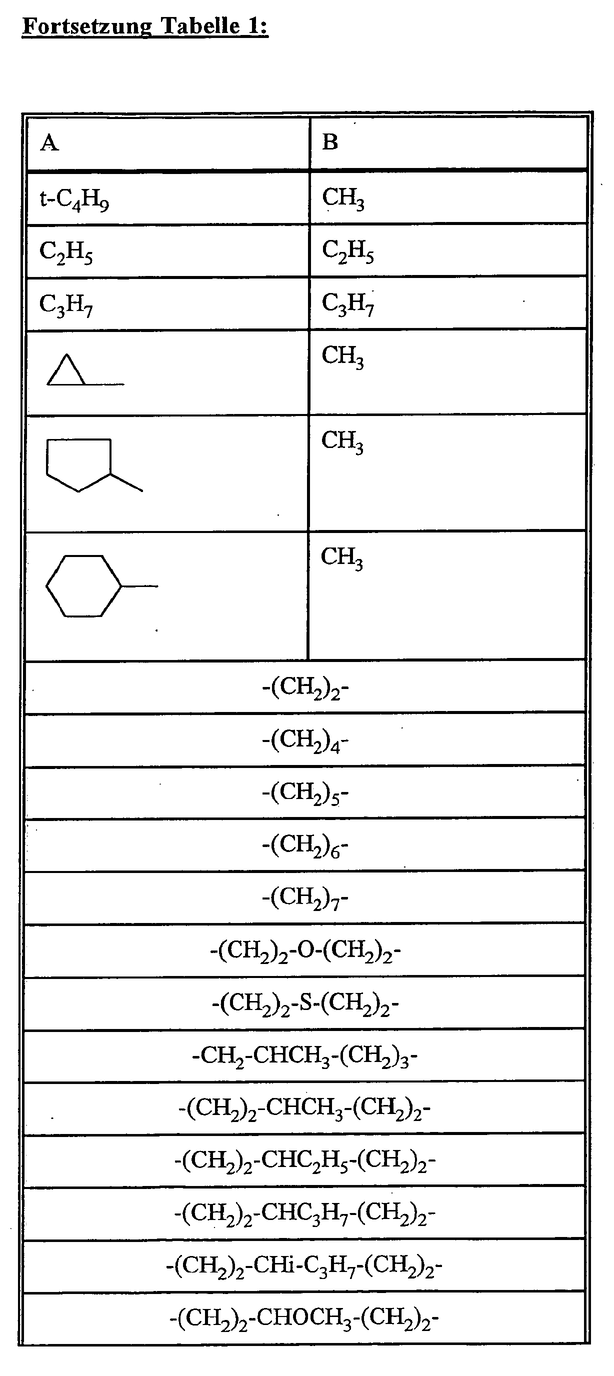

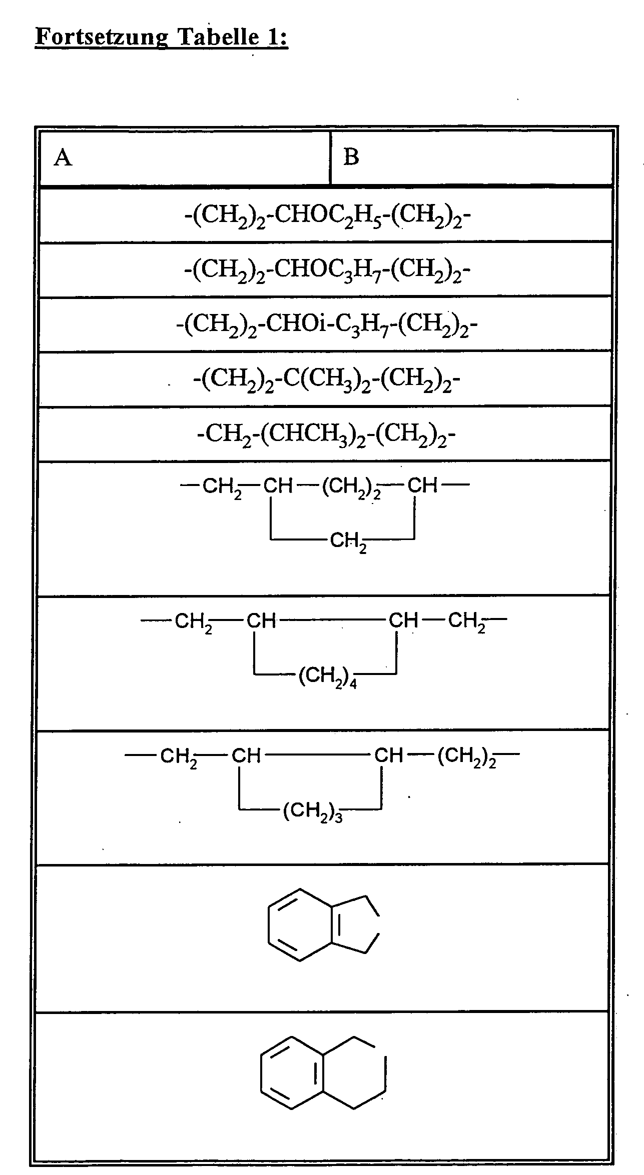

- **Tabelle 2:**: A und B haben die gleiche Bedeutung wie in Tabelle 1 mit V = H; W = CH₃; X = CH₃; Y = H; Z = C1
- **Tabelle 3:**: A und B haben die gleiche Bedeutung wie in Tabelle 1 mit V = H; W = CH₃; X = CH₃; Y = CH₃; Z = F

- **Tabelle 4:**: A und B haben die gleiche Bedeutung wie in Tabelle 1 mit V = H; W = CH₃; X = CH₃; Y = CH₃; Z = Cl
- **Tabelle 5:**: A und B haben die gleiche Bedeutung wie in Tabelle 1 mit V = H; W = CH₃; X = CH₃; Y = CH₃; Z = Br
- **Tabelle 6:**: A und B haben die gleiche Bedeutung wie in Tabelle 1 mit V = CH₃; W = CH₃, X = CH₃; Y = H; Z = CH₃
- **Tabelle 7:**: A und B haben die gleiche Bedeutung wie in Tabelle 1 mit V = CH₃; W = CH₃; X = H; Y = CH₃; Z = CH₃
- **Tabelle 8:**: A und B haben die gleiche Bedeutung wie in Tabelle 1 mit V = H; W = CH₃; X = CH₃; Y H; Z = Br
- **Tabelle 9:**: A und B haben die gleiche Bedeutung wie in Tabelle 1 mit V = H; W = Cl; X = Cl; Y = H; Z = Br
- **Tabelle 10:**: A und B haben die gleiche Bedeutung wie in Tabelle 1 mit V = H; W = Br; X = Br; Y, Z = -(CH₂)₃-
- **Tabelle 11:**: A und B haben die gleiche Bedeutung wie in Tabelle 1 mit V = H; W = CH₃; X = OCH₃; Y = H; Z = Br
- **Tabelle 12:**: A und B haben die gleiche Bedeutung wie in Tabelle 1 mit V = CH₃; W = CH₃; X = CH₃; Y = CH₃; Z = CH₃
- **Tabelle 13:**: A und B haben die gleiche Bedeutung wie in Tabelle 1 mit V = H; W = Cl; X = Cl; Y = Cl; Z = CH₃
- **Tabelle 14:**: A und B haben die gleiche Bedeutung wie in Tabelle 1 mit V = H; W = Br; X = Br; Y = Br; Z = CH₃

Im einzelnen seien außer den bei den Herstellungsbeispielen genannten Verbindungen die folgenden Verbindungen der Formel (I-2-a) genannt:

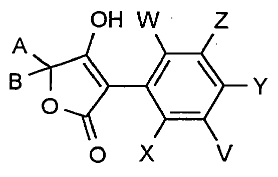

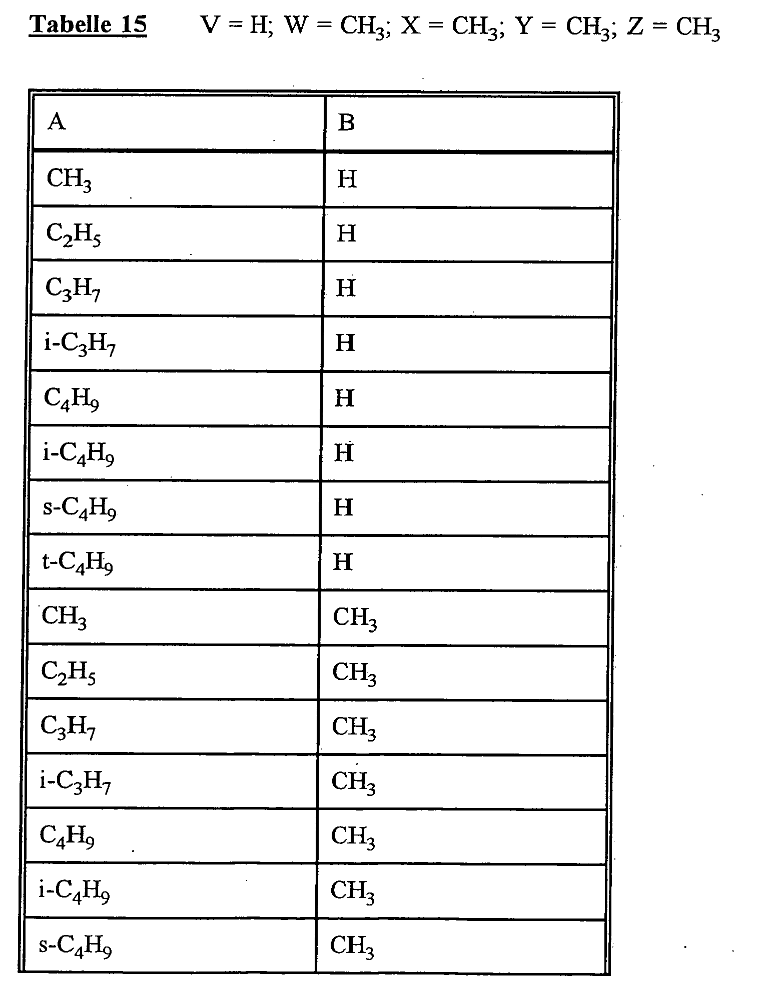

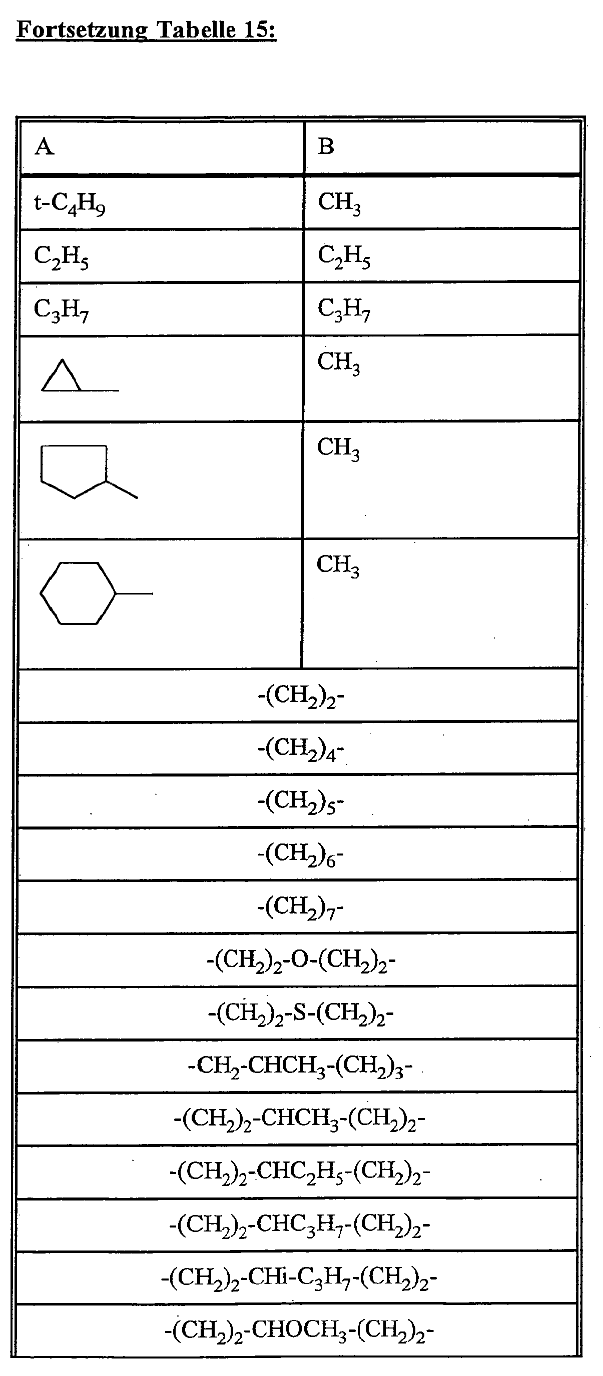

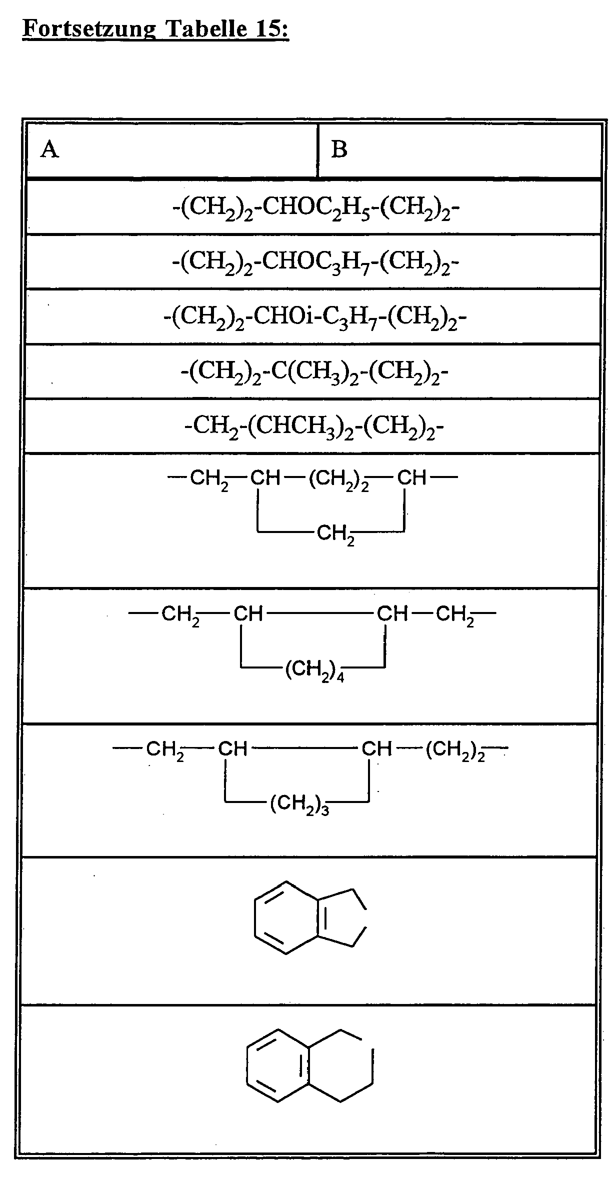

- **Tabelle 16:**: A und B haben die gleiche Bedeutung wie in Tabelle 15 mit V = H; W = CH₃; X = CH₃; Y = H; Z = Cl
- **Tabelle 17:**: A und B haben die gleiche Bedeutung wie in Tabelle 15 mit V = CH₃; W = CH₃; X = CH₃; Y = H; Z = CH₃
- **Tabelle 18:**: A und B haben die gleiche Bedeutung wie in Tabelle 15 mit V = CH₃, W = CH₃; X = H; Y = CH₃; Z = CH₃
- **Tabelle 19:**: A und B haben die gleiche Bedeutung wie in Tabelle 15 mit V = H; W = CH₃; X = CH₃; Y = H; Z = Br
- **Tabelle 20:**: A und B haben die gleiche Bedeutung wie in Tabelle 15 mit V = H; W = Cl; X=Cl; Y=H; Z = Br
- **Tabelle 21:**: A und B haben die gleiche Bedeutung wie in Tabelle 15 mit V = H; W = Br; X = Br; Y, Z = -(CH₃)₂-
- **Tabelle 22:**: A und B haben die gleiche Bedeutung wie in Tabelle 15 mit V = H; W = CH₃; X = OCH₃; Y = H; Z = Br
- **Tabelle 23:**: A und B haben die gleiche Bedeutung wie in Tabelle 15 mit V = CH₃, W = CH₃; X = CH₃; Y = CH₃; Z = CH₃
- **Tabelle 24:**: A und B haben die gleiche Bedeutung wie in Tabelle 15 mit V = H; W = Cl; X = Cl; Y = Cl; Z = CH₃
- **Tabelle 25:**: A und B haben die gleiche Bedeutung wie in Tabelle 15 mit V = H; W = Br; X = Br; Y = Br; Z = CH₃

Im einzelnen seien außer den bei den Herstellungsbeispielen genannten Verbindungen die folgenden Verbindungen der Formel (I-3-a) genannt:

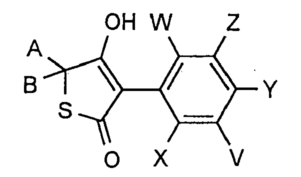

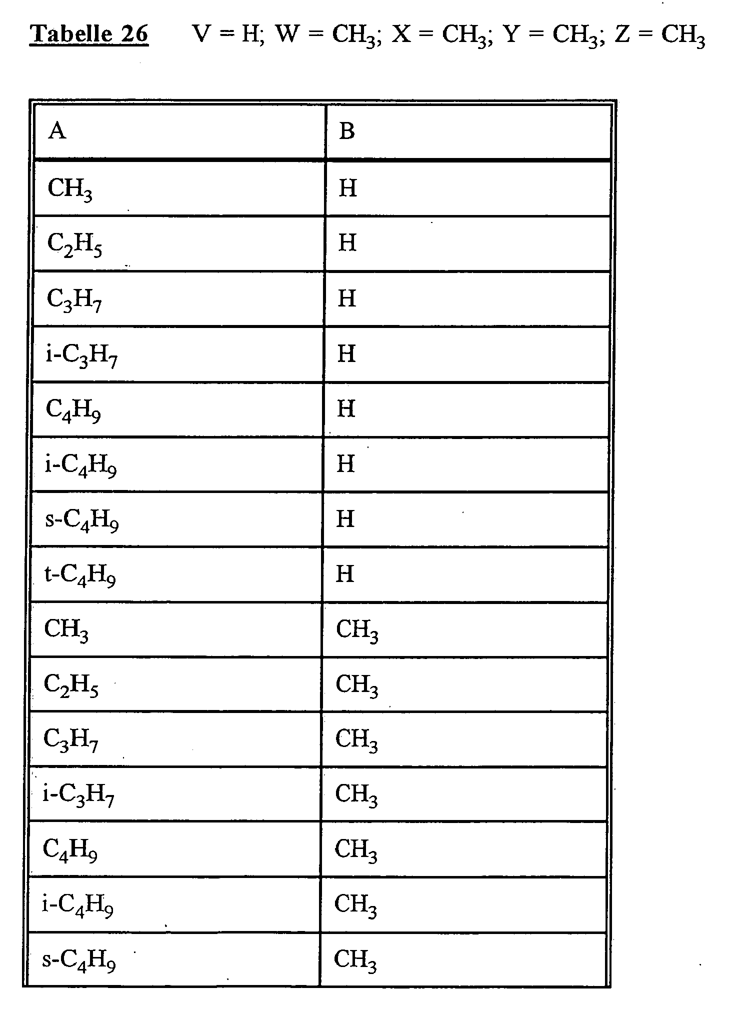

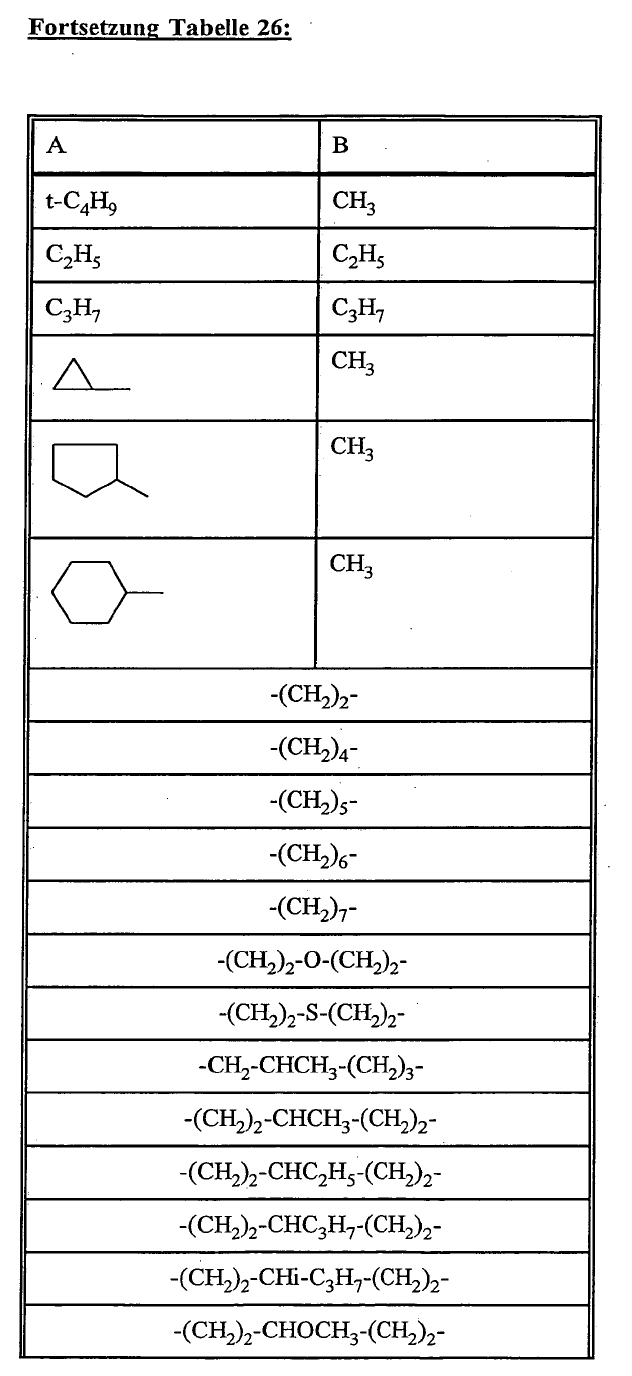

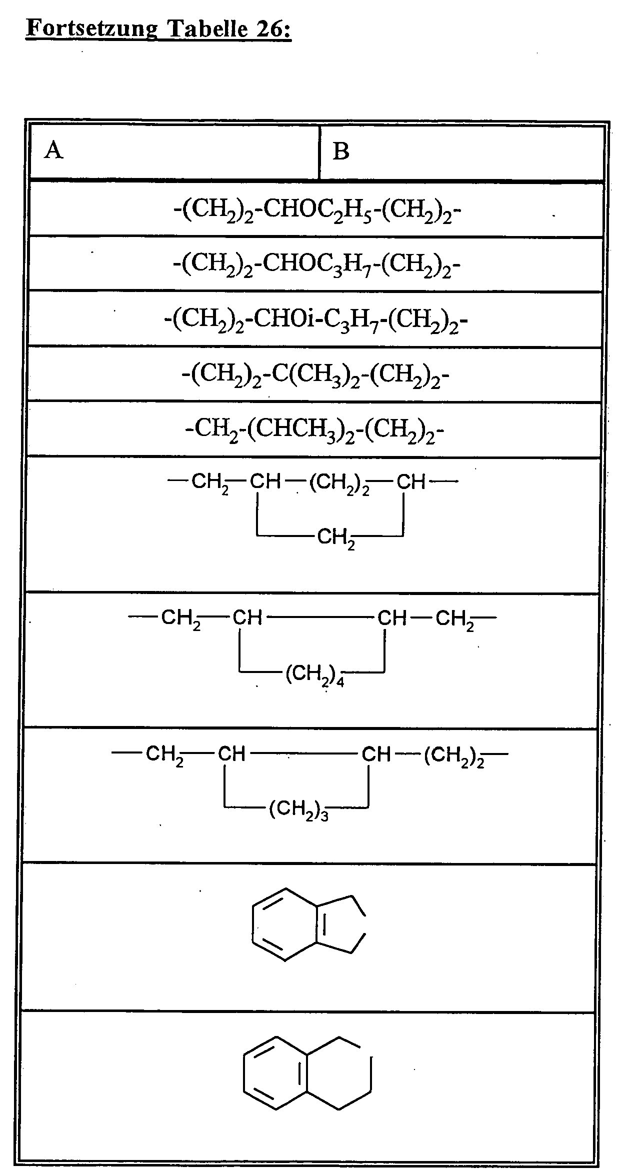

- **Tabelle 27:**: A und B haben die gleiche Bedeutung wie in Tabelle 26 mit V = H; W=CH₃; X=CH₃; Y=H; Z = Cl
- **Tabelle 28:**: A und B haben die gleiche Bedeutung wie in Tabelle 26 mit V = H; W = CH₃; X = CH₃; Y = CH₃; Z = Cl

- **Tabelle 29:**: A und B haben die gleiche Bedeutung wie in Tabelle 26 mit V = H; W = CH₃; X = CH₃; Y = CH₃; Z = F
- **Tabelle 30:**: A und B haben die gleiche Bedeutung wie in Tabelle 26 mit V = H; W = CH₃; X = CH₃; Y = CH₃; Z = Br
- **Tabelle 31:**: A und B haben die gleiche Bedeutung wie in Tabelle 26 mit V = CH₃ W = CH₃; X = CH₃; Y = H; Z = CH₃
- **Tabelle 32:**: A und B haben die gleiche Bedeutung wie in Tabelle 26 mit V = CH₃; W = CH₃; X = H; Y = CH₃; Z = CH₃
- **Tabelle 33:**: A und B haben die gleiche Bedeutung wie in Tabelle 26 mit V = H; W = CH₃; X = CH₃; Y = H; Z = Br
- **Tabelle 34:**: A und B haben die gleiche Bedeutung wie in Tabelle 26 mit V = H; W = Cl; X = Cl; Y = H; Z = Br
- **Tabelle 35:**: A und B haben die gleiche Bedeutung wie in Tabelle 26 mit V = H; W = Br; X - Br; Y, Z = -(CH₂)₃-
- **Tabelle 36:**: A und B haben die gleiche Bedeutung wie in Tabelle 26 mit V = H; W = CH₃; X=OCH₃; Y = H; Z = Br
- **Tabelle 37:**: A und B haben die gleiche Bedeutung wie in Tabelle 26 mit V = CH₃; W = CH₃; X = CH₃; Y = CH₃; Z = CH₃
- **Tabelle 38:**: A und B haben die gleiche Bedeutung wie in Tabelle 26 mit V = H; W = Cl; X = Cl; Y = Cl, Z = CH₃
- **Tabelle 39:**: A und B haben die gleiche Bedeutung wie in Tabelle 26 mit V=H; W=Br; X = Br; Y = Br; Z = CH₃

Im einzelnen seien außer den bei den Herstellungsbeispielen genannten Verbindungen die folgenden Verbindungen der Formel (I-4-a) genannt:

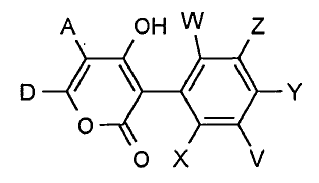

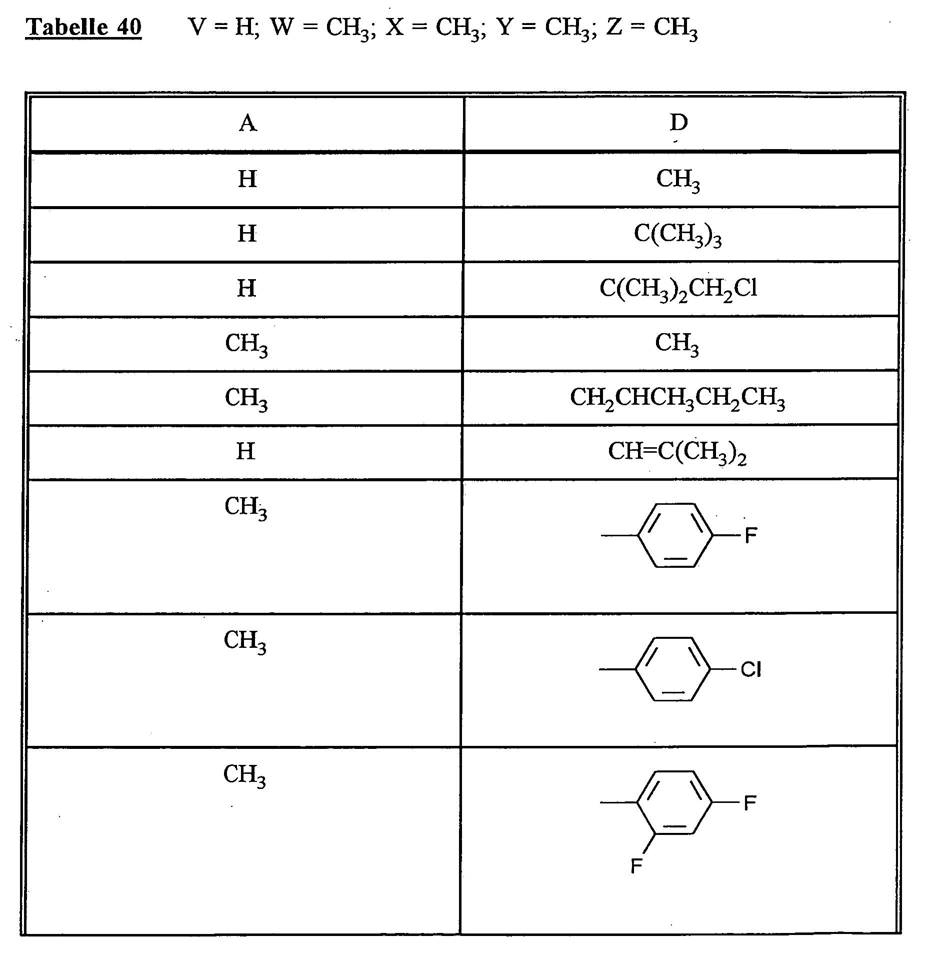

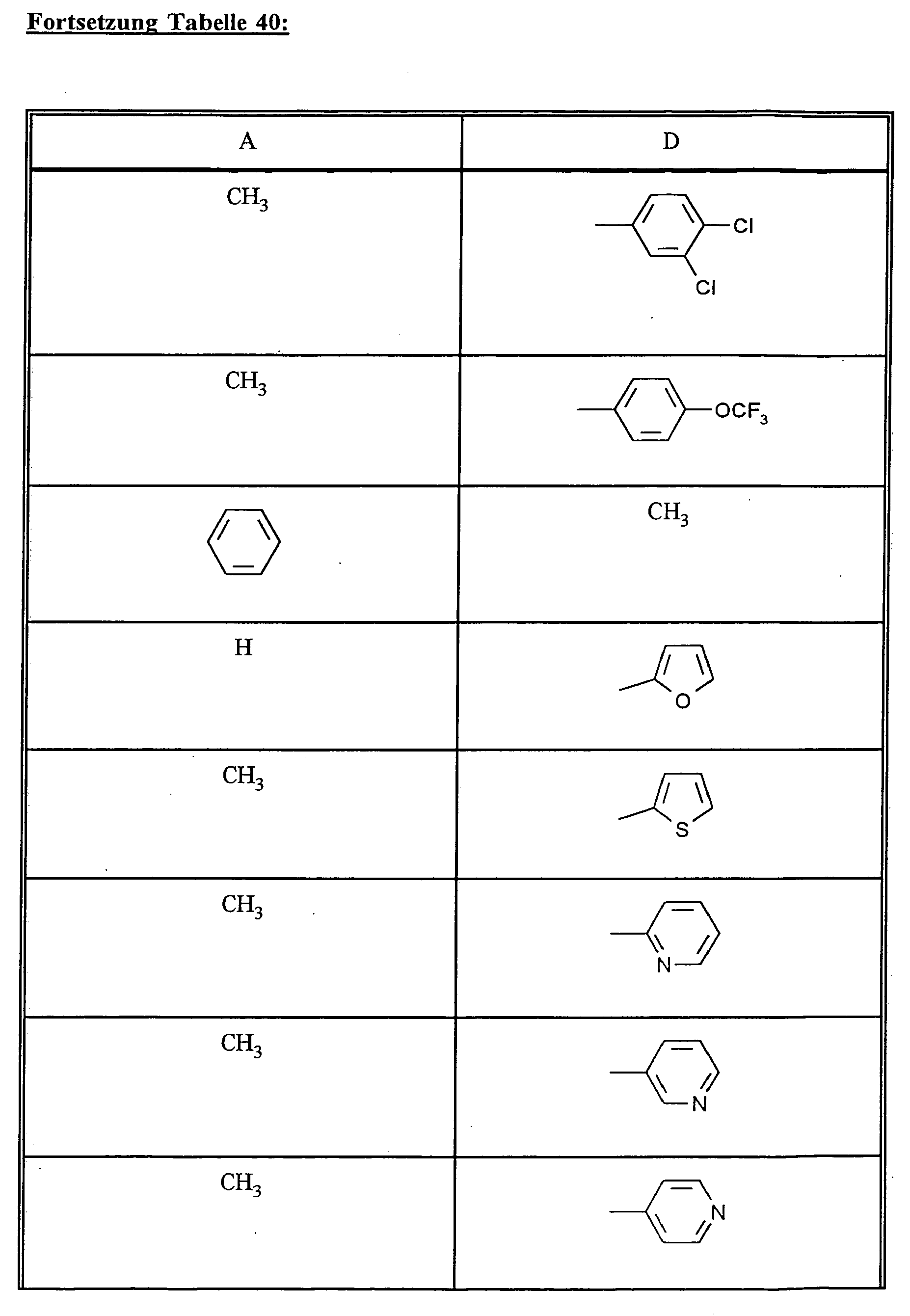

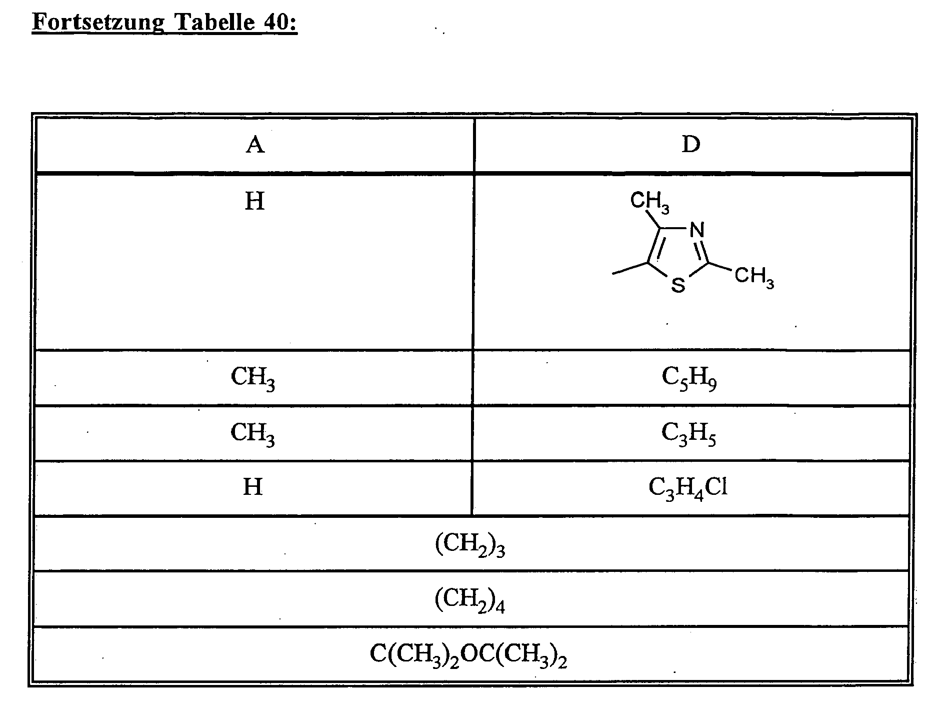

- **Tabelle 41:**: A und D haben die gleiche Bedeutung wie in Tabelle 40 mit V = H; W = CH₃; X = CH₃; Y = H; Z = Cl
- **Tabelle 42:**: A und D haben die gleiche Bedeutung wie in Tabelle 40 mit V = H; W = CH₃; X = CH₃; Y = CH₃; Z = Cl
- **Tabelle 43:**: A und D haben die gleiche Bedeutung wie in Tabelle 40 mit V = H; W = CH₃; X = CH₃; Y = CH₃; Z = F
- **Tabelle 44:**: A und D haben die gleiche Bedeutung wie in Tabelle 40 mit V = H; W = CH₃; X = CH₃; Y = CH₃; Z = Br
- **Tabelle 45:**: A und D haben die gleiche Bedeutung wie in Tabelle 40 mit V = CH₃; W = CH₃; X = CH₃; Y = H; Z = CH₃
- **Tabelle 46:**: A und D haben die gleiche Bedeutung wie in Tabelle 40 mit V = CH₃; W = CH₃; X = H; Y = CH₃; Z = CH₃
- **Tabelle 47:**: A und D haben die gleiche Bedeutung wie in Tabelle 40 mit V = H; W = CH₃; X = CH₃; Y = H; Z = Br
- **Tabelle 48:**: A und D haben die gleiche Bedeutung wie in Tabelle 40 mit V = H; W = Cl; X = Cl; Y = H; Z = Br
- **Tabelle 49:**: A und D haben die gleiche Bedeutung wie in Tabelle 40 mit V = H; W = Br; X = Br; Y, Z = -(CH₂)₃-
- **Tabelle 50:**: A und D haben die gleiche Bedeutung wie in Tabelle 40 mit V = H; W = CH₃; X=OCH₃; Y=H; Z = Br
- **Tabelle 51:**: A und D haben die gleiche Bedeutung wie in Tabelle 40 mit V = CH₃; W = CH₃; X = CH₃; Y = CH₃; Z = CH₃
- **Tabelle 52:**: A und D haben die gleiche Bedeutung wie in Tabelle 40 mit V = H; W = Cl; X = Cl; Y = Cl; Z = CH₃

Im einzelnen seien außer den bei den Herstellungsbeispielen genannten Verbindungen die folgenden Verbindungen der Formel (I-5-a) genannt:

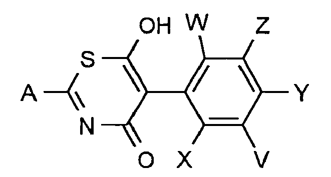

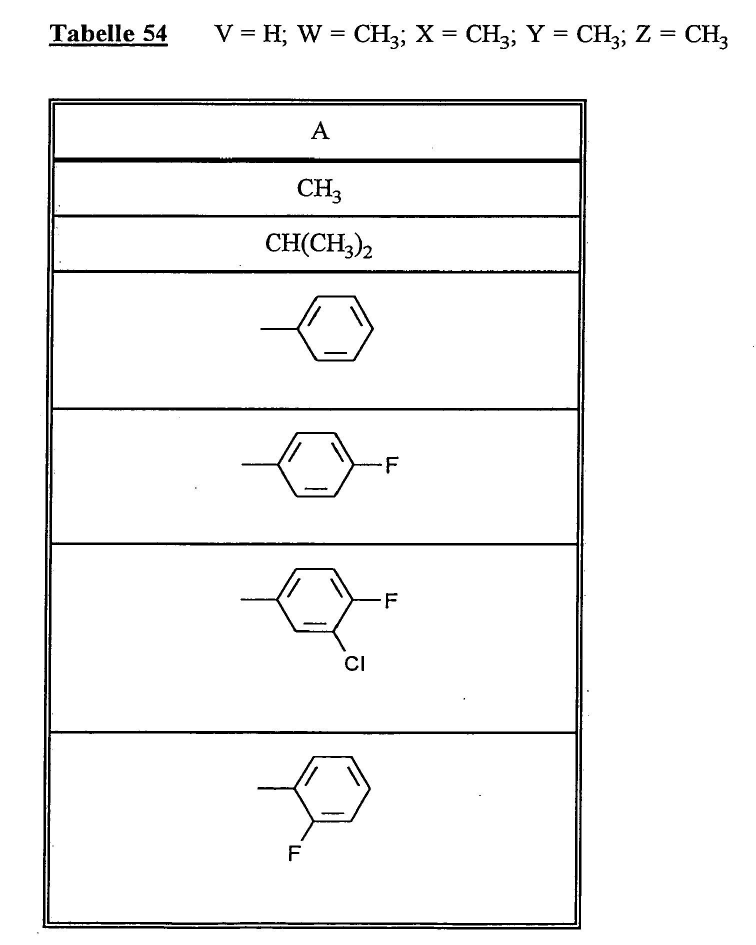

- **Tabelle 55:**: A hat die gleiche Bedeutung wie in Tabelle 54 mit V=H; W=CH₃; X=CH₃; Y=H; Z=Cl
- **Tabelle 56:**: A hat die gleiche Bedeutung wie in Tabelle 54 mit V = H; W = CH₃, X = CH₃; Y = CH₃; Z = Cl

Verwendet man gemäß Verfahren (A) N-[(3,4-Dichlor-2,6-dimethyl)-phenylacetyl]-1-amino-4-ethyl-cyclohexan-carbonsäureethylester als Ausgangsstoff, so kann der Verlauf des erfindungsgemäßen Verfahrens durch folgendes Reaktionsschema wiedergegeben werden:

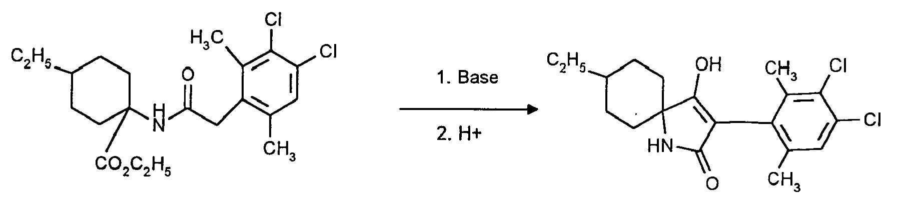

Verwendet man gemäß Verfahren (B) O-[(2,5-Dichlor-6-methyl)-phenylacetyl]-hydroxyessigsäureethylester, so kann der Verlauf des erfindungsgemäßen Verfahrens durch folgendes Reaktionsschema wiedergegeben werden:

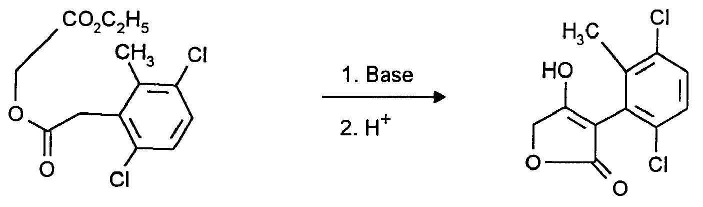

Verwendet man gemäß Verfahren (C) 2-[(2-Chlor-4,5,6-trimethyl)-phenyl]-4-(4-methoxy)-benzylmercapto-4-methyl-3-oxo-valeriansäure-ethylester, so kann der Verlauf des erfindungsgemäßen Verfahrens durch folgendes Reaktionsschema wiedergegeben werden:

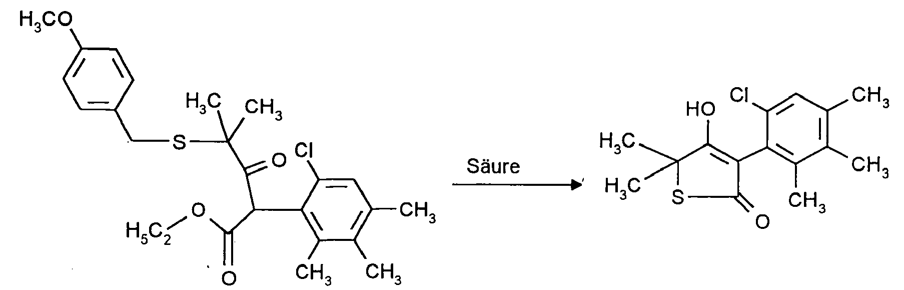

Verwendet man beispielsweise gemäß Verfahren (D) (Chlorcarbonyl)-2-[(3,4-Dichlor-2,6-dimethyl)-phenyl]-keten und Aceton als Ausgangsverbindungen, so kann der Verlauf des erfindungsgemäßen Verfahrens durch das folgende Reaktionsschema wiedergegeben werden:

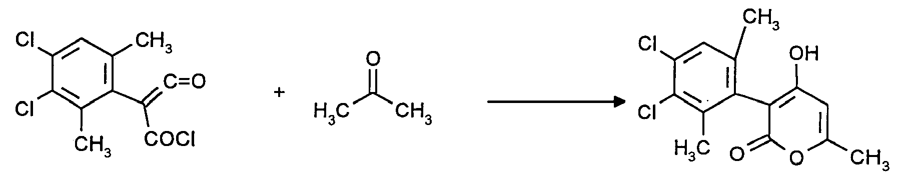

Verwendet man beispielsweise gemäß Verfahren (E) (Chlorcarbonyl)-2-[(2,3,4,6-tetramethyl)-phenyl]-keten und Thiobenzamid als Ausgangsverbindungen, so kann der Verlauf des erfindungsgemäßen Verfahrens durch das folgende Reaktionsschema wiedergegeben werden:

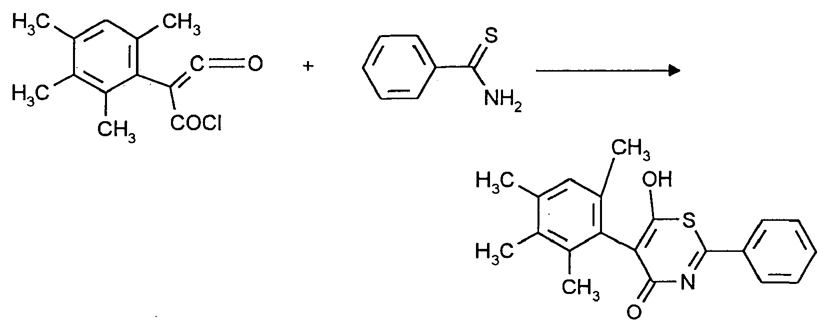

Verwendet man gemäß Verfahren (Fα) 3-[(2,5-Dichlor-4,6-dimethyl)-phenyl]-5,5-dimethyl-pyrrolidin-2,4-dion und Pivaloylchlorid als Ausgangsstoffe, so kann der Verlauf des erfindungsgemäßen Verfahrens durch folgendes Reaktionsschema wiedergegeben werden:

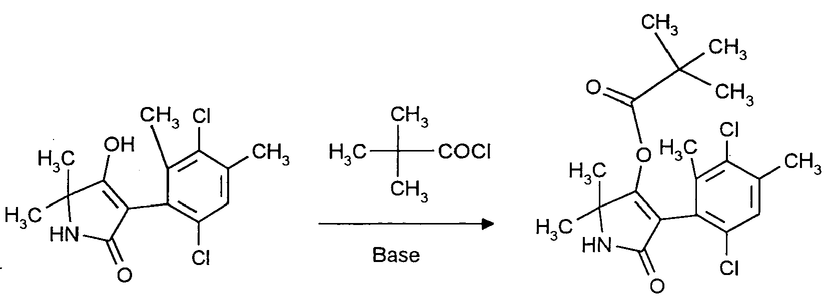

Verwendet man gemäß Verfahren (F) (Variante β) 3-[(2,3-Dichlor)-phenyl]-4-hydroxy-5-phenyl-Δ³-dihydrofuran-2-on und Acetanhydrid als Ausgangsverbindungen, so kann der Verlauf des erfindungsgemäßen Verfahrens durch folgendes Reaktionsschema wiedergegeben werden:

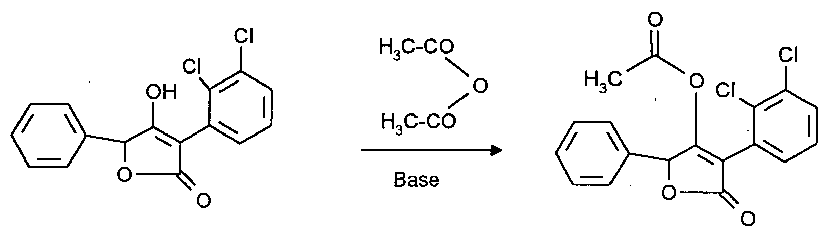

Verwendet man gemäß Verfahren (G) 8-[(2,4-Dichlor-3-methyl)-phenyl]-5,5-pentamethylen-pyrrolidin-2,4-dion und Chlorameisensäureethoxyethylester als Ausgangsverbindungen, so kann der Verlauf des erfindungsgemäßen Verfahrens durch folgendes Reaktionsschema wiedergegeben werden:

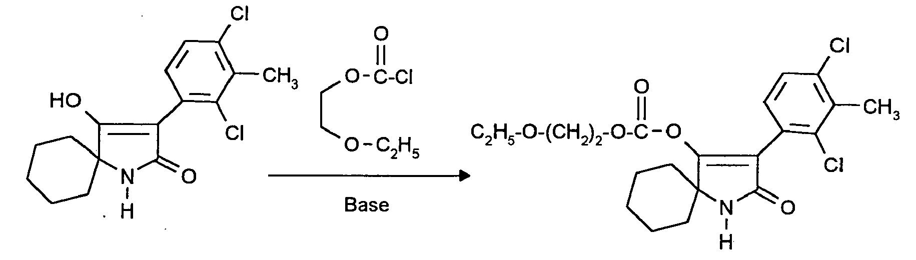

Verwendet man gemäß Verfahren (H), (Variante α) 3-[(2,6-Dibrom-3,4-dimethyl)-phenyl]-4-hydroxy-6-(3-pyridyl)-pyron und Chlormonothioameisensäuremethylester als Ausgangsprodukte, so kann der Reaktionsverlauf folgendermaßen wiedergegeben werden:

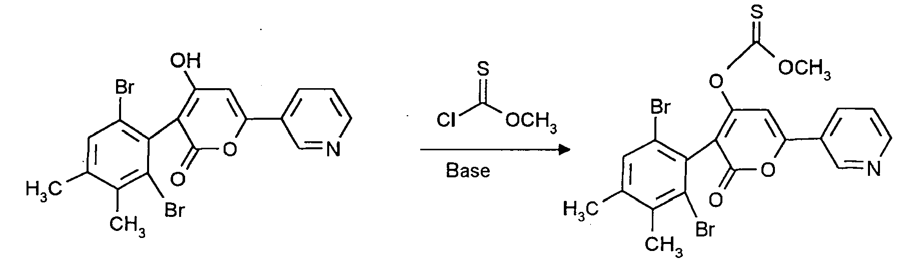

Verwendet man gemäß Verfahren (H), (Variante β) 5-[(3-Chlor-2,6-dimethyl)-phenyl]-6-hydroxy-2-(4-chlorphenyl)-thiazin-4-on, Schwefelkohlenstoff und Methyliodid als Ausgangskomponenten, so kann der Reaktionsverlauf wie folgt wiedergegeben werden:

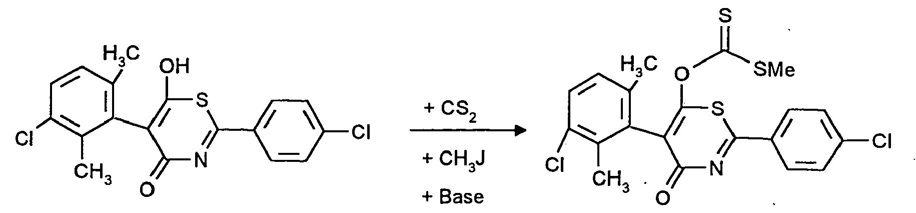

Verwendet man gemäß Verfahren (I) 2-[(2,3,4,6-Tetramethyl)-phenyl]-5,5[-(3-methyl)pentamethylen]-pyrrolidin-2,4-dion und Methansulfonsäurechlorid als Ausgangsprodukt, so kann der Reaktionsverlauf durch folgendes Reaktionsschema wiedergegeben werden:

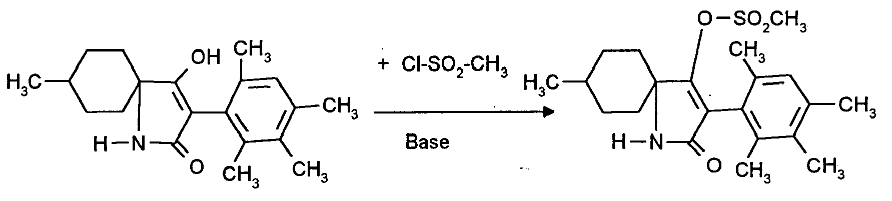

Verwendet man gemäß Verfahren (J) 2-[(2-Chlor-5,6-dimethyl)-phenyl]-4-hydroxy-5-methyl-6-(2-pyridyl)-pyron und Methanthio-phosphonsäurechlorid-(2,2,2-trifluorethylester) als Ausgangsprodukte, so kann der Reaktionsverlauf durch folgendes Reaktionsschema wiedergegeben werden:

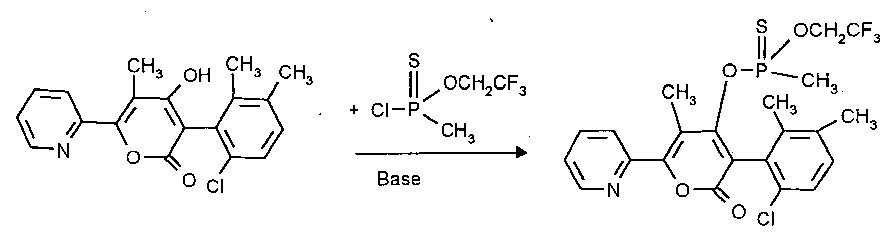

Verwendet man gemäß Verfahren (K) 3-[(2,4,5-Trichlor)-6-methylphenyl]-5-cyclopropyl-5-methyl-pyrrolidin-2,4-dion und NaOH als Komponenten, so kann der Verlauf des erfindungsgemäßen Verfahrens durch folgendes Reaktionsschema wiedergegeben werden:

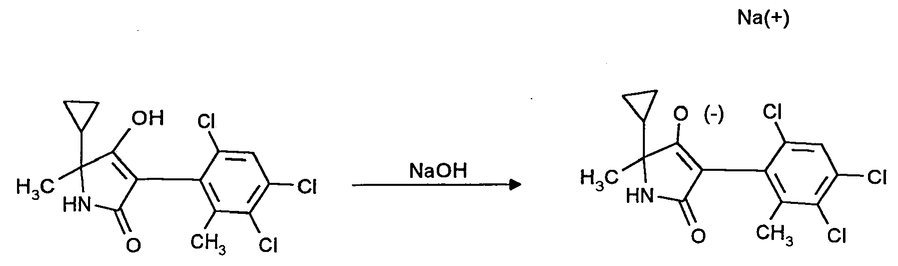

Verwendet man gemäß Verfahren (L) (Variante α) 3-[(2,6-Dichlor-4-brom-3-methyl)-phenyl]-4-hydroxy-5,5-tetramethylen-Δ³-dihydro-furan-2-on und Ethylisocyanat als Ausgangsprodukte, so kann der Reaktionsverlauf durch folgendes Reaktionsschema wiedergegeben werden:

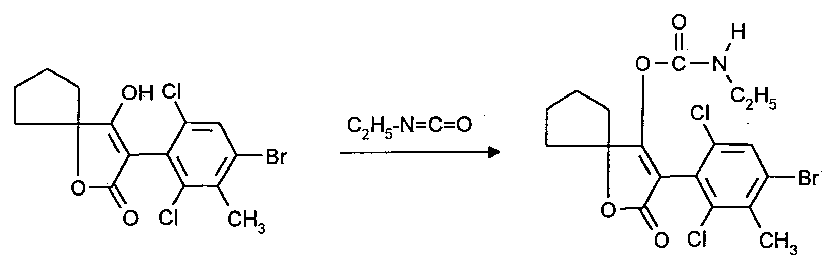

Verwendet man gemäß Verfahren (L) (Variante ß) 3-[(2-Chlor-5,6-dimethyl)-phenyl]-5-methyl-pyrrolidin-2,4-dion und Dimethylcarbamidsäurechlorid als Ausgangsprodukte, so kann der Reaktionsverlauf durch folgendes Schema wiedergegeben werden:

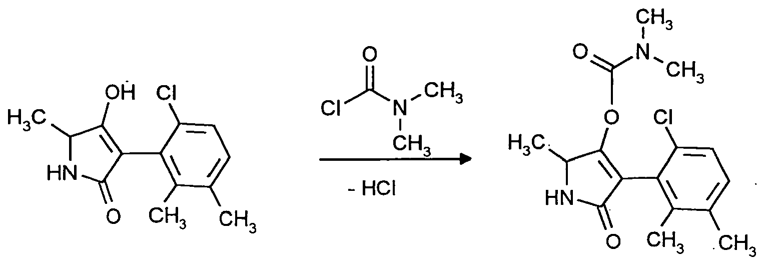

Die beim erfindungsgemäßen Verfahren (A) als Ausgangsstoffe benötigten Verbindungen der Formel (II) 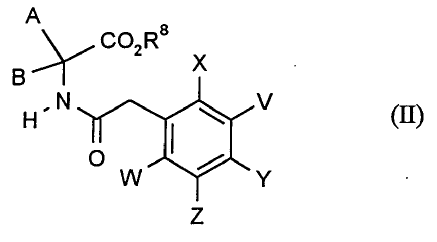 in welcher
- A, B, V, W, X, Y, Z und R⁸: die oben angegebenen Bedeutungen haben,
sind neu.

Man erhält die Acylaminosäureester der Formel (II) beispielsweise, wenn man Aminosäurederivate der Formel (XIX) 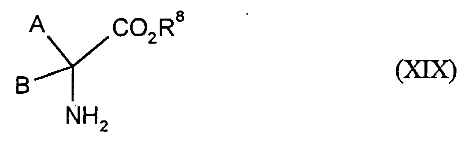 in welcher
- A, B und R⁸: die oben angegebenen Bedeutungen haben,
mit substituierten Phenylessigsäurehalogeniden der Formel (XX) 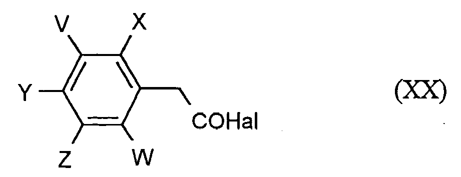 in welcher
- V, W, X, Y und Z: die oben angegebenen Bedeutungen haben und
- Hal: für Chlor oder Brom steht,
acyliert (Chem. Reviews 52, 237-416 (1953); Bhattacharya, Indian J. Chem. 6, 341-5, 1968)
oder wenn man Acylaminosäuren der Formel (XXI) 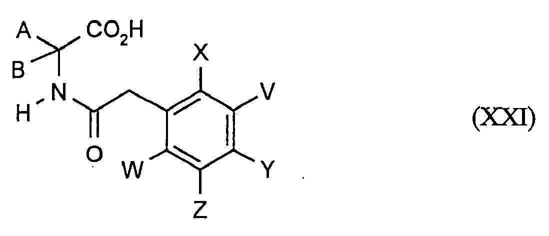 in welcher
- A, B, V, W, X, Y und Z: die oben angegebenen Bedeutungen haben,
verestert (Chem. Ind. (London) 1568 (1968)).

Die Verbindungen der Formel (XXI) 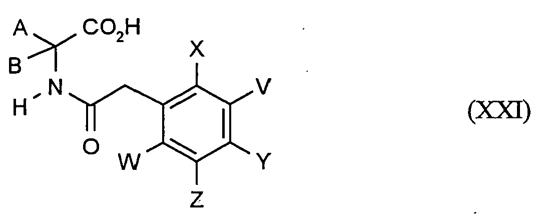 in welcher
- A, B, V, W, X, Y und Z: die oben angegebenen Bedeutungen haben,
sind neu.

Man erhält die Verbindungen der Formel (XXI), wenn man Aminosäuren der Formel (XXII) 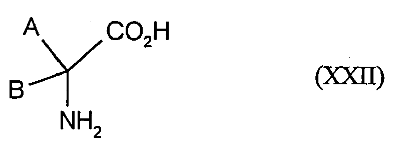 in welcher
- A und B: die oben angegebenen Bedeutungen haben,
mit substituierten Phenylessigsäurehalogeniden der Formel (XX) 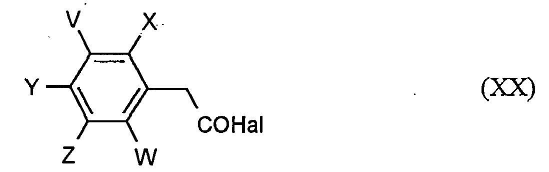 in welcher
- V, W, X, Y und Z: die oben angegebenen Bedeutungen haben und
- Hal: für Chlor oder Brom steht,
nach Schotten-Baumann acyliert (Organikum, VEB Deutscher Verlag der Wissenschaften, Berlin 1977, S. 505).

Die Verbindungen der Formel (XX) sind teilweise neu und lassen sich nach bekannten Verfahren herstellen.

Man erhält die Verbindungen der Formel (XX) beispielsweise, indem man substituierte Phenylessigsäuren der Formel (XXIII) 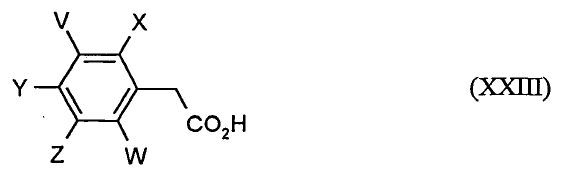 in welcher
- V, W, X, Y und Z: die oben angegebene Bedeutung haben,
mit Halogenierungsmitteln (z.B. Thionylchlorid, Thionylbromid, Oxalylchlorid, Phosgen, Phosphortrichlorid, Phosphortribromid oder Phosphorpentachlorid) gegebenenfalls in Gegenwart eines Verdünnungsmittels (z.B. gegebenenfalls chlorierten aliphatischen oder aromatischen Kohlenwasserstoffen wie Toluol oder Methylenchlorid) bei Temperaturen von -20°C bis 150°C, bevorzugt von -10°C bis 100°C, umsetzt.

Die Verbindungen der Formel (XXIII) sind teilweise neu, sie lassen sich nach literaturbekannten Verfahren herstellen (Organikum 15. Auflage, S. 533, VEB Deutscher Verlag der Wissenschaften, Berlin 1977). Man erhält die Verbindungen der Formel (XXIII) beispielsweise, indem man substituierte Phenylessigsäureester der Formel (XXIV) 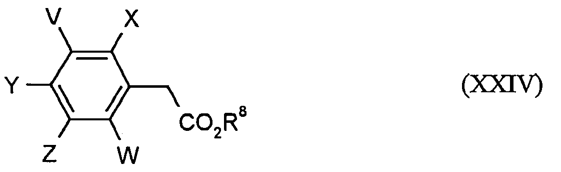 in welcher
- V, W, X, Y, Z und R⁸: die oben angegebene Bedeutung haben,
in Gegenwart einer Säure (z.B. einer anorganischen Säure wie Chlorwasserstoffsäure) oder einer Base (z.B. eines Alkalihydroxids wie Natrium- oder Kaliumhydroxid) und gegebenenfalls eines Verdünnungsmittels (z.B. eines wässrigen Alkohols wie Methanol oder Ethanol) bei Temperaturen zwischen 0°C und 150°C, bevorzugt zwischen 20°C und 100°C, hydrolysiert.

Die Verbindungen der Formel (XXIV) sind teilweise neu, sie lassen sich nach im Prinzip bekannten Verfahren herstellen.

Man erhält die Verbindungen der Formel (XXIV) beispielsweise, indem man substituierte 1,1,1-Trichlor-2-phenylethane der Formel (XXV) 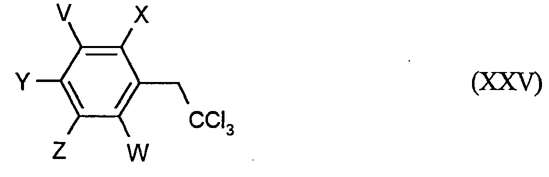 in welcher
- V, W, X, Y und Z: die oben angegebene Bedeutung haben,
zunächst mit Alkoholaten (z.B. Alkalimetallalkoholaten wie Natriummethylat oder Natriumethylat) in Gegenwart eines Verdünnungsmittels (z.B. dem vom Alkoholat abgeleiteten Alkohol) bei Temperaturen zwischen 0°C und 150°C, bevorzugt zwischen 20°C und 120°C, und anschließend mit einer Säure (bevorzugt eine anorganische Säure wie z.B. Schwefelsäure) bei Temperaturen zwischen -20°C und 150°C, bevorzugt 0°C und 100°C, umsetzt (vgl. DE 3 314 249).

Die Verbindungen der Formel (XXV) sind teilweise neu, sie lassen sich nach im Prinzip bekannten Verfahren herstellen.

Man erhält die Verbindungen der Formel (XXV) beispielsweise, wenn man Aniline der Formel (XXVI) 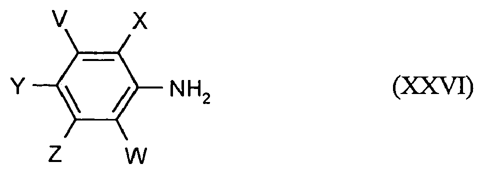 in welcher
- V, W, X, Y und Z: die oben angegebene Bedeutung haben,
in Gegenwart eines Alkylnitrits der Formel (XXVII)

R²¹-ONO (XXVII)

in welcher
- R²¹: für Alkyl, bevorzugt C₁-C₆-Alkyl steht,
in Gegenwart von Kupfer(II)-chlorid und gegebenenfalls in Gegenwart eines Verdünnungsmittels (z.B. eines aliphatischen Nitrils wie Acetonitril) bei einer Temperatur von -20°C bis 80°C, bevorzugt 0°C bis 60°C, mit Vinylidenchlorid (CH₂=CCl₂) umsetzt.

Die Verbindungen der Formel (XXVI) sind teilweise bekannte Verbindungen oder lassen sich nach im Prinzip bekannten Verfahren herstellen. Die Verbindungen der Formel (XXVII) sind bekannte Verbindungen der Organischen Chemie. Kupfer(II)-chlorid und Vinylidenchlorid sind lange bekannt und käuflich.

Die Verbindungen der Formel (XIX) und (XXII) sind teilweise bekannt und/oder lassen sich nach bekannten Verfahren darstellen (siehe z.B. Compagnon, Miocque Ann. Chim. (Paris) [14] 5, S. 11-22, 23-27 (1970)).

Die substituierten cyclischen Aminocarbonsäuren der Formel (XXIIa), in der A und B einen Ring bilden, sind im allgemeinen nach der Bucherer-Bergs-Synthese oder nach der Strecker-Synthese erhältlich und fallen dabei jeweils in unterschiedlichen Isomerenformen an. So erhält man nach den Bedingungen der Bucherer-Bergs-Synthese vorwiegend die Isomeren (im folgenden der Einfachheit halber als β bezeichnet), in welchen die Reste R und die Carboxylgruppe äquatorial stehen, während nach den Bedingungen der Strecker-Synthese vorwiegend die Isomeren (im folgenden der Einfachheit halber als α bezeichnet) anfallen, bei denen die Aminogruppe und die Reste R äquatorial stehen. 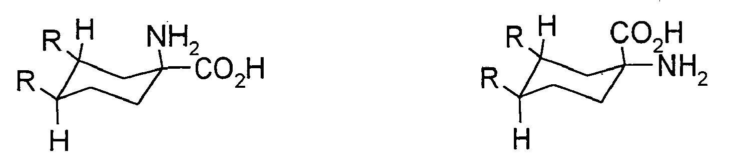
- Bucherer-Bergs-Synthese: Strecker-Synthese
- (β-Isomeres): (α-Isomeres)

(L. Munday, J. Chem. Soc. 4372 (1961); J.T. Eward, C. Jitrangeri, Can. J. Chem. 53, 3339 (1975).

Weiterhin lassen sich die bei dem obigen Verfahren (A) verwendeten Ausgangsstoffe der Formel (II) 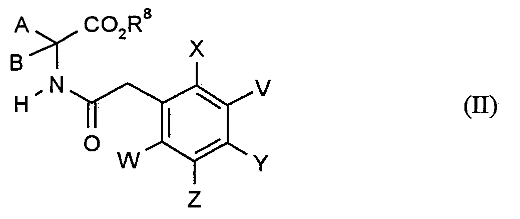 in welcher
- A, B, V, W, X, Y, Z und R⁸: die oben angegebenen Bedeutungen haben,
herstellen, wenn man Aminonitrile der Formel (XXVIII) 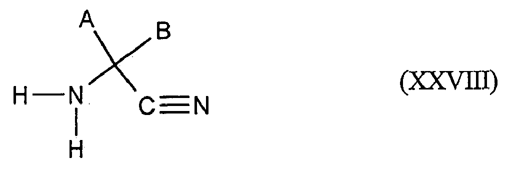 in welcher
- A und B: die oben angegebenen Bedeutungen haben,
mit substituierten Phenylessigsäurehalogeniden der Formel (XX) 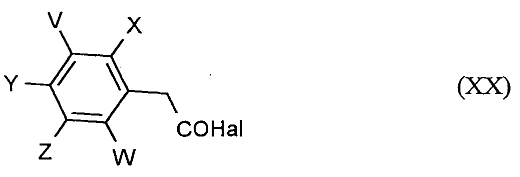 in welcher
- V, W, X, Y, Z und Hal: die oben angegebenen Bedeutungen haben,
zu Verbindungen der Formel (XXIX) 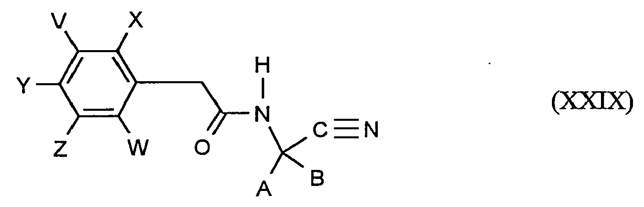 in welcher
- A, B, V, W, X, Y und Z: die oben angegebenen Bedeutungen haben,
umsetzt,
und diese anschließend einer sauren Alkoholyse unterwirft.

Die Verbindungen der Formel (XXIX) sind ebenfalls neu.

Die bei dem erfindungsgemäßen Verfahren (B) als Ausgangstoffe benötigten Verbindungen der Formel (III) 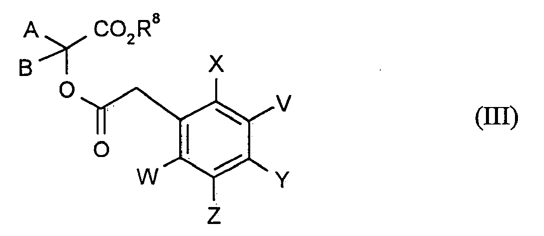 in welcher
- A, B, V, W, X, Y, Z und R⁸: die oben angegebenen Bedeutungen haben,
sind neu.

Sie lassen sich nach im Prinzip bekannten Methoden in einfacher Weise herstellen.

Man erhält die Verbindungen der Formel (III) beispielsweise, wenn man

2-Hydroxycarbonsäureester der Formel (XXX) 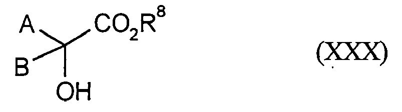 in welcher
- A, B und R⁸: die oben angegebenen Bedeutungen haben,
mit substituierten Phenylessigsäurehalogeniden der Formel (XX) 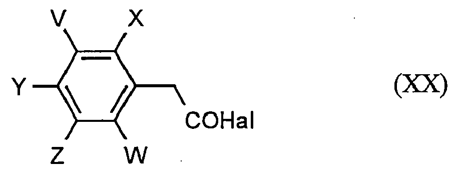 in welcher
- V, W, X, Y, Z und Hal: die oben angegebenen Bedeutungen haben,
acyliert (Chem. Reviews 52, 237-416 (1953)).

Weiterhin erhält man Verbindungen der Formel (III), wenn man
substituierte Phenylessigsäuren der Formel (XXIII) 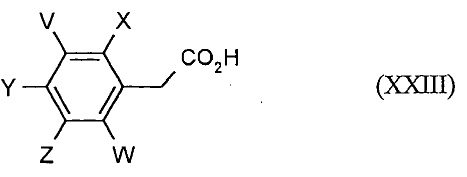 in welcher
- V, W, X, Y und Z: die oben angegebenen Bedeutungen haben,
mit α-Halogencarbonsäureestern der Formel (XXXI) 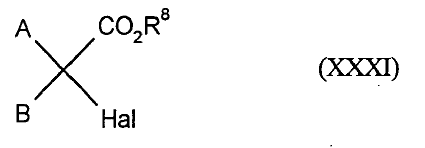 in welcher
- A, B und R⁸: die oben angegebenen Bedeutungen haben und
- Hal: für Chlor oder Brom steht,
alkyliert.

Die Verbindungen der Formel (XXXI) sind käuflich.

Die bei dem obigen Verfahren (C) als Ausgangsstoffe benötigten Verbindungen der Formel (IV) 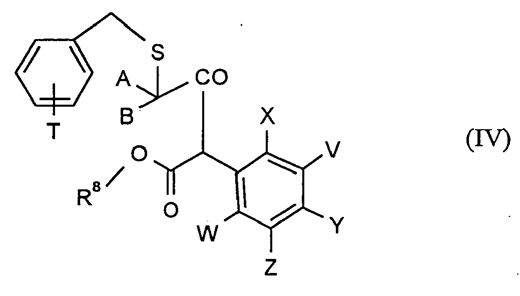 in welcher
- A, B, T, V, W, X, Y, Z und R⁸: die oben angegebenen Bedeutungen haben,
sind neu.

Sie lassen sich nach im Prinzip bekannten Methoden herstellen.

Man erhält die Verbindungen der Formel (IV) beispielsweise, wenn man
substituierte Phenylessigsäureester der Formel (XXIV) 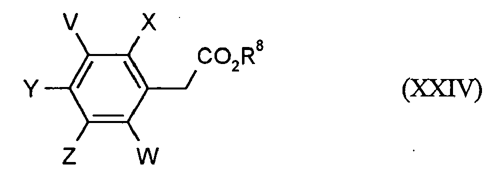 in welcher
- V, W, X, Y, R⁸ und Z: die oben angegebenen Bedeutungen haben,
mit 2-Benzylthio-carbonsäurehalogeniden der Formel (XXXII) 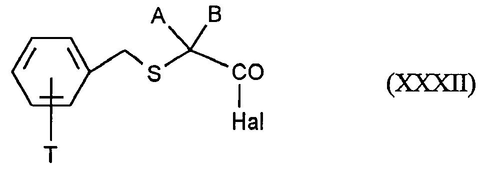 in welcher
- A, B und T: die oben angegebenen Bedeutungen haben und
- Hal: für Halogen (insbesondere Chlor oder Brom) steht,
in Gegenwart von starken Basen acyliert (siehe z.B. M.S. Chambers, E.J. Thomas, D.J. Williams, J. Chem. Soc. Chem. Commun., (1987), 1228).

Die Benzylthio-carbonsäurehalogenide der Formel (XXXII) sind teilweise bekannt und/oder lassen sich nach bekannten Verfahren herstellen (J. Antibiotics (1983), 26, 1589).

Die beim Verfahren (D) als Ausgangsstoffe benötigten Halogencarbonylketene der Formel (VI) sind neu. Sie lassen sich nach im Prinzip bekannten Methoden in einfacher Weise herstellen (vgl. beispielsweise Org. Prep. Proced. Int., 7, (4), 155-158, 1975 und DE 1 945 703). Man erhält die Verbindungen der Formel (VI) 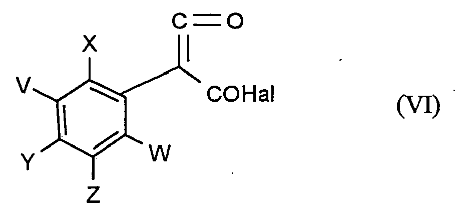 in welcher
- V, W, X, Y und Z: die oben angegebenen Bedeutungen haben und
- Hal: für Chlor oder Brom steht,
wenn man
substituierte Phenylmalonsäuren der Formel (XXXIII) 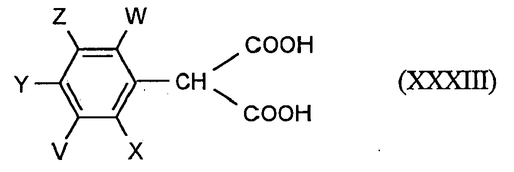 in welcher
- V, W, X, Y und Z: die oben angegebenen Bedeutungen haben,
mit Säurehalogeniden, wie beispielsweise Thionylchlorid, Phosphor(V)chlorid, Phosphor(III)chlorid, Oxalylchlorid, Phosgen oder Thionylbromid gegebenenfalls in Gegenwart von Katalysatoren, wie beispielsweise Diethylformamid, Methyl-Sterylformamid oder Triphenylphosphin und gegebenenfalls in Gegenwart von Basen wie z.B. Pyridin oder Triethylamin, bei einer Temperatur zwischen -20°C und 200°C, bevorzugt zwischen 0°C und 150°C, umsetzt.

Die substituierten Phenylmalonsäuren der Formel (XXXIII) sind neu. Sie lassen sich aber in einfacher Weise nach bekannten Verfahren herstellen (vgl. z.B. Organikum, VEB Deutscher Verlag der Wissenschaften, Berlin 1977, S. 517 ff), z.B. aus substituierten Phenylmalonsäureestern der Formel (XXXIV) 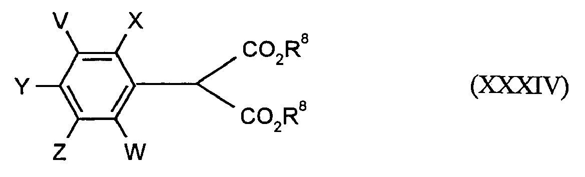 in welcher
- V, W, X, Y, Z und R⁸: die oben angegebene Bedeutung haben,
durch Verseifung erhalten.

Die für das erfindungsgemäße Verfahren (E) als Ausgangsstoffe benötigten Carbonylverbindungen der Formel (V) oder deren Silylenolether der Formel (Va) 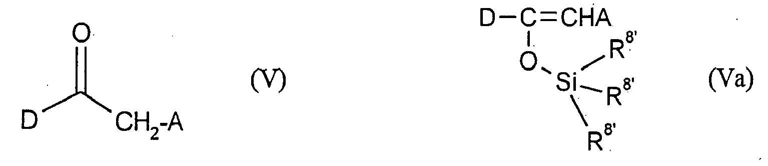 in welchen
- A, D und R^{8'}: die oben angegebenen Bedeutungen haben,
sind käufliche, allgemeine bekannte oder nach bekannten Verfahren zugängliche Verbindungen.

Die Herstellung der zur Durchführung des erfindungsgemäßen Verfahrens (E) als Ausgangsstoffe benötigten Ketensäurechloride der Formel (VI) wurde bereits beim erfindungsgemäßen Verfahren (D) beschrieben. Die zur Durchführung des erfindungsgemäßen Verfahrens (E) benötigten Thioamide der Formel (VII) 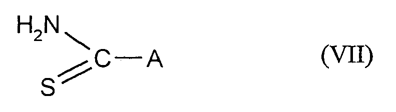 in welcher
- A: die oben angegebene Bedeutung hat,
sind allgemein in der Organischen Chemie bekannte Verbindungen.

Die Malonsäureester der Formel (XXXIV) 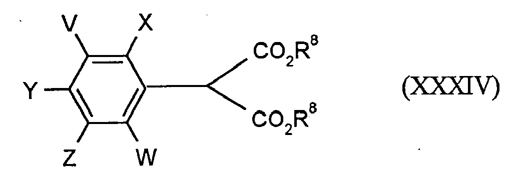 in welcher
- R⁸, V, W, X, Y und Z: die oben angegebenen Bedeutungen haben,
sind neu und lassen sich nach allgemein bekannten Methoden der Organischen Chemie darstellen (vgl. z.B. Tetrahedron Lett. 27, 2763 (1986) und Organikum VEB Deutscher Verlag der Wissenschaften, Berlin 1977, S. 587 ff.).

Die zur Durchführung der erfindungsgemäßen Verfahren (F), (G), (H), (I), (J), (K) und (L) außerdem als Ausgangsstoffe benötigten Säurehalogenide der Formel (VIII), Carbonsäureanhydride der Formel (IX), Chlorameisensäureester oder Chlorameisensäurethioester der Formel (X), Chlormonothioameisensäureester oder Chlordithioameisensäureester der Formel (XI), Alkylhalogenide der Formel (XII), Sulfonsäurechloride der Formel (XIII), Phosphorverbindungen der Formel (XIV) und Metallhydroxide, Metallalkoxide oder Amine der Formel (XV) und (XVI) und Isocyanate der Formel (XVII) und Carbamidsäurechloride der Formel (XVIII) sind allgemein bekannte Verbindungen der organischen bzw. anorganischen Chemie.

Die Verbindungen der Formeln (V), (VII) bis (XVIII), (XIX), (XXII), (XXVIII), (XXX), (XXXI), (XXXII), (XXXIII) und (XXXIV) sind darüber hinaus aus den eingangs zitierten Patentanmeldungen bekannt und/oder lassen sich nach den dort angegebenen Methoden herstellen.

Das Verfahren (A) ist dadurch gekennzeichnet, daß man Verbindungen der Formel (II), in welcher A, B, V, W, X, Y, Z und R⁸ die oben angegebenen Bedeutungen haben, in Gegenwart eines Verdünnungsmittels und in Gegenwart einer Base einer intramolekularen Kondensation unterwirft.

Als Verdünnungsmittel können bei dem erfindungsgemäßen Verfahren (A) alle gegenüber den Reaktionsteilnehmern inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon, sowie Alkohole wie Methanol, Ethanol, Propanol, Iso-Propanol, Butanol, Iso-Butanol und tert.-Butanol.

Als Base (Deprotonierungsmittel) können bei der Durchführung des erfindungsgemäßen Verfahrens (A) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natriummethylat, Natriumethylat und Kalium-tert.-butylat einsetzbar.

Die Reaktionstemperatur kann bei der Durchführung des erfindungsgemäßen Verfahrens (A) innerhalb eines größeren Bereiches variiert werden. Im allgemeinen arbeitet man bei Temperaturen zwischen -50°C und 250°C, vorzugsweise zwischen -30°C und 150°C.

Das erfindungsgemäße Verfahren (A) wird im allgemeinen unter Normaldruck durchgeführt.

Bei der Durchführung des erfindungsgemäßen Verfahrens (A) setzt man die Reaktionskomponente der Formel (II) und die deprotonierende Base im allgemeinen in äquimolaren bis etwa doppeltäquimolaren Mengen ein. Es ist jedoch auch möglich, die eine oder andere Komponente in einem größeren Überschuß (bis zu 3 Mol) zu verwenden.

Das Verfahren (B) ist dadurch gekennzeichnet, daß man Verbindungen der Formel (III), in welcher A, B, V, W, X, Y, Z und R⁸ die oben angegebenen Bedeutungen haben, in Gegenwart eines Verdünnungsmittels und in Gegenwart einer Base intramolekular kondensiert.

Als Verdünnungsmittel können bei dem erfindungsgemäßen Verfahren (B) alle gegenüber den Reaktionsteilnehmern inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon. Weiterhin können Alkohole wie Methanol, Ethanol, Propanol, Iso-Propanol, Butanol, Iso-Butanol und tert.-Butanol eingesetzt werden.

Als Base (Deprotonierungsmittel) können bei der Durchführung des erfindungsgemäßen Verfahrens (B) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natriummethylat, Natriumethylat und Kalium-tert.-butylat einsetzbar.

Die Reaktionstemperatur kann bei der Durchführung des erfindungsgemäßen Verfahrens (B) innerhalb eines größeren Bereiches variiert werden. Im allgemeinen arbeitet man bei Temperaturen zwischen -50°C und 250°C, vorzugsweise zwischen -30°C und 150°C.

Das erfindungsgemäße Verfahren (B) wird im allgemeinen unter Normaldruck durchgeführt.

Bei der Durchführung des erfindungsgemäßen Verfahrens (B) setzt man die Reaktionskomponenten der Formel (III) und die deprotonierenden Basen im allgemeinen in etwa äquimolaren Mengen ein. Es ist jedoch auch möglich, die eine oder andere Komponente in einem größeren Überschuß (bis zu 3 Mol) zu verwenden.

Das Verfahren (C) ist dadurch gekennzeichnet, daß man Verbindungen der Formel (IV) in welcher A, B, T, V, W, X, Y, Z und R⁸ die oben angegebene Bedeutung haben, in Gegenwart einer Säure und gegebenenfalls in Gegenwart eines Verdünnungsmittels intramolekular cyclisiert.

Als Verdünnungsmittel können bei dem erfindungsgemäßen Verfahren (C) alle gegenüber den Reaktionsteilnehmern inerten organischen Solventien eingesetzt werden. Vorzugsweise verwendbar sind Kohlenwasserstoffe, wie Toluol und Xylol, ferner halogenierte Kohlenwasserstoffe wie Dichlormethan, Chloroform, Ethylenchlorid, Chlorbenzol, Dichlorbenzol, außerdem polare Lösungsmittel, wie Dimethylsulfoxid, Sulfolan, Dimethylformamid und N-Methyl-pyrrolidon. Weiterhin können Alkohole wie Methanol, Ethanol, Propanol, iso-Propanol, Butanol, Iso-butanol, tert.-Butanol eingesetzt werden.

Gegebenenfalls kann auch die eingesetzte Säure als Verdünnungsmittel dienen.

Als Säure können bei dem erfindungsgemäßen Verfahren (C) alle üblichen anorganischen und organischen Säuren eingesetzt werden, wie z.B. Halogenwasserstoffsäuren, Schwefelsäure, Alkyl-, Aryl- und Haloalkylsulfonsäuren, insbesondere werden halogenierte Alkylcarbonsäuren wie z.B. Trifluoressigsäure verwendet.

Die Reaktionstemperatur kann bei der Durchführung des erfindungsgemäßen Verfahrens (C) innerhalb eines größeren Bereiches variiert werden. Im allgemeinen arbeitet man bei Temperaturen zwischen -30°C und 250°C, vorzugsweise zwischen 0°C und 150°C.

Das erfindungsgemäße Verfahren (C) wird im allgemeinen unter Normaldruck durchgeführt.

Bei der Durchführung des erfindungsgemäßen Verfahrens (C) setzt man die Reaktionskomponenten der Formeln (IV) und die Säure z.B. in äquimolaren Mengen ein. Es ist jedoch gegebenenfalls auch möglich, die Säure in katalytischen Mengen einzusetzen.

Das erfindungsgemäße Verfahren (D) ist dadurch gekennzeichnet, daß man Carbonylverbindungen der Formel (V) oder deren Silylenolether der Formel (Va), in welchen A und B die oben angegebene Bedeutung haben, mit Ketensäurehalogeniden der Formel (VI), in welcher V, W, X, Y und Z die oben angegebene Bedeutung haben, gegebenenfalls in Gegenwart eines Verdünnungsmittels und gegebenenfalls in Gegenwart eines Säureakzeptors umsetzt.

Als Verdünnungsmittel können bei dem erfindungsgemäßen Verfahren (D) alle gegenüber den Reaktionsteilnehmern inerten organischen Solventien eingesetzt werden. Vorzugsweise verwendbar sind Kohlenwasserstoffe, wie o-Dichlorbenzol, Tetralin, Toluol und Xylol, ferner Ether, wie Dibutylether, Glykoldimethylether und Diglykoldimethylether, außerdem polare Lösungsmittel, wie Dimethylsulfoxid, Sulfolan, Dimethylformamid oder N-Methyl-pyrrolidon.

Als Säureakzeptoren können bei der Durchführung des erfindungsgemäßen Verfahrens (D) alle üblichen Säureakzeptoren verwendet werden.

Vorzugsweise verwendbar sind tertiäre Amine, wie Triethylamin, Pyridin, Diazabicyclooctan (DABCO), Diazabicycloundecen (DBU), Diazabicyclononen (DBN), Hünig-Base oder N,N-Dimethyl-anilin.

Die Reaktionstemperatur kann bei der Durchführung des erfindungsgemäßen Verfahrens (D) innerhalb eines größeren Bereiches variiert werden. Zweckmäßigerweise arbeitet man bei Temperaturen zwischen 0°C und 250°C, vorzugsweise zwischen 50°C und 220°C.

Das erfindungsgemäße Verfahren (D) wird vorzugsweise unter Normaldruck durchgeführt.

Bei der Durchführung des erfindungsgemäßen Verfahrens (D) setzt man die Reaktionskomponenten der Formeln (V) und (VI) und gegebenenfalls den Säureakzeptor im allgemeinen in etwa äquimolaren Mengen ein. Es ist jedoch auch möglich, die eine oder andere Komponente in einem größeren Überschuß (bis zu 5 Mol) zu verwenden.

Das erfindungsgemäße Verfahren (E) ist dadurch gekennzeichnet, daß man Thioamide der Formel (VII), in welcher A die oben angegebene Bedeutung hat, mit Ketensäurehalogeniden der Formel (VI), in welcher V, W, X, Y und Z die oben angegebene Bedeutung haben, gegebenenfalls in Gegenwart eines Verdünnungsmittels und gegebenenfalls in Gegenwart eines Säureakzeptors umsetzt.

Als Verdünnungsmittel können bei der erfindungsgemäßen Verfahrensvariante (E) alle inerten organischen Solventien eingesetzt werden. Vorzugsweise verwendbar sind Kohlenwasserstoffe, wie o-Dichlorbenzol, Tetralin, Toluol und Xylol, ferner Ether, wie Dibutylether, Glykoldimethylether und Diglykoldimethylether, außerdem polare Lösungsmittel, wie Dimethylsulfoxid, Sulfolan, Dimethylformamid und N-Methyl-pyrrolidon.

Als Säureakzeptoren können bei der Durchführung des erfindungsgemäßen Verfahrens (E) alle üblichen Säureakzeptoren verwendet werden.

Vorzugsweise verwendbar sind tertiäre Amine, wie Triethylamin, Pyridin, Diazabicyclooctan (DABCO), Diazabicycloundecan (DBU), Diazabicyclononen (DBN), Hünig-Base und N,N-Dimethyl-anilin.

Die Reaktionstemperatur kann bei der Durchführung des erfindungsgemäßen Verfahrens (E) innerhalb eines größeren Bereiches variiert werden. Zweckmäßigerweise arbeitet man bei Temperaturen zwischen 0°C und 250°C, vorzugsweise zwischen 20°C und 220°C.

Das erfindungsgemäße Verfahren (E) wird zweckmäßigerweise unter Normaldruck durchgeführt.

Bei der Durchführung des erfindungsgemäßen Verfahrens (E) setzt man die Reaktionskomponenten der Formeln (VII) und (VI) und gegebenenfalls die Säureakzeptoren im allgemeinen in etwa äquimolaren Mengen ein. Es ist jedoch auch möglich, die eine oder andere Komponente in einem größeren Überschuß (bis zu 5 Mol) zu verwenden.

Das Verfahren (Fα) ist dadurch gekennzeichnet, daß man Verbindungen der Formeln (I-1-a) bis (1-5-a) jeweils mit Carbonsäurehalogeniden der Formel (VIII) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (Fα) alle gegenüber den Säurehalogeniden inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüberhinaus Carbonsäureester, wie Ethylacetat, und auch stark polare Solventien, wie Dimethylformamid, Dimethylsulfoxid und Sulfolan. Wenn die Hydrolysestabilität des Säurehalogenids es zuläßt, kann die Umsetzung auch in Gegenwart von Wasser durchgeführt werden.

Als Säurebindemittel kommen bei der Umsetzung nach dem erfindungsgemäßen Verfahren (Fα) alle üblichen Säureakzeptoren in Betracht. Vorzugsweise verwendbar sind tertiäre Amine, wie Triethylamin, Pyridin, Diazabicyclooctan (DABCO), Diazabicycloundecen (DBU), Diazabicyclononen (DBN), Hünig-Base und N,N-Dimethyl-anilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat sowie Alkalihydroxide wie Natriumhydroxid und Kaliumhydroxid.

Die Reaktionstemperatur kann bei dem erfindungsgemäßen Verfahren (Fα) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (Fα) werden die Ausgangsstoffe der Formeln (I-1-a) bis (I-5-a) und das Carbonsäurehalogenid der Formel (VIII) im allgemeinen jeweils in angenähert äquivalenten Mengen verwendet. Es ist jedoch auch möglich, das Carbonsäurehalogenid in einem größeren Überschuß (bis zu 5 Mol) einzusetzen. Die Aufarbeitung erfolgt nach üblichen Methoden.

Das Verfahren (Fβ) ist dadurch gekennzeichnet, daß man Verbindungen der Formeln (I-1-a) bis (I-5-a) jeweils mit Carbonsäureanhydriden der Formel (IX) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (Fβ) vorzugsweise diejenigen Verdünnungsmittel verwendet werden, die auch bei der Verwendung von Säurehalogeniden vorzugsweise in Betracht kommen. Im übrigen kann auch ein im Überschuß eingesetztes Carbonsäureanhydrid gleichzeitig als Verdünnungsmittel fungieren.

Als gegebenenfalls zugesetzte Säurebindemittel kommen beim Verfahren (Fß) vorzugsweise diejenigen Säurebindemittel in Frage, die auch bei der Verwendung von Säurehalogeniden vorzugsweise in Betracht kommen.

Die Reaktionstemperatur kann bei dem erfindungsgemäßen Verfahren (Fβ) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (Fβ) werden die Ausgangsstoffe der Formeln (I-1-a) bis (I-5-a) und das Carbonsäureanhydrid der Formel (IX) im allgemeinen in jeweils angenähert äquivalenten Mengen verwendet. Es ist jedoch auch möglich, das Carbonsäureanhydrid in einem größeren Überschuß (bis zu 5 Mol) einzusetzen. Die Aufarbeitung erfolgt nach üblichen Methoden.

Im allgemeinen geht man so vor, daß man Verdünnungsmittel und im Überschuß vorhandenes Carbonsäureanhydrid sowie die entstehende Carbonsäure durch Destillation oder durch Waschen mit einem organischen Lösungsmittel oder mit Wasser entfernt.

Das Verfahren (G) ist dadurch gekennzeichnet, daß man Verbindungen der Formeln (I-1-a) bis (I-5-a) jeweils mit Chlorameisensäureestern oder Chlorameisensäurethiolestem der Formel (X) gegebenenfalls in Gegenwart eines Verdünnungsmittels und gegebenenfalls in Gegenwart eines Säurebindemittels umsetzt.

Als Säurebindemittel kommen bei dem erfindungsgemäßen Verfahren (G) alle üblichen Säureakzeptoren in Betracht. Vorzugsweise verwendbar sind tertiäre Amine, wie Triethylamin, Pyridin, DABCO, DBU, DBA, Hünig-Base und N,N-Dimethyl-anilin, ferner Erdalkalimetalloxide, wie Magnesium- und Calciumoxid, außerdem Alkali- und Erdalkalimetallcarbonate, wie Natriumcarbonat, Kaliumcarbonat und Calciumcarbonat sowie Alkalihydroxide wie Natriumhydroxid und Kaliumhydroxid.

Als Verdünnungsmittel können bei dem erfindungsgemäßen Verfahren (G) alle gegenüber den Chlorameisensäureestern bzw. Chlorameisensäurethiolestern inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, außerdem Nitrile wie Acetonitril und auch stark polare Solventien, wie Dimethylformamid, Dimethylsulfoxid und Sulfolan.

Die Reaktionstemperatur kann bei der Durchführung des erfindungsgemäßen Verfahrens (G) innerhalb eines größeren Bereiches variiert werden. Die Reaktionstemperatur liegt im allgemeinen zwischen -20°C und +100°C, vorzugsweise zwischen 0°C und 50°C.

Das erfindungsgemäße Verfahren (G) wird im allgemeinen unter Normaldruck durchgeführt.

Bei der Durchführung des erfindungsgemäßen Verfahrens (G) werden die Ausgangsstoffe der Formeln (I-1-a) bis (I-5-a) und der entsprechende Chlorameisensäureester bzw. Chlorameisensäurethiolester der Formel (X) im allgemeinen jeweils in angenähert äquivalenten Mengen verwendet. Es ist jedoch auch möglich, die eine oder andere Komponente in einem größeren Überschuß (bis zu 2 Mol) einzusetzen. Die Aufarbeitung erfolgt nach üblichen Methoden. Im allgemeinen geht man so vor, daß man ausgefallene Salze entfernt und das verbleibende Reaktionsgemisch durch Abziehen des Verdünnungsmittels einengt.

Das erfindungsgemäße Verfahren (H) ist dadurch gekennzeichnet, daß man Verbindungen der Formeln (I-1-a) bis (I-5-a) jeweils mit (Hα) Verbindungen der Formel (XI) in Gegenwart eines Verdünnungsmittels und gegebenenfalls in Gegenwart eines Säurebindemittels oder (Hβ) Schwefelkohlenstoff und anschließend mit Alkylhalogeniden der Formel (XII) gegebenenfalls in Gegennwart eines Verdünnungsmittels und gegebenenfalls in Gegenwart einer Base umsetzt.

Beim Herstellungsverfahren (Hα) setzt man pro Mol Ausgangsverbindung der Formeln (I-1-a) bis (I-5-a) ca. 1 Mol Chlormonothioameisensäureester bzw. Chlordithioameisensäureester der Formel (XI) bei 0 bis 120°C, vorzugsweise bei 20 bis 60°C um.

Als gegebenenfalls zugesetzte Verdünnungsmittel kommen alle inerten polaren organischen Lösungsmittel in Frage, wie Ether, Amide, Sulfone, Sulfoxide, aber auch Halogenalkane.

Vorzugsweise werden Dimethylsulfoxid, Tetrahydrofuran, Dimethylformamid oder Methylenchlorid eingesetzt.

Stellt man in einer bevorzugten Ausführungsform durch Zusatz von starken Deprotonierungsmitteln wie z.B. Natriumhydrid oder Kaliumtertiärbutylat das Enolatsalz der Verbindungen (I-1-a) bis (I-5-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin und Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Beim Herstellungsverfahren (Hβ) setzt man pro Mol Ausgangsverbindungen der Formeln (I-1-a) bis (I-5-a) jeweils die äquimolare Menge bzw. einen Überschuß Schwefelkohlenstoff zu. Man arbeitet hierbei vorzugsweise bei Temperaturen von 0 bis 50°C und insbesondere bei 20 bis 30°C.

Oft ist es zweckmäßig zunächst aus den Verbindungen der Formeln (I-1-a) bis (I-5-a) durch Zusatz einer Base (wie z.B. Kaliumtertiärbutylat oder Natriumhydrid) das entsprechende Salz herzustellen. Man setzt die Verbindungen (I-1-a) bis (I-5-a) jeweils so lange mit Schwefelkohlenstoff um, bis die Bildung der Zwischenverbindung abgeschlossen ist, z.B. nach mehrstündigem Rühren bei Raumtemperatur.

Als Basen können beim Verfahren (Hβ) alle üblichen Protonenakzeptoren eingesetzt werden. Vorzugsweise verwendbar sind Alkalimetallhydride, Alkalimetallalkoholate, Alkali- oder Erdalkalimetallcarbonate oder -hydrogencarbonate oder Stickstoffbasen. Genannt seien beispielsweise Natriumhydrid, Natriummethanolat, Natriumhydroxid, Calciumhydroxid, Kaliumcarbonat, Natriumhydrogencarbonat, Triethylamin, Dibenzylamin, Diisopropylamin, Pyridin, Chinolin, Diazabicyclooctan (DABCO), Diazabicyclononen (DBN) und Diazabicycloundecen (DBU).

Als Verdünnungsmittel können bei diesem Verfahren alle üblichen Lösungsmittel verwendet werden.

Vorzugsweise sind verwendbar aromatische Kohlenwasserstoffe wie Benzol oder Toluol, Alkohole wie Methanol, Ethanol, Isopropanol oder Ethylenglykol, Nitrile wie Acetonitril, Ether wie Tetrahydrofuran oder Dioxan, Amide wie Dimethylformamid oder andere polare Lösungsmittel wie Dimethylsulfoxid oder Sulfolan.

Die weitere Umsetzung mit dem Alkylhalogenid der Formel (XII) erfolgt vorzugsweise bei 0 bis 70°C und insbesondere bei 20 bis 50°C. Hierbei wird mindestens die äquimolare Menge Alkylhalogenid eingesetzt.

Man arbeitet bei Normaldruck oder unter erhöhtem Druck, vorzugsweise bei Normaldruck.

Die Aufarbeitung erfolgt wiederum nach üblichen Methoden.

Das erfindungsgemäße Verfahren (I) ist dadurch gekennzeichnet, daß man Verbindungen der Formeln (I-1-a) bis (1-5-a) jeweils mit Sulfonsäurechloriden der Formel (XIII) gegebenenfalls in Gegenwart eines Verdünnungsmittels und gegebenenfalls in Gegenwart eines Säurebindemittels umsetzt.

Beim Herstellungsverfahren (I) setzt man pro Mol Ausgangsverbindung der Formel (I-1-a) bis (I-5-a) ca. 1 Mol Sulfonsäurechlorid der Formel (XIII) bei -20 bis 150°C, vorzugsweise bei 0 bis 70°C um.

Das Verfahren (I) wird vorzugsweise in Gegenwart eines Verdünnungsmittels durchgeführt.

Als Verdünnungsmittel kommen alle inerten polaren organischen Lösungsmittel in Frage wie Ether, Amide, Ketone, Carbonsäureester, Nitrile, Sulfone, Sulfoxide oder halogenierte Kohlenwasserstoffe wie Methylenchlorid.

Vorzugsweise werden Dimethylsulfoxid, Tetrahydrofuran, Dimethylformamid, Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindungen (I-1-a) bis (I-5-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin und Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Das erfindungsgemäße Verfahren (J) ist dadurch gekennzeichnet, daß man Verbindungen der Formeln (I-1-a) bis (I-5-a) jeweils mit Phosphorverbindungen der Formel (XIV) gegebenenfalls in Gegenwart eines Verdünnungsmittels und gegebenenfalls in Gegenwart eines Säurebindemittels umsetzt.

Beim Herstellungsverfahren (J) setzt man zum Erhalt von Verbindungen der Formeln (I-1-e) bis (I-5-e) auf 1 Mol der Verbindungen (I-1-a) bis (I-5-a), 1 bis 2, vorzugsweise 1 bis 1,3 Mol der Phosphorverbindung der Formel (XIV) bei Temperaturen zwischen -40°C und 150°C, vorzugsweise zwischen -10 und 110°C um.

Das Verfahren (J) wird vorzugsweise in Gegenwart eines Verdünnungsmittels durchgeführt.

Als Verdünnungsmittel kommen alle inerten, polaren organischen Lösungsmittel in Frage wie Ether, Carbonsäureester, halogenierte Kohlenwasserstoffe, Ketone, Amide, Nitrile, Sulfone, Sulfoxide etc.

Vorzugsweise werden Acetonitril, Dimethylsulfoxid, Tetrahydrofuran, Dimethylformamid, Methylenchlorid eingesetzt.

Als gegebenenfalls zugesetzte Säurebindemittel kommen übliche anorganische oder organische Basen in Frage wie Hydroxide, Carbonate oder Amine. Beispielhaft seien Natriumhydroxid, Natriumcarbonat, Kaliumcarbonat, Pyridin und Triethylamin aufgeführt.

Die Umsetzung kann bei Normaldruck oder unter erhöhtem Druck durchgeführt werden, vorzugsweise wird bei Normaldruck gearbeitet. Die Aufarbeitung geschieht nach üblichen Methoden der Organischen Chemie. Die Endprodukte werden vorzugsweise durch Kristallisation, chrömatographische Reinigung oder durch sogenanntes "Andestillieren", d.h. Entfernung der flüchtigen Bestandteile im Vakuum gereinigt.

Das Verfahren (K) ist dadurch gekennzeichnet, daß man Verbindungen der Formeln (I-1-a) bis (I-5-a) jeweils mit Metallhydroxiden bzw. Metallalkoxiden der Formel (XV) oder Aminen der Formel (XVI), gegebenenfalls in Gegenwart eines Verdünnungsmittels, umsetzt.

Als Verdünnungsmittel können bei dem erfindungsgemäßen Verfahren (K) vorzugsweise Ether wie Tetrahydrofuran, Dioxan, Diethylether oder aber Alkohole wie Methanol, Ethanol, Isopropanol, aber auch Wasser eingesetzt werden. Das erfindungsgemäße Verfahren (K) wird im allgemeinen unter Normaldruck durchgeführt. Die Reaktionstemperatur liegt im allgemeinen zwischen -20°C und 100°C, vorzugsweise zwischen 0°C und 50°C.

Das erfindungsgemäße Verfahren (L) ist dadurch gekennzeichnet, daß man Verbindungen der Formeln (I-1-a) bis (I-5-a) jeweils mit (Lα) Verbindungen der Formel (XVII) gegebenenfalls in Gegenwart eines Verdünnungsmittels und gegebenenfalls in Gegenwart eines Katalysators oder (Lβ) mit Verbindungen der Formel (XVIII) gegebenenfalls in Gegenwart eines Verdünnungsmittels und gegebenenfalls in Gegenwart eines Säurebindemittels umsetzt.

Bei Herstellungsverfahren (Lα) setzt man pro Mol Ausgangsverbindung der Formeln (I-1-a) bis (I-5-a) ca. 1 Mol Isocyanat der Formel (XVII) bei 0 bis 100°C, vorzugsweise bei 20 bis 50°C um.

Das Verfahren (Lα) wird vorzugsweise in Gegenwart eines Verdünnungsmittels durchgeführt.

Als Verdünnungsmittel kommen alle inerten organischen Lösungsmittel in Frage, wie aromatische Kohlenwasserstoffe, halogenierte Kohlenwasserstoffe, Ether, Amide, Nitrile, Sulfone oder Sulfoxide.

Gegebenenfalls können Katalysatoren zur Beschleunigung der Reaktion zugesetzt werden. Als Katalysatoren können sehr vorteilhaft zinnorganische Verbindungen, wie z.B. Dibutylzinndilaurat eingesetzt werden.

Es wird vorzugsweise bei Normaldruck gearbeitet.

Beim Herstellungsverfahren (Lβ) setzt man pro Mol Ausgangsverbindung der Formeln (I-1-a) bis (I-5-a) ca. 1 Mol Carbamidsäurechlorid der Formel (XVIII) bei 0 bis 150°C, vorzugsweise bei 20 bis 70°C um.

Als gegebenenfalls zugesetzte Verdünnungsmittel kommen alle inerten polaren organischen Lösungsmittel in Frage wie Ether, Carbonsäureester, Nitrile, Ketone, Amide, Sulfone, Sulfoxide oder halogenierte Kohlenwasserstoffe.

Vorzugsweise werden Dimethylsulfoxid, Tetrahydrofuran, Dimethylformamid oder Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindung (I-1-a) bis (I-5-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Triethylamin oder Pyridin genannt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Die Wirkstoffe eignen sich zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.
Aus der Ordnung der Isoptera z.B. Reticulitermes spp..

Aus der Ordnung der Anoplura z.B. Phylloxera vastatrix, Pemphigus spp., Pediculus humanus corporis, Haematopinus spp., Linognathus spp..
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Hylotrupes bajulus, Agelastica alni, Leptinotarsa decemlineata, Phaedon cochleariae, Diabrotica spp., Psylliodes chrysocephala, Epilachna varive stis, Atomaria spp., Oryzaephilus surinamensis, Antho nomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Cono derus spp., Melolontha melolontha, Amphimallon solsti tialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp.. Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die erfindungsgemäßen Wirkstoffe zeichnen sich insbesondere durch eine hohe insektizide und akarizide Wirksamkeit aus.

Sie lassen sich mit besonders gutem Erfolg zur Bekämpfung von pflanzenschädigenden Insekten, wie beispielsweise gegen die Larven des Meerettichblattkäfers (Phaedon cochleariae) oder gegen die Larven der grünen Reiszikade (Nephotettix cincticeps) gegen die Raupen der Kohlschabe (Plutella maculipennis).

Die erfindungsgemäßen Wirkstoffe können weiterhin als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die zur Unkrautbekämpfung notwendigen Dosierungen der erfindungsgemäßen Wirkstoffe liegen zwischen 0,001 und 10 kg/ha, vorzugsweise zwischen 0,005 und 5 kg/ha.

Die erfindungsgemäßen Wirkstoffe können z.B. bei den folgenden Pflanzen verwendet werden:

Dikotyle Unkräter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotola, Lindemia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cycnodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Wirkstoffe eignen sich sehr gut zur selektiven Bekämpfung monokotyler Unkräuter in dikotylen Kulturen im Vor- und Nachlaufverfahren. Sie können beispielsweise in Baumwolle oder Zuckerrüben mit sehr gutem Erfolg zur Bekämpfung von Schadgräser eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Besonders günstige Mischpartner sind z.B. die folgenden:

### Fungizide:

2-Aminobutan; 2-Anilino-4-methyl-6-cyclopropyl-pyrimidin; 2',6'-Dibromo-2-methyl-4'-trifluoromethoxy-4'-trifluoro-methyl-1,3-thiazol-5-carboxanilid; 2,6-Dichloro-N-(4-trifluoromethylbenzyl)-benzamid; (E)-2-Methoxyimino-N-methyl-2-(2-phenoxyphenyl)-acetamid; 8-Hydroxyquinolinsulfat; Methyl-(E)-2-{2-[6-(2-cyanophenoxy)-pyrimidin-4-yloxy]-phenyl}-3-methoxyacrylat; Methyl-(E)-methoximino-[alpha-(o-tolyloxy)-o-tolyl]acetat; 2-Phenylphenol (OPP), Aldimorph, Ampropylfos, Anilazin, Azaconazol,
Benalaxyl, Benodanil, Benomyl, Binapacryl, Biphenyl, Bitertanol, Blasticidin-S, Bromuconazole, Bupirimate, Buthiobate,
Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
Guazatine,
Hexachlorobenzol, Hexaconazol, Hymexazol,
Imazalil, Imibenconazol, Iminoctadin, Iprobenfos (IBP), Iprodion, Isoprothiolan, Kasugamycin, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Mepanipyrim, Mepronil, Metalaxyl, Metconazol, Methasulfocarb, Methfuroxam, Metiram, Metsulfovax, Myclobutanil,
Nickel-dimethyldithiocarbamat, Nitrothal-isopropyl, Nuarimol,
Ofurace, Oxadixyl, Oxamocarb, Oxycarboxin,
Pefurazoat, Penconazol, Pencycuron, Phosdiphen, Phthalid, Pimaricin, Piperalin, Polycarbamate, Polyoxin, Probenazol, Prochloraz, Procymidon, Propamocarb, Propiconazole, Propineb, Pyrazophos, Pyrifenox, Pyrimethanil, Pyroquilon, Quintozen (PCNB),
Schwefel und Schwefel-Zubereitungen,
Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
Validamycin A, Vinclozolin,
Zineb, Ziram.

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, Bendiocarb, Benfuracarb, Bensultap, Betacyfluthrin, Bifenthrin, BPMC, Brofenprox, Bromophos A, Bufencarb, Buprofezin, Butocarboxim, Butylpyridaben,
Cadusafos, Carbaryl, Carbofuran, Carbophenothion, Carbosulfan, Cartap, CGA 157 419, CGA 184699, Chloethocarb, Chlorethoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat, Dimethylvinphos, Dioxathion, Disulfoton,
Edifenphos, Emamectin, Esfenvalerat, Ethiofencarb, Ethion, Ethofenprox, Ethoprophos, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivermectin, Lambda-cyhalothrin, Lufenuron,
Malathion, Mecarbam, Mevinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, NI 25, Nitenpyram,
Omethoat, Oxamyl, Oxydemethon M, Oxydeprofos,
Parathion A, Parathion M, Permethrin, Phenthoat, Phorat, Phosalon, Phosmet, Phosphamidon, Phoxim, Pirimicarb, Pirimiphos M, Pirimiphos A, Profenofos, Promecarb, Propaphos, Propoxur, Prothiofos, Prothoat, Pymetrozin, Pyrachlophos, Pyridaphenthion, Pyresmethrin, Pyrethrum, Pyridaben, Pyrimidifen, Pyriproxifen, Quinalphos,
RH 5992,
Salithion, Sebufos, Silafluofen, Sulfotep, Sulprofos,
Tebufenozid, Tebufenpyrad, Tebupirimiphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, YI 5301 / 5302, Zetamethrin.

### Herbizide:

beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB, 2,4 DP, Fluroxypyr, MCPA, MCPP und Triclopyr; Aryloxy-phenoxyalkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuronethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Der erfindungsgemäße Wirkstoff kann ferner in seinen handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:

Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp., Xenopsylla spp., Ceratophyllus spp..

Aus der Ordnung der Heteropterida z.B. Cimex spp., Triatoma spp., Rhodnius spp., Panstrongylus spp..

Aus der Ordnung der Blattarida z.B. Blatta orientalis, Periplaneta americana, Blattela germanica, Supella spp..

Aus der Unterklasse der Acaria (Acarida) und den Ordnungen der Meta- sowie Mesostigmata z.B. Argas spp., Ornithodorus spp., Otabius spp., Ixodes spp., Amblyomma spp., Boophilus spp., Dermacentor spp., Haemaphysalis spp., Hyalomma spp., Rhipicephalus spp., Dermanyssus spp., Raillietia spp., Pneumonyssus spp., Sternostoma spp., Varroa spp..

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Beispielsweise zeigen sie eine hervorragende Wirksamkeit gegen Boophilus microplus und Lucilia cuprina.

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen der Formel (I) eine hohe insektizide Wirkung gegen Insekten zeigen, die technische Materialien zerstören.

Beispielhaft und vorzugsweise - ohne jedoch zu limitieren - seien die folgenden Insekten genannt:

### Käfer wie

Hylotrupes bajulus, Chlorophorus pilosis, Anobium punctatum, Xestobium rufovillosum, Ptilinus pecticomis, Dendrobium pertinex, Emobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus.

### Hautflügler wie

Sirex juvencus, Urocerus gigas, Urocerus gigas taignus, Urocerus augur.

### Termiten wie

Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus.

### Borstenschwänze wie

Lepisma saccharina.

Unter technischen Materialien sind im vorliegenden Zusainmenhang nicht-lebende Materialien zu verstehen, wie vorzugsweise Kunststoffe, Klebstoffe, Leime, Papiere und Kartone, Leder, Holz und Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen: Bauholz, Holzbalken, Eisenbahnschwellen, Brückenteile, Bootsstege, Holzfahrzeuge, Kisten, Paletten, Container, Telefonmasten, Holzverkleidungen, Holzfenster und -türen, Sperrholz, Spanplatten, Tischlerarbeiten oder Holzprodukte, die ganz allgemein beim Hausbau oder in der Bautischlerei Verwendung finden.

Die Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindeöl und/oder Monochlornaphthalin, vorzugsweise α-Monochlornaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, daß das Lösungsmittelgemisch ebenfalls eine Verdunstungszahl über 35 und einen Flammpunkt oberhalb 30°C, vorzugsweise oberhalb 45°C, aufweist und daß das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches durch ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder ein Weichmacher(gemisch) ersetzt werden. Diese Zusätze sollen einer Verflüchtigung der Wirkstoffe sowie einer Kristallisation bzw. Ausfällem vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der WO 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner seien Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron und Triflumuron,
sowie Fungizide wie Epoxyconazole, Hexaconazole, Azaconazole, Propiconazole, Tebuconazole, Cyproconazole, Metconazole, Imazalil, Dichlofluanid, Tolylfluanid, 3-Iod-2-propinyl-butylcarbamat, N-Octyl-isothiazolin-3-on und 4,5-Dichlor-N-octylisothiazolin-3-on genannt.

Die Herstellung und die Verwendung der erfindungsgemäßen Wirkstoffe geht aus den nachfolgenden Beispielen hervor.

### Herstellungsbeispiele

### Beispiel (I-1-a-1)

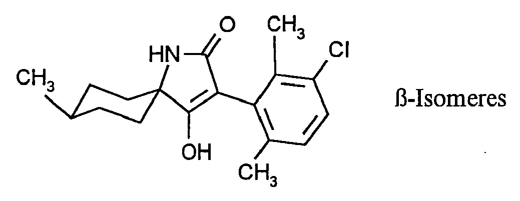

Zu 22,7 g (0,2 Mol) Kalium-tert.-butylat in 60 ml wasserfreiem Tetrahydrofuran (THF) tropft man bei Rückflußtemperatur 29,6 g (0,0764 Mol) der Verbindung gemäß Beispiel (II-1) in 160 ml wasserfreiem Toluol und rührt 1,5 Stunden unter Rückfluß. Zur Aufarbeitung gibt man 230 ml Wasser zu, trennt die wäßrige Phase ab, extrahiert die Toluolphase mit 110 ml Wasser, vereinigt die wäßrigen Phasen, wäscht sie mit Toluol und säuert bei 10 bis 20°C mit konz. HCl auf pH 1 an. Das Produkt wird abgesaugt, gewaschen, getrocknet und durch Verrühren in Methyltert.-butyl(MTB)-ether/n-Hexan gewaschen.

Ausbeute: 13,7 g (56 % der Theorie), Fp.: >220°C.

Analog bzw. gemäß den allgemeinen Angaben zur Herstellung erhält man die folgenden Verbindungen der Formel (I-1-a):

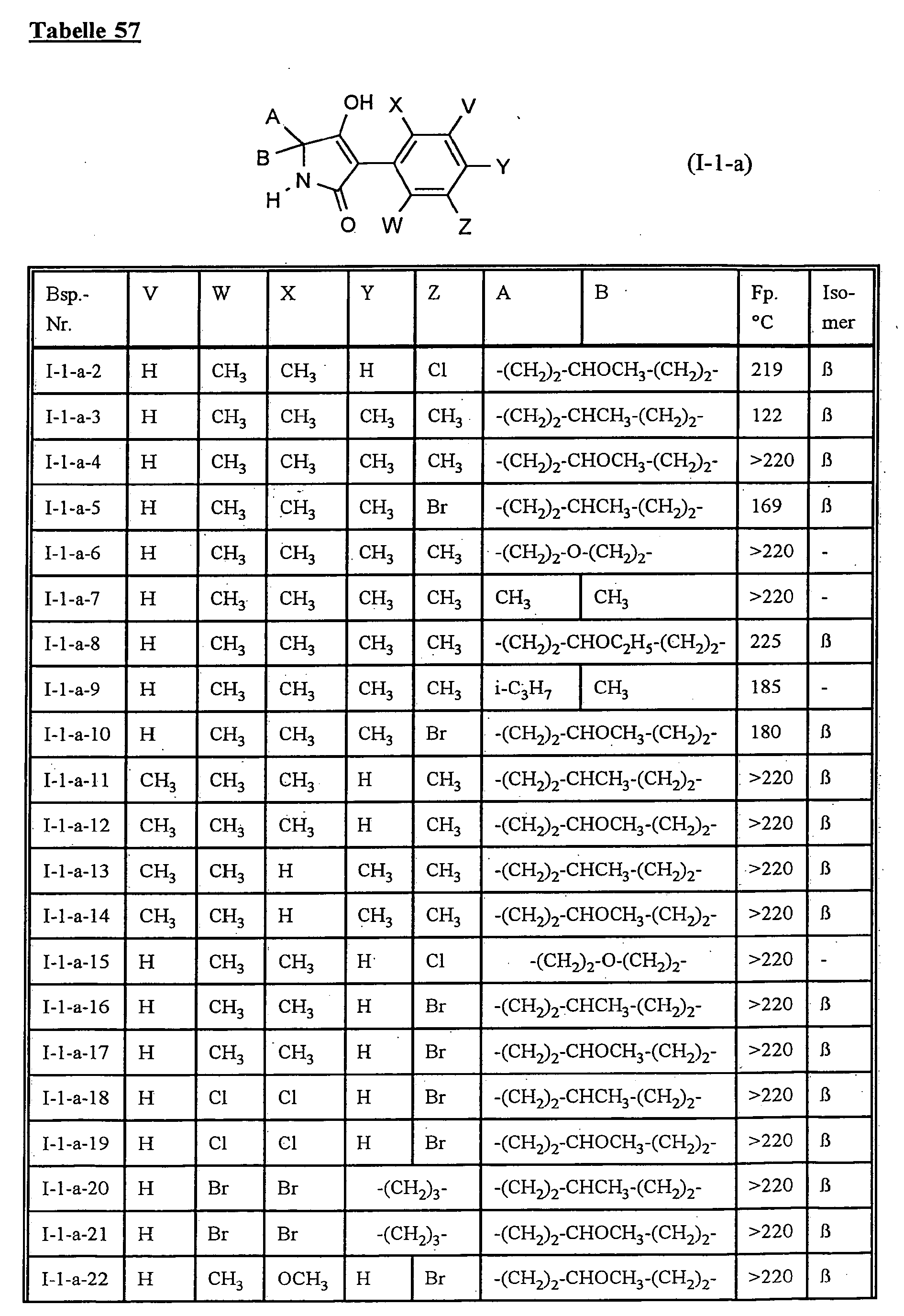

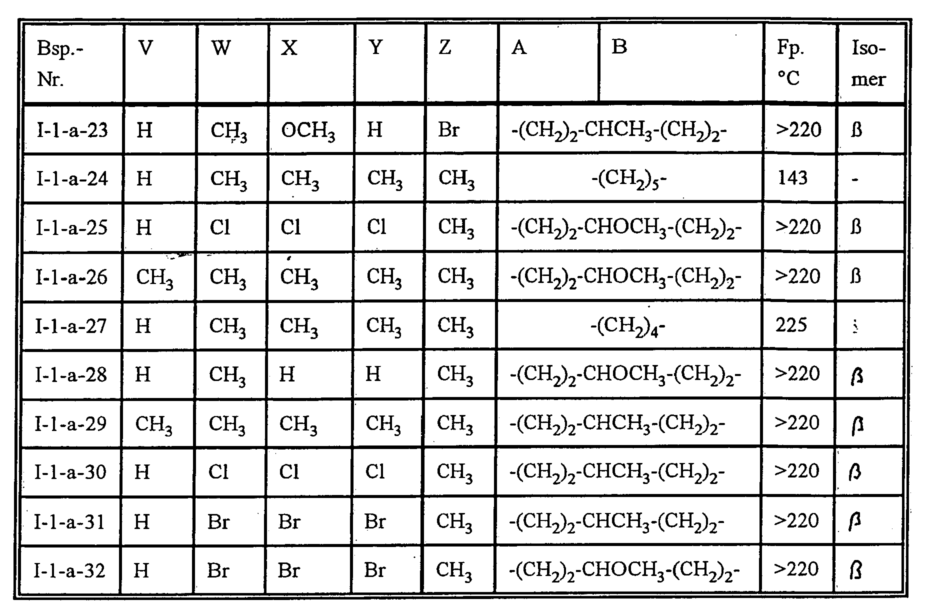

### Beispiel (I-1-b-1)

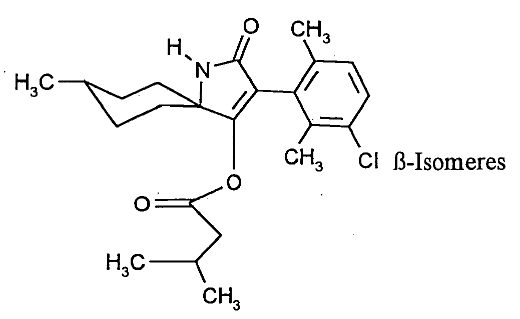

3,84 g (0,012 Mol) der Verbindung gemäß Beispiel (I-1-a-1) und 2,5 ml (18 mMol) Triethylamin in 70 ml wasserfreiem Methylenchlorid werden bei 0 bis 10°C mit 2,2 ml (0,18 Mol) Isovaleriansäurechlorid in 5 ml wasserfreiem Methylenchlorid versetzt. Bei Raumtemperatur wird gerührt, bis die Reaktion nach dünnschichtchromatographischer Kontrolle beendet ist. Zur Aufarbeitung wird 2 mal mit 50 ml 0,5 N Natronlauge gewaschen, über Magnesiumsulfat getrocknet und eingedampft. Das Rohprodukt wird aus MTB-Ether/n-Hexan umkristallisiert.

Ausbeute: 1,6 g (33 % der Theorie), Fp.: 218°C.

Analog bzw. gemäß den allgemeinen Angaben zur Herstellung erhält man die folgenden Verbindungen der Formel (I-b-1):

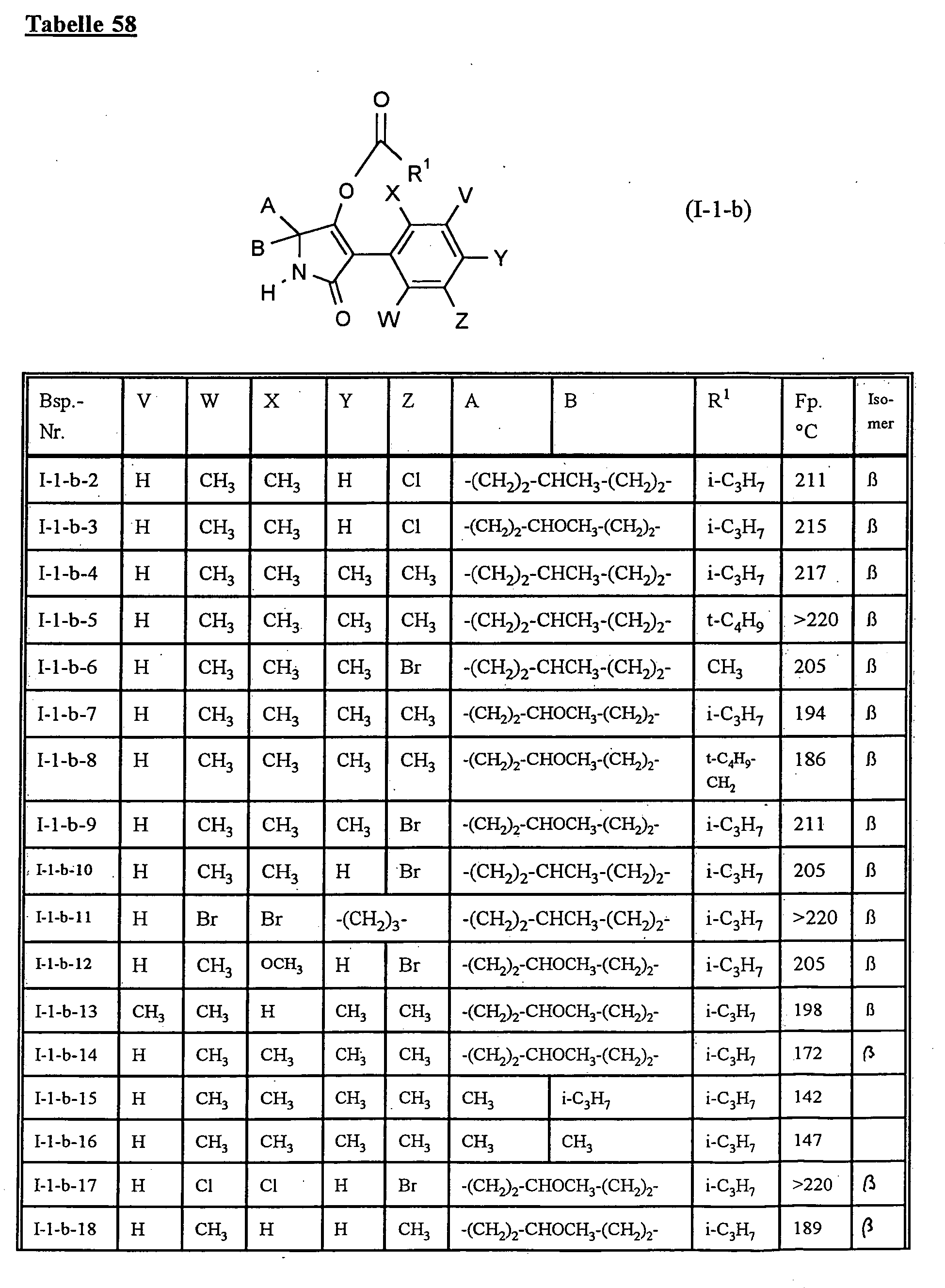

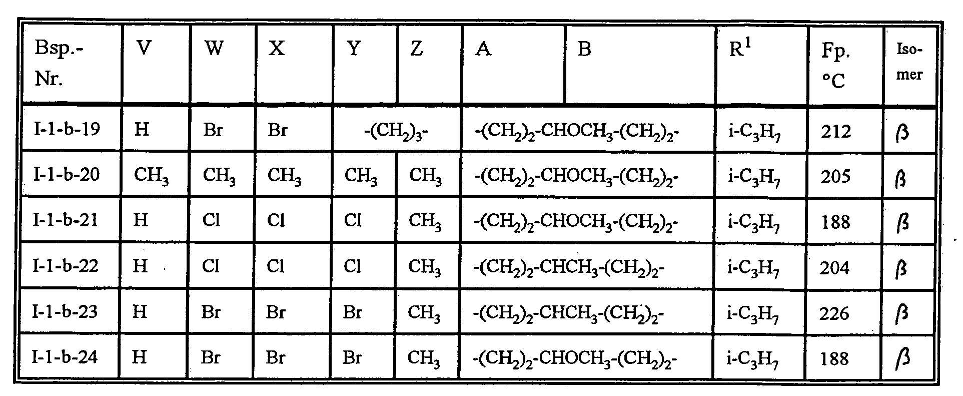

### Beispiel (I-1-c-1)

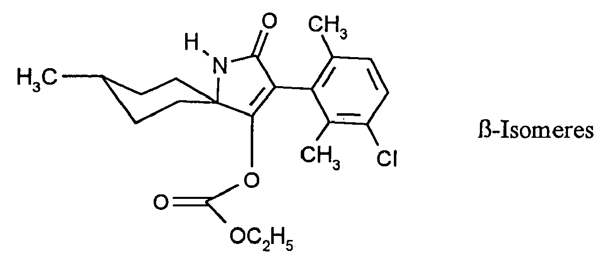

Zu 3,84 g (0,012 Mol) der Verbindung gemäß Beispiel (I-1-a-1) und 1,7 ml (0,012 Mol) Triethylamin in 70 ml wasserfreiem CH₂Cl₂ tropft man bei 0 bis 10°C 1,2 ml (0,012 Mol) Chlorameisensäureethylester in 5 ml wasserfreiem Methylenchlorid und rührt bei Raumtemperatur, bis die Reaktion nach dünnschichtchromatographischer Kontrolle beendet ist. Zur Aufarbeitung wird 2 mal mit 50 ml 0,5 N Natronlauge gewaschen, über Magnesiumsulfat getrocknet und eingedampft.

Ausbeute: 3,6 g (76 % der Theorie), Fp.: >220°C.

Analog bzw. gemäß den allgemeinen Angaben zur Herstellung erhält man die folgenden Verbindungen der Formel (I-1-c):

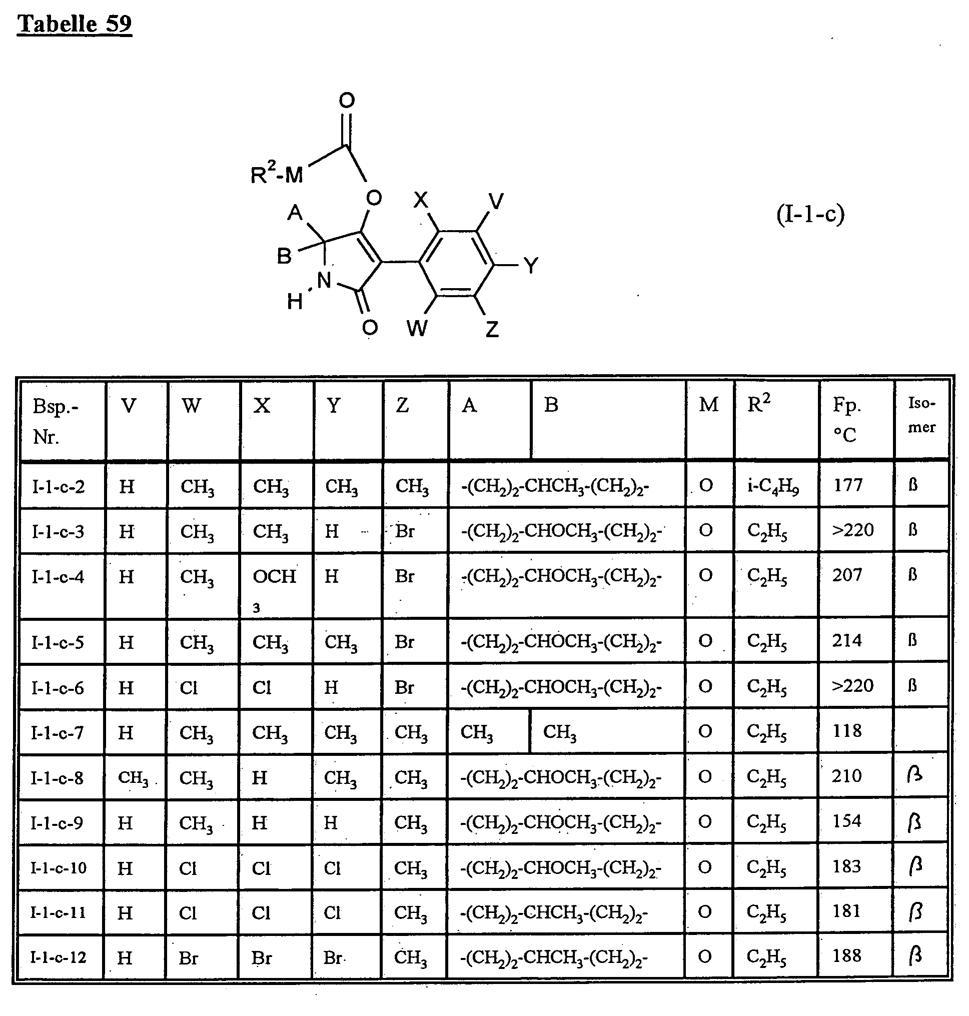

### Beispiel II-1

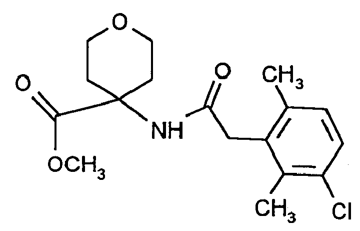

Zu 26,6 g (0,257 Mol) konz. Schwefelsäure tropft man bei 30 bis 40°C vorsichtig 16,7 g (0,0544 Mol) der Verbindung gemäß Beispiel (XXIX-1) in 160 ml wasserfreiem Methylenchlorid und rührt 2 Stunden bei dieser Temperatur. Dann tropft man 37 ml absolutes Methanol so zu, daß sich eine Innentemperatur von etwa 40°C einstellt und rührt noch 6 Stunden bei 40 bis 70°C.

Zur Aufarbeitung gießt man auf 0,28 kg Eis, extrahiert mit Methylenchlorid, wäscht mit wäßriger Natriumhydrogencarbonatlösung, trocknet und dampft ein. Der Rückstand wird aus Methyl-tert.-butylether/n-Hexan umkristallisiert.

Ausbeute: 16,5 g (89 % der Theorie), Fp.: 168°C.

### Beispiel (II-2)

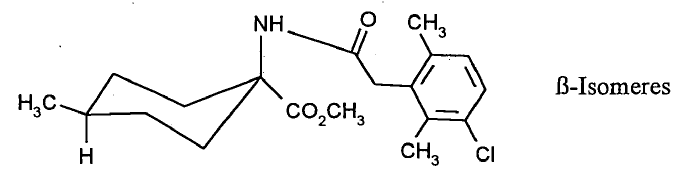

15,9 g (0,08 Mol) 3-Chlor-2,6-dimethylphenylessigsäure und 17,7 ml (0,24 Mol) Thionylchlorid werden 30 Min. bei Raumtemperatur und anschließend bei 80°C gerührt, bis die Gasentwicklung beendet ist. Überschüssiges Thionylchlorid wird bei 50°C im Vakuum entfernt. Dann gibt man 50 ml wasserfreies Toluol zu und dampft erneut ein. Der Rückstand wird in 100 ml wasserfreiem THF aufgenommen (Lösung 1).

Zu 16,8 g cis-4-Methylcyclohexylamin-1-carbonsäuremethylester und 24,6 ml (0,176 Mol) Triethylamin in 160 ml wasserfreiem THF tropft man bei 0 bis 10°C Lösung 1 und rührt anschließend 1 Stunde bei Raumtemperatur. Dann wird abgesaugt, mit wasserfreiem THF gewaschen und eingedampft. Der Rückstand wird in Methylenchlorid aufgenommen, mit 0,5 N HCl gewaschen, getrocknet und eingedampft. Das Rohprodukt wird aus Methyl-tert.-butylether/n-Hexan umkristallisiert.

Ausbeute: 26,9 g (74 % der Theorie), Fp.: 163°C.

Analog zu den Beispielen (II-1) und (II-2) und gemäß den allgemeinen Angaben zur Herstellung werden die folgenden Verbindungen der Formel (II) hergestellt:

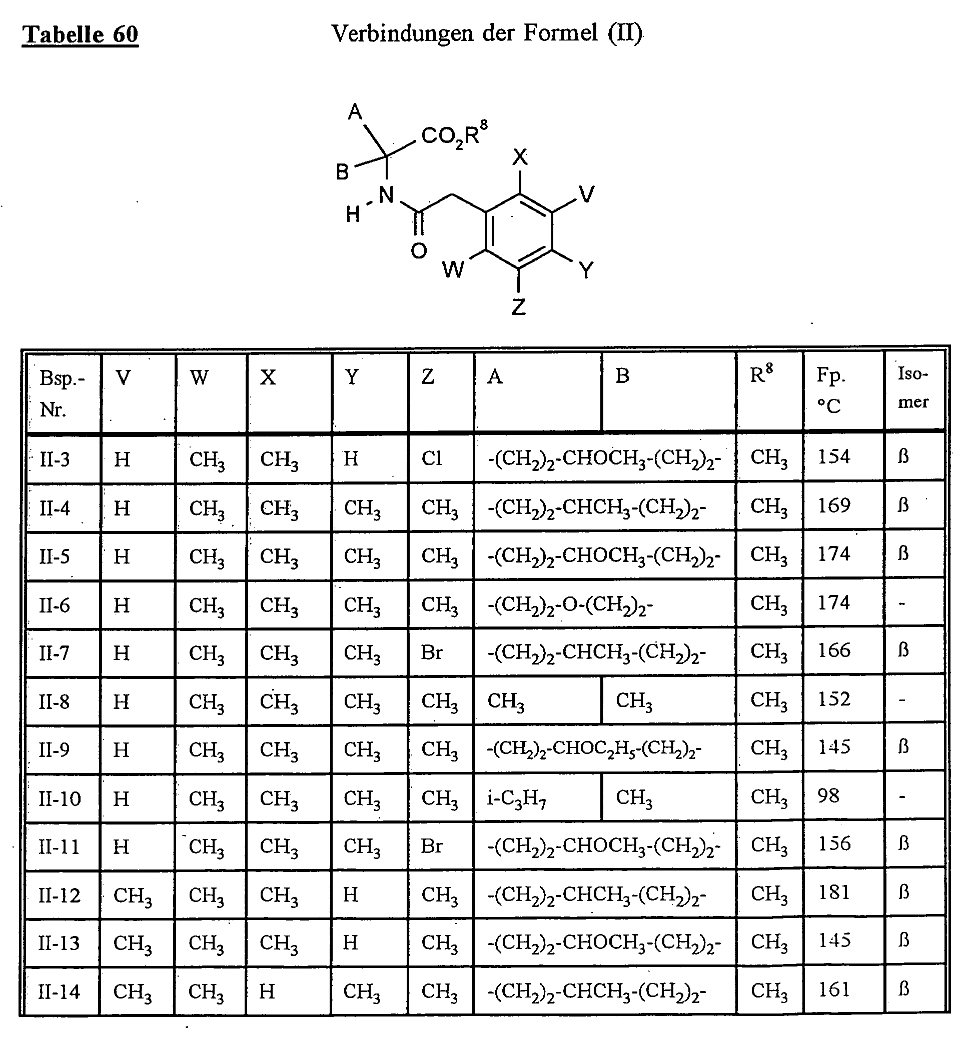

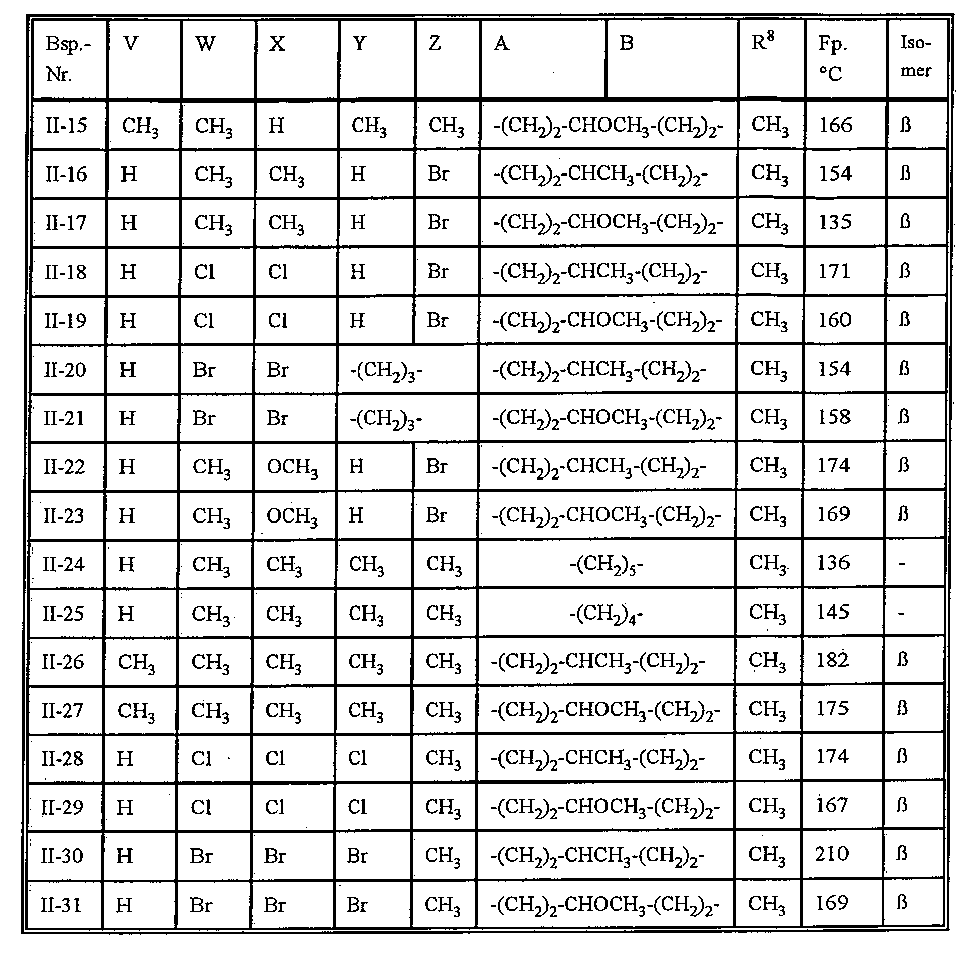

### Beispiel (XXIX-1)

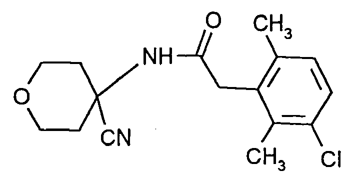

Ausgehend von 12,6 g 3-Chlor-2,6-dimethylphenylessigsäure wird wie in Beispiel (II-2) Lösung 1 hergestellt.

Zu 15,1 g 4-Amino-tetrahydropyran-4-carbonsäurenitril (70 %ig) und 9,2 ml (0,066 Mol) Triethylamin in 120 ml wasserfreiem THF tropft man bei 0 bis 10°C Lösung 1 und rührt noch 1 Stunde bei Raumtemperatur. Dann wird eingedampft und man nimmt den Rückstand in Methylenchlorid auf, wäscht mit 0,5 N HCl, trocknet und dampft ein. Das Rohprodukt wird aus MTB-Ether/n-Hexan umkristallisiert.

Ausbeute: 16,7 g (90 % der Theorie), Fp.: 176°C.

Analog zu Beispiel (XXIX-1) und gemäß den allgemeinen Angaben zur Herstellung werden folgende Verbindungen der Formel (XXIX) hergestellt:

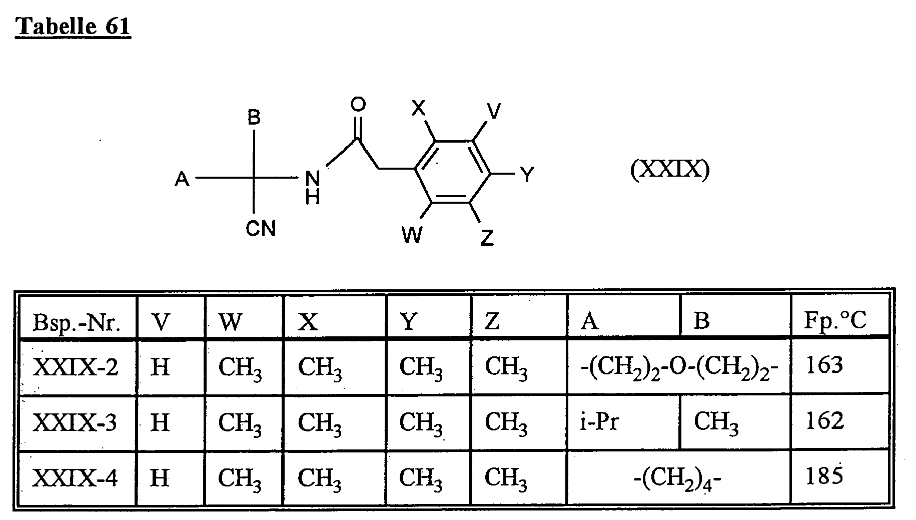

### Beispiel (I-2-a-1)

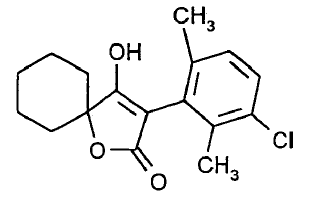

8,42 g (75 mMol) Kalium-tert.-butylat werden in 50 ml wasserfreiem Tetrahydrofuran (THF) vorgelegt, bei 0 bis 10°C eine Lösung von 17,62 g (50 mMol) 3-Chlor-2,6-dimethylphenylessigsäure-(1-ethyloxycarbonyl-cyclohexyl)-ester gemäß Beispiel (III-1) in 50 ml wasserfreiem THF zugetropft und über Nacht bei Raumtemperatur gerührt.

Zur Aufarbeitung gießt man das Reaktionsgemisch in 600 ml Wasser, säuert mit 10 %iger Salzsäure an, saugt das Produkt ab, nimmt es in Methylenchlorid auf, engt ein und verreibt die Kristalle mit Petrolether.

Ausbeute: 9,7 g (63 % der Theorie) vom Fp.: 193°C.

Analog zu Beispiel (I-2-a-1) und gemäß den allgemeinen Angaben zur Herstellung erhält man die folgenden Verbindungen (I-2-a):

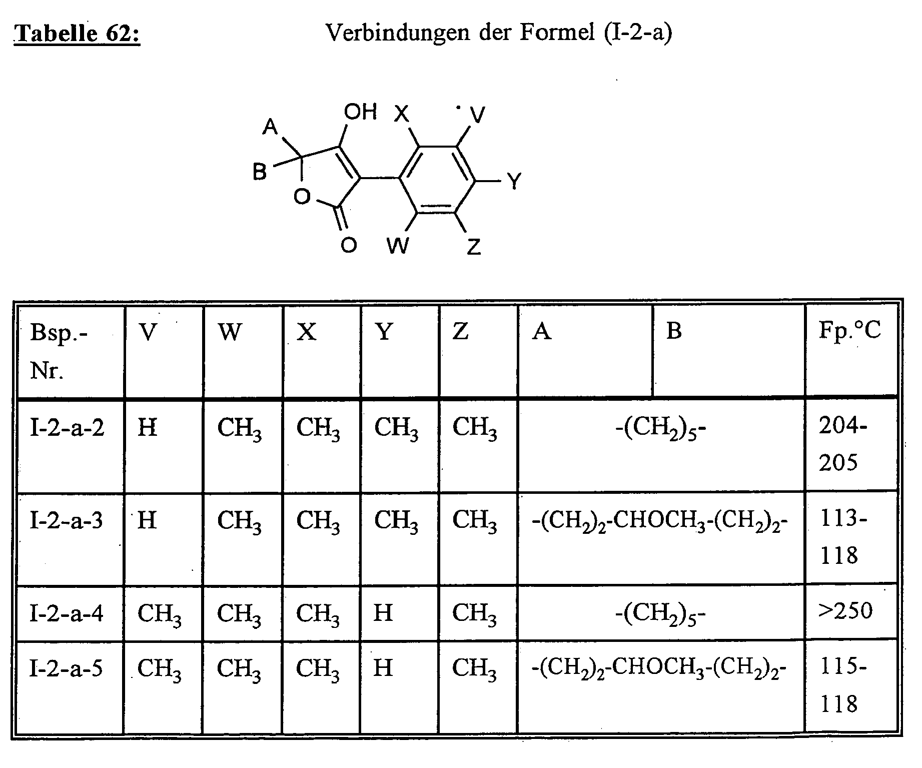

### Beispiel (I-2-b-1)

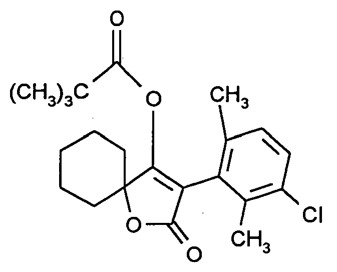

3,06 g (10 mmol) der Verbindung gemäß Beispiel I-2-a-1 werden in 40 ml wasserfreiem THF vorgelegt, 1,1g (11 mMol) Triethylamin zugesetzt, unter Eiskühlung eine Lösung von 1,2 g (10 mMol) Pivaloylchlorid zugetropft und über Nacht bei Raumtemperatur nachgerührt. Zur Aufarbeitung engt man ein, verrührt mit Wasser, saugt ab, trocknet, nimmt den Rückstand in Ether auf, wäscht mit 1N NaOH, trocknet die organische Phase über MgSO₄ und dampft ein. Das Rohprodukt wird zur weiteren Aufreinigung noch mit wenig Petrolether verrührt.

Ausbeute: 3,1 g (79 % der Theorie) vom Fp.: 126°C.

Analog bzw. gemäß den allgemeinen Angaben zur Herstellung erhält man die folgenden Verbindungen der Formel (I-2-b):

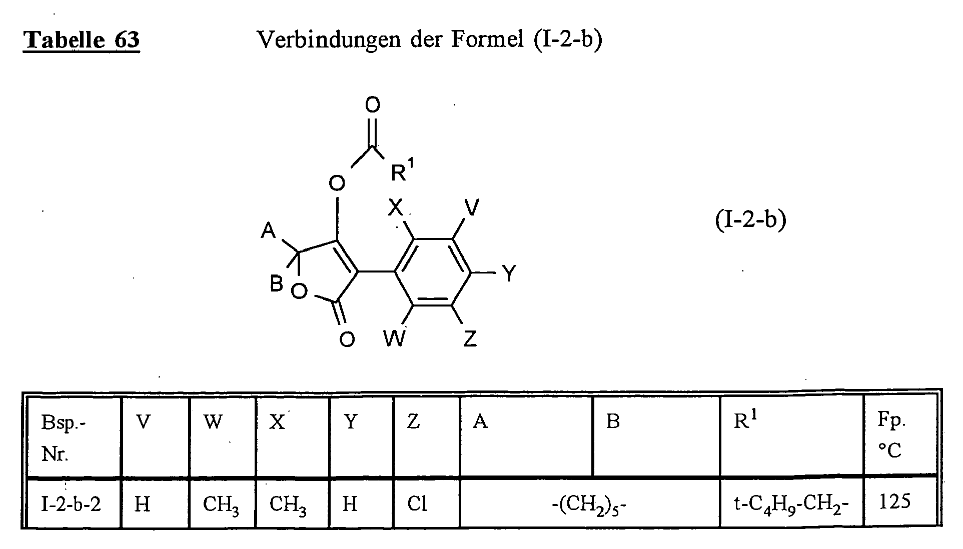

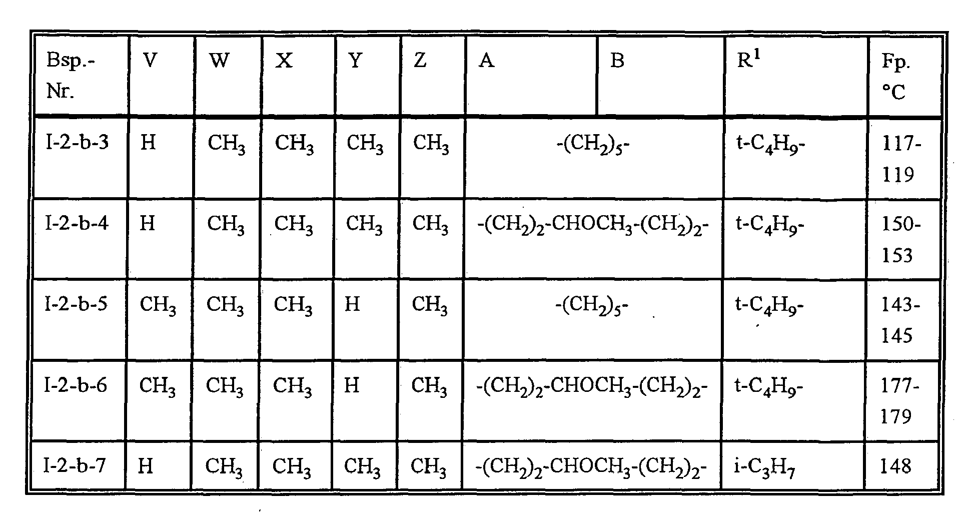

### Beispiel I-2-c-1

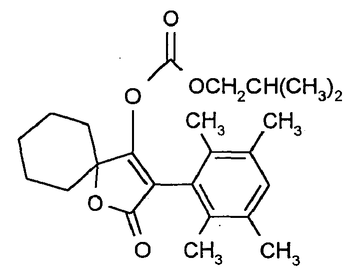

Zu 1.50 g (5 mmol) der Verbindung gemäß Beispiel (I-2-a-4) und 0,55 g (5,5 mmol) Triethylamin in 20 ml Dichlormethan tropft man bei Raumtemperatur 0,68 g (5 mmol) Chlorameisensäureisobutylester und rührt noch 2 Stunden. Dann wäscht man mit 10 %iger wäßriger Citronensäure und 1N Natronlauge, trocknet die Dichlormethanphase und engt ein. Der als Öl erhaltene Rückstand wird mit Petrolether verrührt und der sich bildende Feststoff abgesaugt.

Ausbeute 0,76 g, Fp.: 118 bis 119°C.

Analog bzw. gemäß den allgemeinen Angaben zur Herstellung erhält man die folgenden Verbindungen der Formel (I-2-c):

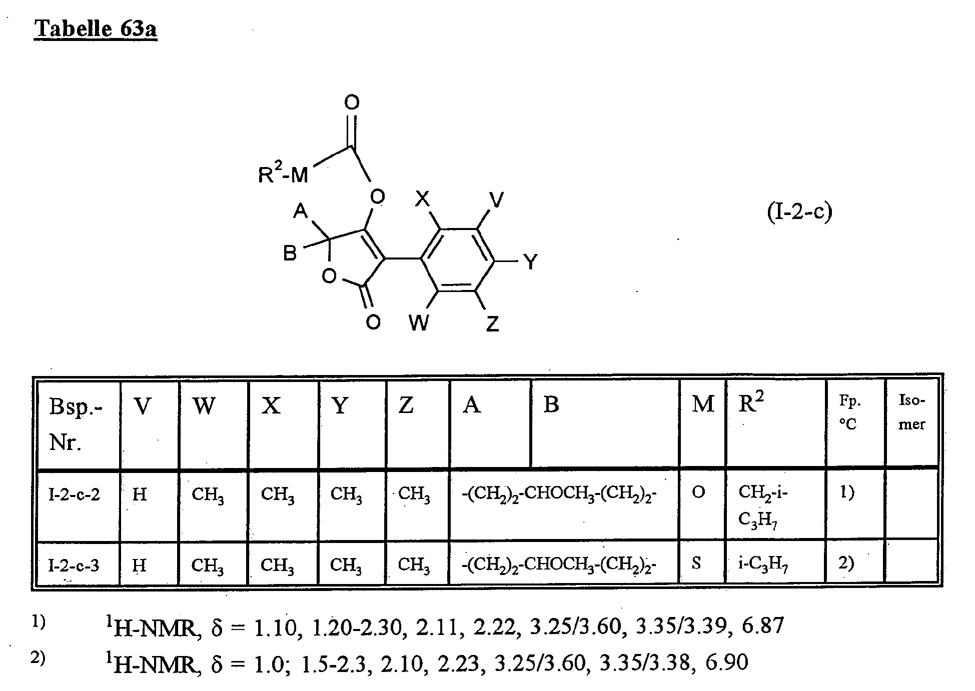

### Beispiel (III-1)

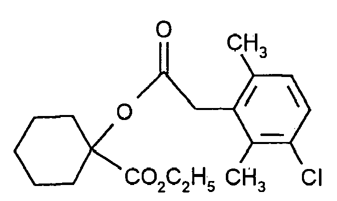

10,85 g (50 mMol) 3-Chlor-2,6-dimethyl-phenylessigsäurechlorid werden zusammen mit 8,6 g (50 mMol) 1-Hydroxy-cyclohexancarbonylsäureethylester 1 h in 100 ml Toluol gekocht, anschließend wird eingedampft.

Ausbeute: 17,5 g (quant.) 3-Chlor-2,6-dimethyl-phenylessigsäure(1-ethoxycarbonylcyclohexyl)-ester als farbloses Öl.

GC/MS m/e⁻ = M⁺ 352 (6 %), 155 (100 %), 109 (48 %), 81 (76 %), 29 (76 %).

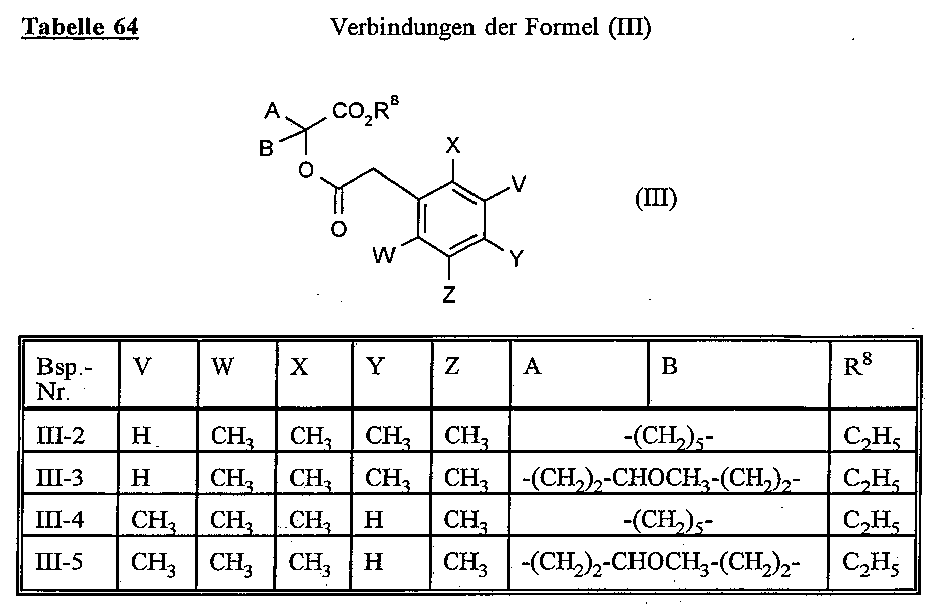

Die Verbindungen der Formel III fallen als zähflüssige Öle an und werden direkt meist ohne weitere Reinigung und Charakterisierung in die Verbindungen der Formel I-2-a überführt.

### Beispiel (I-3-a-1)

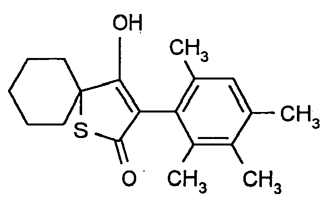

16,2 g (35 mmol) der Verbindungen gemäß Beispiel (IV-1) werden in 70 ml Toluol und 35 ml Trifluoressigsäure vorgelegt und 5 Stunden unter Rückfluß erhitzt. Die Trifluoressigsäure wird im Vakuum abgedampft und der Rückstand 2 x mit Toluol versetzt, das ebenfalls im Vakuum abgedampft wird. Der Rückstand wird mit 400 ml Wasser und 120 ml Methyl-tert.-butylether (MTB-Ether) aufgenommen, mit NaOH alkalisch gestellt, zweimal mit MTB-Ether extrahiert und die wässrige Phase mit Salzsäure angesäuert. Man extrahiert dreimal mit MTB-Ether, trocknet die organischen Phasen und engt ein.
Ausbeute: 8,5 g (78 % der Theorie); Fp.: 226-231°C.

### Beispiel (I-3-b-1)

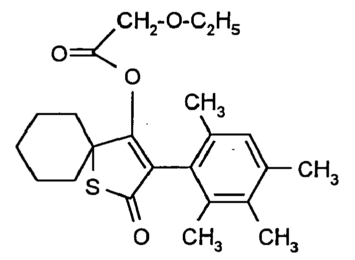

1,5 g (4,7 mMol) der Verbindung gemäß Beispiel (I-3-a-1) in 20 ml absolutem Methylenchlorid werden mit 0,98 ml (1,5 eq) Triethylamin versetzt. Unter Eiskühlung werden 0,86 g (1,5 eq) Ethoxyessigsäurechlorid, gelöst in 3 ml absolutem Methylenchlorid zugetropft. Man rührt ein bis zwei Stunden bei Raumtemperatur, wäscht zweimal mit 10 %iger Citronensäure und extrahiert die vereinigten wässrigen sauren Phasen mit Methylenchlorid. Die vereinigten organischen Phasen werden zweimal mit 1 N NaOH gewaschen und anschließend die wässrigen alkalischen Phasen mit Methylenchlorid extrahiert. Die vereinigten organischen Phasen werden getrocknet und eingeengt.

Ausbeute: 1,70 g (86 % der Theorie), Öl.

¹H-NMR (400 MHz, CDCl₃): 1.08 (t, 3H; 1.1-2.1 (m, 10H); 2.1-2.2 (4s, 12H); 3.1 (q, 2H); 3.9 (m, 2H); 6.83 (s, 1H).

### Beispiel (I-3-b-2)

Analog zu Beispiel (I-3-b-1) wurde die folgende Verbindung unter Verwendung von Pivaloylchlorid hergestellt, die als Öl anfällt.

Ausbeute: 2,00 g (100 % der Theorie).

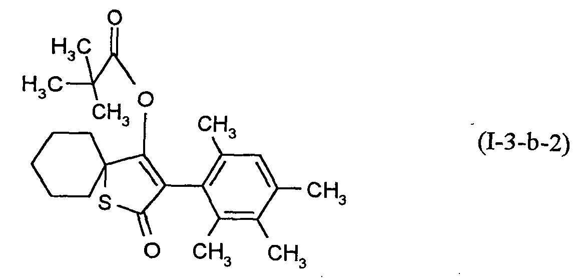

### Beispiel (I-3-c-1)

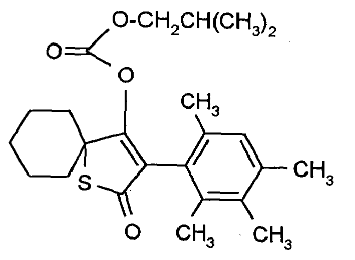

Die Umsetzung von 1,5 g (4,7 mMol) der Verbindung gemäß Beispiel (I-3-a-1) mit 0,91 ml (1,5 eq) Chlorameisensäureisobutylester unter Reaktionsbedingungen wie in Beispiel (I-3-b-1) beschrieben, liefert 1,85 g (94 % der Theorie) der oben gezeigten Verbindung.

Fp.: 83-89°C.

### Beispiel (IV-1)

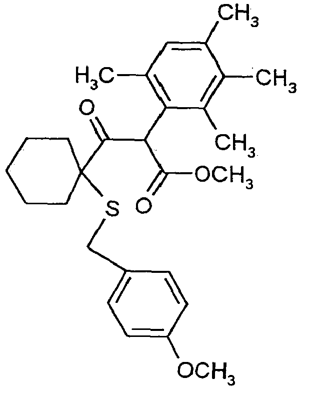

Zu 18 g (64 mMol) der Verbindung 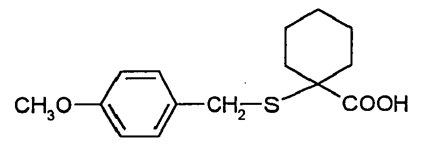 in 70 ml absolutem Toluol gibt man 1 Tropfen Dimethylformamid (DMF) und anschließend 11,4 g (96 mmol) Thionylchlorid. Man rührt 5 Minuten bei Raumtemperatur und anschließend bei 100°C, bis die Gasentwicklung beendet ist. Man engt im Vakuum ein, versetzt 2 mal mit Toluol, welches jeweils wieder abdestilliert wird und rührt anschließend noch 1 Stunde im Hochvakuum. Das erhaltene Säurechlorid wird in 40 ml absolutem Tetrahydrofuran (THF) gelöst (Lösung A).

In 100 ml absolutem THF legt man bei ca. 0°C 57 ml (177 mmol) einer Lösung von Lithiumdiisopropylamid (LDA) in
vor, tropft bei dieser Temperatur 22,1 g (107 mmol) 2,3,4,6-Tetramethylphenylessigsäuremethylester, gelöst in 40 ml absolutem THF, zu und rührt weitere 30 Minuten.

Anschließend wird wiederum bei ca. 0°C die Lösung A zugetropft und eine Stunde ohne Kühlung gerührt.

Man versetzt mit 350 ml MTB-Ether und einigen Tropfen Wasser, wäscht 2 mal mit 10 %iger wässriger Ammoniumchloridlösung, trocknent die organische Phase und engt ein. Das Rohprodukt wird mit Petrolether verrührt. Man saugt ab und trocknet.

Ausbeute: 16,20 g (54 % der Theorie). Fp.: 114-116°C.

### Beispiel (I-4-a-1)

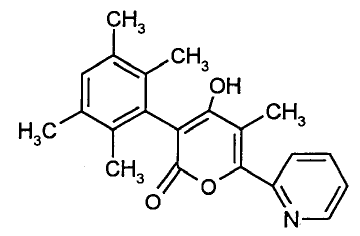

2,4 g (10 mMol) 2,3,5,6-Tetramethyl-phenyl-chlorcarbonylketen werden in 20 ml Toluol (p.a.) vorgelegt. Man gibt bei Raumtemperatur 1,35 g (10 mMol) 2-Pyridyl-ethylketon zu und rührt 8 Stunden unter Rückfluß. Bei der Abkühlung kristallisiert das Produkt aus. Der Niederschlag wird abgesaugt und zweimal mit Cyclohexan gewaschen.

Ausbeute: 1,4 g (41 % der Theorie). Fp.: 202-205°C.

Analog zu Beispiel (I-4-a-1) bzw. gemäß den allgemeinen Angaben zur Herstellung wurden die in der folgenden Tabelle aufgeführten Verbindungen der Formel (I-4-a) hergestellt:

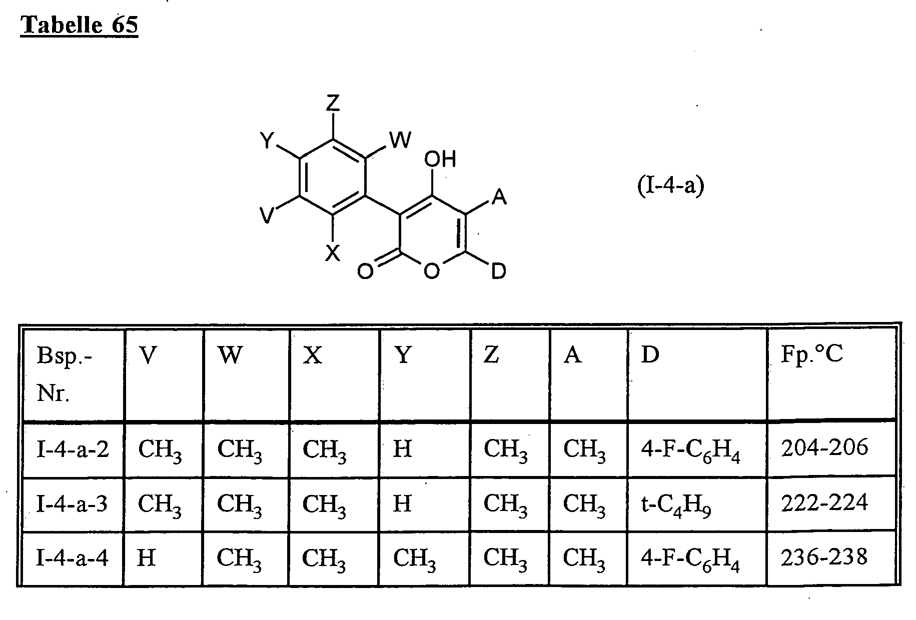

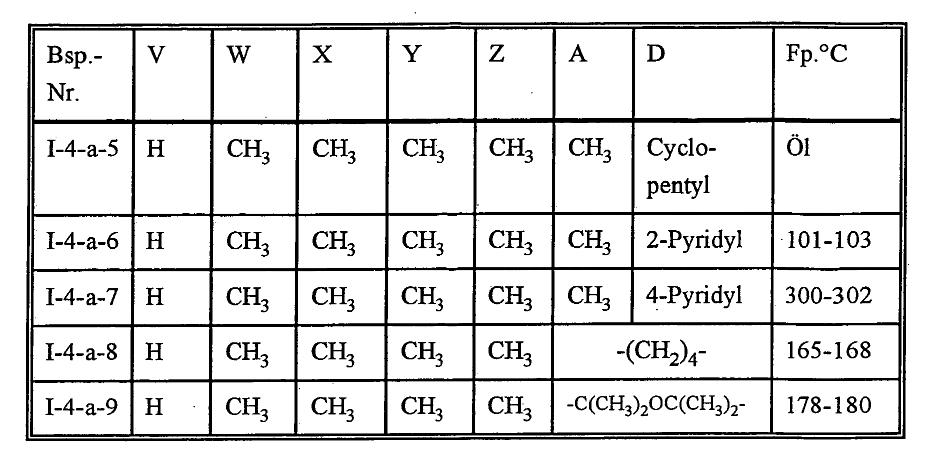

### Beispiel I-4-b-1

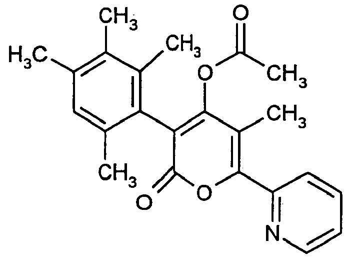

1,7 g (5 mmol) der Verbindung gemäß Beispiel I-4-a-6 werden in 20 ml Ethylacetat p.A. vorgelegt. Dazu gibt man bei 20°C 0,5 g (5 mmol) Triethylamin und tropft bei 0°C 0,39 g (5 mmol) ClCOCH₃ in 5 ml Ethylacetat zu. Die Reaktion wird unter Feuchtigkeitsausschluß gefahren. Man rührt 20 Stunden bei 20°C. Abtrennung des Niederschlags, Waschen mit Ethylacetat. Die organische Phase wird mit 2 x 25 ml halbkonzentrierter NaCl-Lösung gewaschen, über Natriumsulfat getrocknet und eingedampft.

Ausbeute 1 g (53 % der Theorie), Fp.: 170 bis 172°C.

Analog bzw. gemäß den allgemeinen Angaben zur Herstellung erhält man die folgenden Verbindungen der Formel (I-4-b):

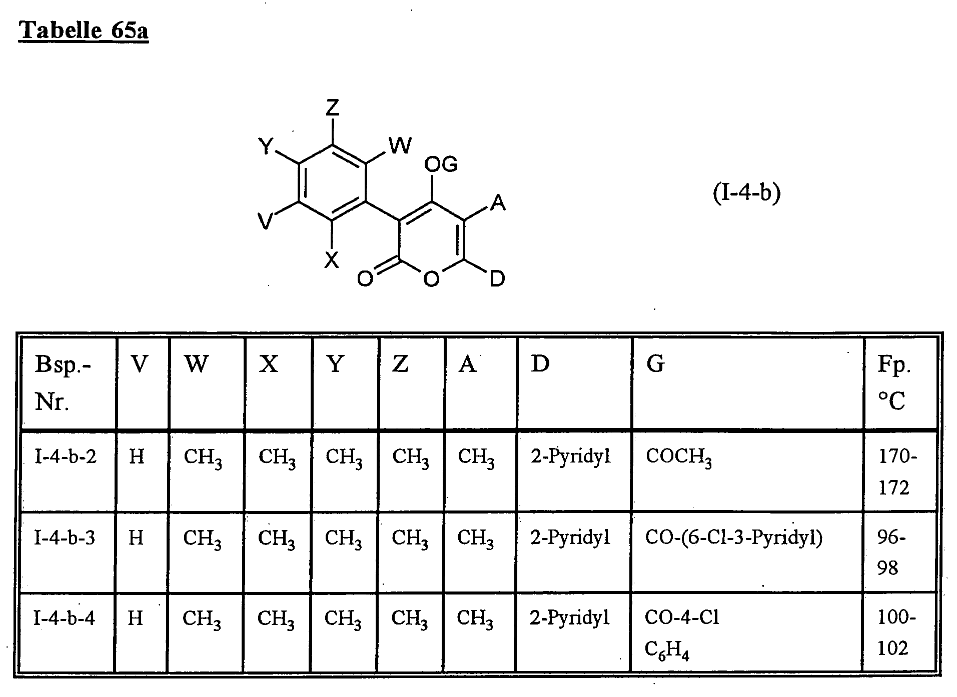

### Beispiel I-4-c-1

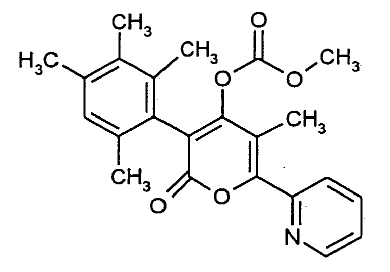

1,7 g (5 mmol) der Verbindung gemäß Beispiel I-4-a-6 werden in 20 ml Ethylacetat p.A. vorgelegt. Dazu gibt man bei 20°C 0,5 g (5 mmol) Triethylamin und tropft bei 0°C 0,47 g (5 mmol) ClCO₂CH₃ in 5 ml Ethylacetat zu. Die Reaktion wird unter Feuchtigkeitsausschluß gefahren. Man rührt 20 Stunden bei 20°C. Aufarbeitung: Abtrennung des Niederschlags, Waschen mit Ethylacetat. Die organische Phase wird mit 2 x 25 ml halbkonzentrierter NaCl-Lösung gewaschen, über Natriumsulfat getrocknet und eingedampft.
Ausbeute 1,6 g (81 % der Theorie), Fp.: 136 bis 139°C.

### Beispiel (VI-1)

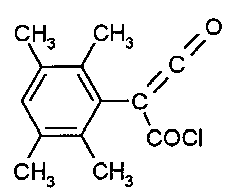

Zu 15 g (63,5 mmol) der Verbindung gemäß Beispiel (XXXIII-1) in 20 ml Toluol tropft man bei Raumtemperatur 31 ml Thionylchlorid und rührt zunächst 1 Stunde bei Raumtemperatur, dann bei ca. 95°C über Nacht. Nach dem Abkühlen treibt man bei etwa 45°C HCl und SO₂ mit durchgeleitetem Argon aus und entfernt anschließend Reste flüchtiger Verbindungen im Hochvakuum.

Ausbeute: 7,4 g (52 % der Theorie),. Kp.: 110-1H°C/0.08 mbar.

### Beispiel (VI-2)

Analog zu Beispiel (VI-1) bzw. gemäß den allgemeinen Angaben zur Herstellung erhält man die Verbindung

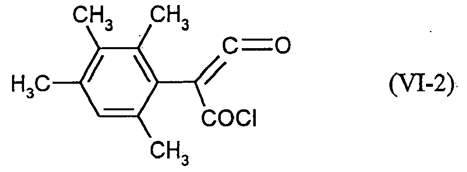

¹H-NMR (400 MHz, CDCl₃): δ = 2.13 (s, 3H, Ar-CH₃), 2.22 (s, 3H, Ar-CH₃), 2.28 (s, 6H, 2 x Ar-CH₃); 6.94 (s, 1H, Ar-H).

### Beispiel (XXXIII-1)

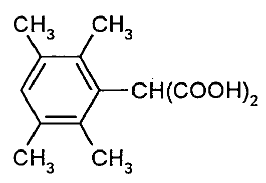

Zu 28,2 g Kaliumhydroxid in 37,8 g Wasser und 75,6 ml Methanol trägt man bei Raumtemperatur 40 g (0,155 Mol) der Verbindung gemäß Beispiel (XXXIV-1) ein und erhitzt 10 Stunden unter Rückfluß.

Nach dem Abkühlen wird im Vakuum eingeengt, der Rückstand mit ca. 100 ml Eiswasser gelöst und unter Kühlung mit halbkonzentrierter Salzsäure angesäuert. Man trennt die Phasen und extrahiert mit Toluol. Der sich abscheidende Feststoff wird abgesaugt, mit wenig Toluol verrührt und erneut abgesaugt.

Ausbeute: 35,20 g (70,3 % der Theorie). Fp.: 193-198°C.

### Beispiel (XXXIII-2)

Analog zu Beispiel (XXXIII-1) bzw. gemäß den allgemeinen Angaben zur Herstellung erhält man die Verbindung 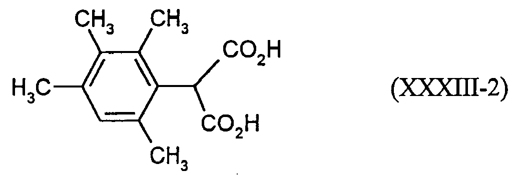 vom Fp.: 181°C (Zersetzung).

### Beispiel (XXXIV-1)

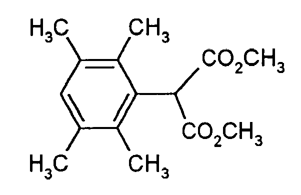

Zu 7,9 g (0,264 Mol) 80 %igem Natriumhydrid tropft man bei Raumtemperatur 350 g (3,5 Mol) Dimethylcarbonat und anschließend bei 85-90°C langsam 42,0 g (0,203 Mol) 2,3,5,6-Tetramethylphenylessigsäuremethylester und rührt über Nacht. Man läßt abkühlen, gibt etwas Methanol zu, gießt auf ca. 1 1 Eiswasser und säuert mit halbkonzentrierter Salzsäure an. Die organische Phase wird abgetrennt, die wässrige Phase mit Toluol extrahiert, die vereinigten organischen Phasen werden getrocknet und eingeengt. Anschließend wird im Hochvakuum destilliert.

Ausbeute: 41,9 g (82 % der Theorie). Fp.: 125-129°C.

Analog zu Beispiel (XXXIV-1) und gemäß den allgemeinen Angaben zur Herstellung erhält man die Verbindung 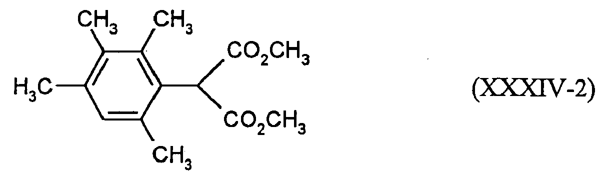 vom Fp.: 132-134°C.

### Beispiel (XXIII-1)

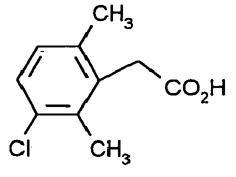

Zu einer Mischung aus 85,8 g (1,532 Mol) KOH in 110,2 ml Wasser und 224 ml Methanol tropft man bei Raumtemperatur 171,9 g der Verbindung gemäß Beispiel XXVI-1 und erhitzt 5 h unter Rückfluß. Nach dem Abkühlen wird mit 300 ml Wasser verdünnt, mit Methyl-tert.-butylether gewaschen. Die wäßrige Phase wird mit halbkonzentrierter Salzsäure angesäuert, abgesaugt, getrocknet und der Rückstand aus Toluol umkristallisiert.

Ausbeute: 111,4 g (Δ 69 % der Theorie) Fp.: 128-130°C.

Analog zu Beispiel (XXIII-1) und gemäß den allgemeinen Angaben zur Herstellung wurden folgende Verbindungen der Formel (XXIII) erhalten:

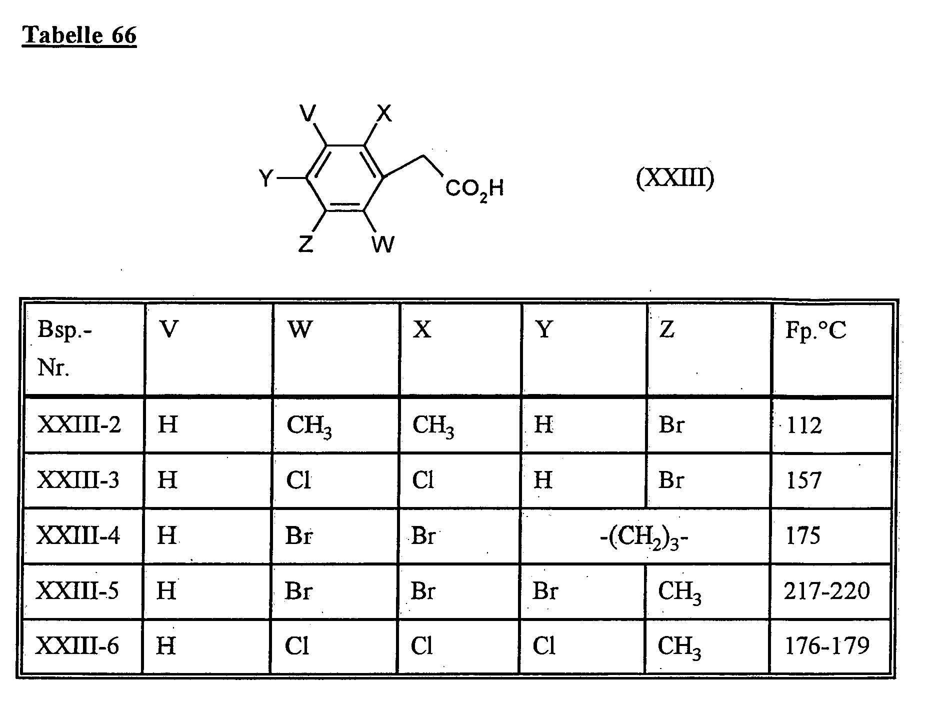

### Beispiel (XXIV-1)

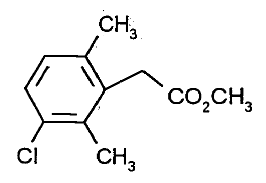

Zu einer Lösung von 353,7 g (1,3 Mol) der Verbindung gemäß Beispiel XXV-1 in 560 ml Methanol tropft man bei Raumtemperatur 992 ml 30 %ige Natriummethylatlösung in Methanol und kocht 5 h unter Rückfluß. Nach Abkühlen auf Raumtemperatur werden 148 ml konz. Schwefelsäure zugetropft, eine Stunde unter Rückfluß erwärmt, abgekühlt, eingeengt, mit Wasser versetzt, mit Methylenchlorid extrahiert, getrocknet und eingeengt. Man erhält 179,1 g eines Öls aus dem gewünschten Produkt XXIV-1 (ca. 51 % nach GC), der Säure XXIII-1 und 1,3-Dichlor-2,6-dimethylbenzol. Für die Umsetzung gemäß Beispiel XXIII-1 wurde das Gemisch verwendet.

Analog zu Beispiel (XXIV-1) und gemäß den allgemeinen Angaben zur Herstellung wurden folgende Verbindungen der Formel (XXIV) erhalten:

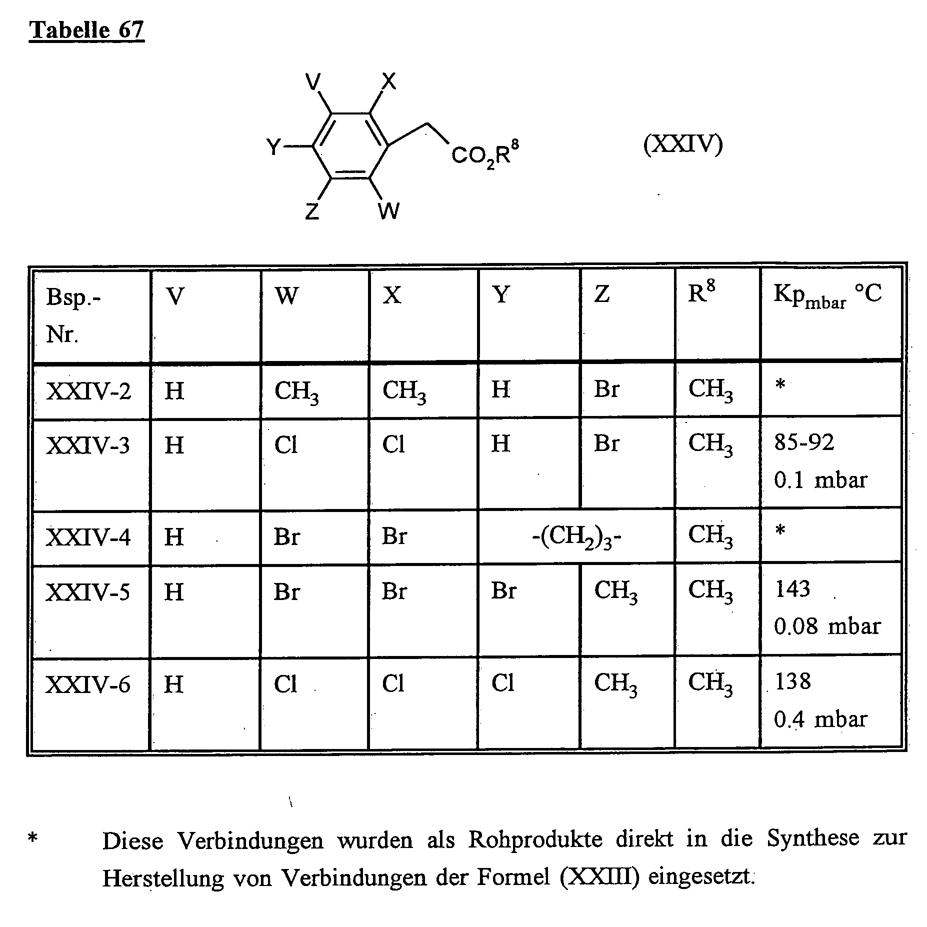

### Beispiel (XXV-1)

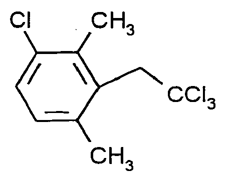

Zu einer gut gekühlten Mischung von 229,7 g (2,27 Mol) tert.-Butylnitrit und 255 g (1,776 Mol) wasserfreiem Kupfer-II-chlorid in 990 ml wasserfreiem Acetonitril tropft man 2205 g (22,8 Mol) 1,1-Dichlorethylen (Vinylidenchlorid), wobei man die Mischung auf Raumtemperatur hält. Dann wird bei einer Temperatur von unter 30°C eine Mischung aus 232 g (1,49 Mol) 3-Chlor-2,6-dimethylanilin in 1500 ml wasserfreiem Acetonitril zugetropft. Es wird bei Raumtemperatur bis zum Ende der Gasentwicklung gerührt, die Mischung wird dann vorsichtig in 6 Liter 20 %ige Salzsäure gegossen und mit Methylenchlorid extrahiert. Die organischen Phasen werden vereint und erneut mit 20 %iger Salzsäure gewaschen, getrocknet und eingeengt. Das zurückbleibende Öl wird ohne weitere Aufarbeitung für die Umsetzung gemäß Beispiel (XXIV-1) verwendet.

Analog zu Beispiel (XXV-1) und gemäß den allgemeinen Angaben zur Herstellung wurden folgende Verbindungen der Formel (XXV) erhalten:

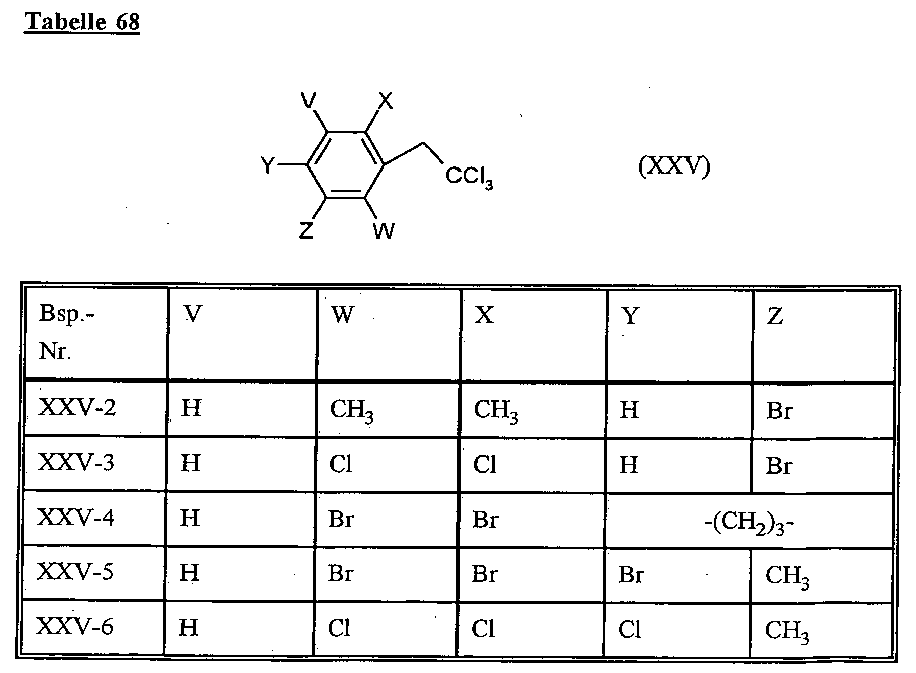

Die in der Tabelle 68 aufgeführten Verbindungen wurden als Rohprodukte in die Verseifung zu den Verbindungen der Formel (XXIV) eingesetzt und deshalb nicht näher charakterisiert.

### Anwendungsbeispiele

### Beispiel 1

**Phaedon-Larven-Test**

| | | |
|---|---|---|
| Lösungsmittel: | 7 Gewichtsteile | Dimethylformamid |
| Emulgator: | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käfer-Larven abgetötet wurden; 0 % bedeutet, daß keine Käfer-Larven abgetötet wurden.

In diesem Test bewirkten z.B. die Verbindungen gemäß Herstellungsbeispielen (I-1-a-2), (I-1-a-3), (I-1-b-1), (I-1-b-2) und (I-1-c-1) bei einer beispielhaften Wirkstoffkonzentration von 1000 ppm eine Abtötung von 100 % nach 7 Tagen.

### Beispiel 2

**Tetranychus-Test (OP-resistent/Spritzbehandlung)**

| | | |
|---|---|---|
| Lösungsmittel: | 3 Gewichtsteile | Dimethylformamid |
| Emulgator: | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschten Konzentrationen.

Bohnenpflanzen (Phaseolus vulgaris), die stark von allen Entwicklungsstadien der gemeinen Spinnmilbe oder Bohnenspinnmilbe (Tetranychus urticae) befallen sind, werden mit einer Wirkstoffzubereitung der gewünschten Konzentration gespritzt.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

In diesem Test hatte z.B. die Verbindung gemäß Herstellungsbeispiel (I-1-a-2) bei einer beispielhaften Wirkstoffkonzentration von 1000 ppm eine Wirkung von 100 % nach 7 Tagen.

### Beispiel 3

**Plutella-Test**

| | | |
|---|---|---|
| Lösungsmittel: | 7 Gewichtsteile | Dimethylformamid |
| Emulgator: | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

In diesem Test bewirkten z.B. die Verbindungen gemäß den Herstellungsbeispielen (I-1-a-3), (I-1-b-1), (I-1-b-2), (I-1-b-6) und (I-1-c-1) bei einer beispielhaften Wirkstoffkonzentration von 0,1 % eine Abtötung von 100 % nach 7 Tagen.

### Beispiel 4

t

**Spodoptera-Tes**

| | | |
|---|---|---|
| Lösungsmittel: | 7 Gewichtsteile | Dimethylformamid |
| Emulgator: | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Eulenfalters Spodoptera frugiperda) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

In diesem Test bewirkten z.B. die Verbindungen gemäß Herstellungsbeispielen (I-1-a-2), (I-1-a-3) und (I-1-b-4) bei einer beispielhaften Wirkstoffkonzentration von 0,1 % eine Abtötung von mindestens 80 % nach 7 Tagen.

### Beispiel 5

**Nephotettix-Test**

| | | |
|---|---|---|
| Lösungsmittel: | 7 Gewichtsteile | Dimethylformamid |
| Emulgator: | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Reiskeimlinge (Oryzae sativa) werden durch Tauchen in die Wirkstoffzubereitung der gewünschten Konzentration behandelt und mit Larven der Grünen Reiszikade (Nephotettix cincticeps) besetzt, solange die Keimlinge noch feucht sind.

Nach der gewünschten Zeit wird die Abtötung in % bestimmt. Dabei bedeutet 100 %, daß alle Zikaden abgetötet wurden; 0 % bedeutet, daß keine Zikaden abgetötet wurden.

In diesem Test bewirkten z.B. die Verbindungen gemäß Herstellungsbeispielen (I-1-a-1), (I-1-a-2), (I-1-a-3), (I-1-b-1), (I-1-b-2), (I-1-b-3), (I-1-b-4), (I-1-b-5), (I-1-b-6) und (I-1-c-2) bei einer beispielhaften Wirkstoffkonzentration von 0,1 % eine Abtötung von 100 % nach 6 Tagen.

### Beispiel 6

**Myzus-Test**

| | | |
|---|---|---|
| Lösungsmittel: | 7 Gewichtsteile | Dimethylformamid |
| Emulgator: | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea), die stark von der Pfirsichblattlaus (Myzus persicae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Blattläuse abgetötet wurden; 0 % bedeutet, daß keine Blattläuse abgetötet wurden.

In diesem Test bewirkten z.B. die Verbindungen gemäß den Herstellungsbeispielen (I-1-a-1), (I-1-a-2), (I-1-a-3), (I-1-b-2), (I-1-b-3) und (I-2-a-1) bei einer beispielhaften Wirkstoffkonzentration von 0,1 % eine Abtötung von mindestens 80 % nach 6 Tagen.

### Beispiel 7

**Tetranychus-Test (OP-resistent/Tauchbehandlung)**

| | | |
|---|---|---|
| Lösungsmittel: | 3 Gewichtsteile | Dimethylformamid |
| Emulgator: | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe Tetranychus urticae befallen sind, werden in eine Wirkstoffzubereitung der gewünschten Konzentration getaucht.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

In diesem Test bewirkten z.B. die Verbindungen gemäß den Herstellungsbeispielen (I-1-a-1), (I-1-a-2), (I-1-a-3), (I-1-b-2) und (I-2-b-1) bei einer beispielhaften Wirkstoffkonzentration von 0,1 % eine Abtötung von 100 % nach 5 Tagen.

## Patentansprüche

1. Verbindungen der Formel (I) 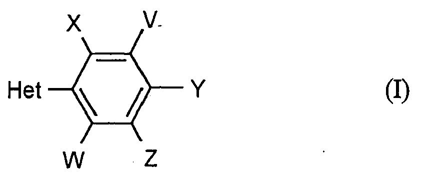 in welcher
V für Wasserstoff, Halogen, Alkyl oder Alkoxy steht,
W für Cyano, Nitro, Halogen, Alkyl, Alkenyl, Alkinyl, Alkoxy, Halogenalkyl, Halogenalkoxy, jeweils gegebenenfalls substituiertes Phenyl, Phenoxy, Phenylthio, Phenylalkoxy oder Phenylalkylthio steht,
X für Wasserstoff, Halogen, Alkyl, Alkenyl, Alkinyl, Alkoxy, Halogenalkyl, Halogenalkoxy, Cyano, Nitro oder für jeweils gegebenenfalls substituiertes Phenyl, Phenoxy, Phenylthio, Phenylalkyloxy oder Phenylalkylthio steht,
Y für Wasserstoff, Halogen, Alkyl, Alkoxy, Halogenalkyl, Halogenalkoxy, Cyano oder Nitro steht,
Z für Halogen, Alkyl, Alkoxy, Halogenalkyl, Halogenalkoxy, Hydroxy, Cyano, Nitro oder jeweils gegebenenfalls substituiertes Phenoxy, Phenylthio, 5- bis 6-gliedriges Hetaryloxy, 5- bis 6-gliedriges Hetarylthio, Phenylalkyloxy oder Phenylalkylthio steht,
Y und Z gemeinsam mit den Kohlenstoffatomen, an die sie gebunden sind, für einen gegebenenfalls substituierten und gegebenenfalls durch ein oder mehrere Heteroatome unterbrochenen Cyclus stehen, wobei V, X und W eine der obengenannten Bedeutungen haben oder
W und Z gemeinsam mit den Kohlenstoffatomen, an die sie gebunden sind, einen gegebenenfalls substituierten und gegebenenfalls durch ein oder mehrere Heteroatome unterbrochenen Cyclus stehen, wobei V,
X und Y eine der obengenannten Bedeutungen haben,
Het Het für die Gruppe 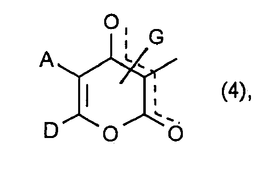 steht,
worin
A A für Wasserstoff, für jeweils gegebenenfalls durch Halogen substituiertes Alkyl, Alkenyl, Alkoxyalkyl, Polyalkoxyalkyl oder Alkylthioalkyl, für jeweils gesättigtes oder ungesättigtes und gegebenenfalls substituiertes Cycloalkyl oder Heterocyclyl oder für jeweils gegebenenfalls durch Halogen, Alkyl, Halogenalkyl, Alkoxy, Halogenalkoxy, Cyano oder Nitro substituiertes Aryl, Arylalkyl oder Hetaryl steht,
D für Wasserstoff oder für einen gegebenenfalls substituierten. Rest aus der Reihe Alkyl, Alkenyl, Alkinyl, Alkoxyalkyl, Polyalkoxyalkyl, Alkylthioalkyl, gesättigtes oder ungesättigtes Cycloalkyl, gesättigtes oder ungesättigtes Heterocyclyl, Arylalkyl, Aryl, Hetarylalkyl oder Hetaryl steht, oder
A und D gemeinsam mit den Atomen an die sie gebunden sind für einen jeweils gegebenenfalls substituierten Carbocyclus oder Heterocyclus stehen,
G für Wasserstoff (a) oder für eine der Gruppen 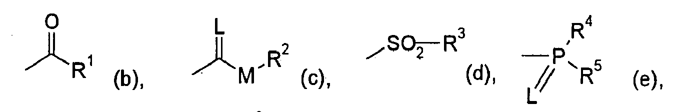 E(f) oder 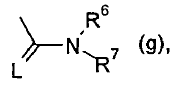 steht, worin
E für ein Metallionäquivalent oder ein Ammoniumion steht,
L für Sauerstoff oder Schwefel steht,
M für Sauerstoff oder Schwefel steht,
R¹ für jeweils gegebenenfalls durch Halogen substituiertes Alkyl, Alkenyl, Alkoxyalkyl, Alkylthioalkyl oder Polyalkoxyalkyl oder für jeweils gegebenenfalls durch Halogen, Alkyl oder Alkoxy substituiertes Cycloalkyl oder Heterocyclyl oder für jeweils gegebenenfalls substituiertes Phenyl, Phenylalkyl, Hetaryl, Phenoxyalkyl oder Hetaryloxyalkyl steht,
R² für jeweils gegebenenfalls durch Halogen substituiertes Alkyl, Alkenyl, Alkoxyalkyl oder Polyalkoxyalkyl oder für jeweils gegebenenfalls substituiertes Cycloalkyl, Phenyl oder Benzyl steht,
R³, R⁴ und R⁵ unabhängig voneinander für jeweils gegebenenfalls durch Halogen substituiertes Alkyl, Alkoxy, Alkylamino, Dialkylamino, Alkylthio, Alkenylthio oder Cycloalkylthio oder für jeweils gegebenenfalls substituiertes Phenyl, Benzyl, Phenoxy oder Phenylthio stehen,
R⁶ und R⁷ unabhängig voneinander für Wasserstoff, für jeweils gegebenenfalls durch Halogen substituiertes Alkyl, Cycloalkyl, Alkenyl, Alkoxy oder Alkoxyalkyl oder für jeweils gegebenenfalls substituiertes Phenyl oder Benzyl stehen, oder gemeinsam mit dem N-Atom, an das sie gebunden sind, einen gegebenenfalls Sauerstoff oder Schwefel enthaltenden und gegebenenfalls substituierten Cyclus bilden,

2. Verfahren zur Herstellung von Verbindungen der Formel (I) gemäß Anspruch 1, **dadurch gekennzeichnet, daß** man
(D) Verbindungen der Formel (I-4-a) 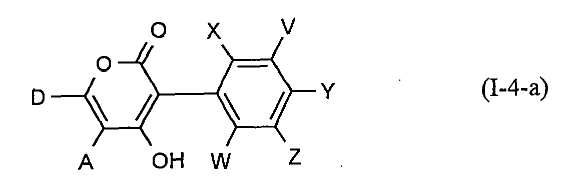 in welcher
A, D, V, W, X, Y und Z die oben angegebenen Bedeutungen haben,
erhält, wenn man
Verbindungen der Formel (V) 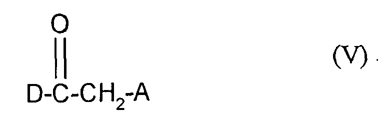 in welcher
A und D die oben angegebenen Bedeutungen haben,
oder deren Silylenolether der Formel (Va) 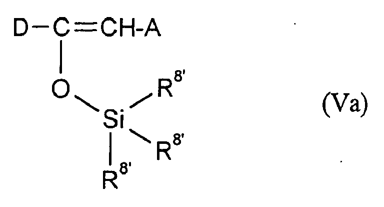 in welcher
A und D die obengenannte Bedeutung haben und
R^{8'} für Alkyl (bevorzugt Methyl) steht,
mit Verbindungen der Formel (VI) 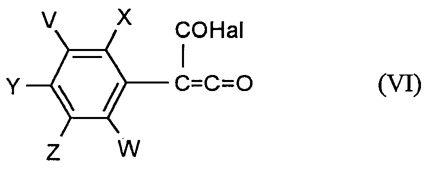 in welcher
V, W, X, Y und Z die oben angegebenen Bedeutungen haben und
Hal für Halogen (vorzugsweise für Chlor oder Brom) steht,
gegebenenfalls in Gegenwart eines Verdünnungsmittels und gegebenenfalls in Gegenwart eines Säureakzeptors umsetzt,
und gegebe-nenfalls anschließend die so erhaltenen Verbindungen der Formel (I-4-a)
(Fα) mit Säurehalogeniden der Formel (VIII) 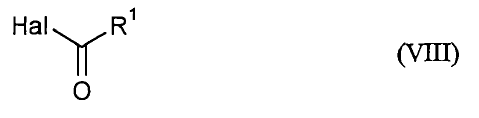 in welcher
R¹ die oben angegebene Bedeutung hat und
Hal für Halogen (insbesondere Chlor oder Brom) steht oder
(Fβ) mit Carbonsäureanhydriden der Formel (IX)
R¹-CO-O-CO-R¹ (IX)
in welcher
R¹ die oben angegebene Bedeutung hat,
gegebenenfalls in Gegenwart eines Verdünnungsmittels und gegebenenfalls in Gegenwart eines Säurebindemittels umsetzt;
oder
(G) mit Chlorameisensäureestern oder Chlorameisensäurethioestern der Formel (X)
R²-M-CO-Cl (X)
in welcher
R² und M die oben angegebenen Bedeutungen haben,
gegebenenfalls in Gegenwart eines Verdünnungsmittels und gegebenenfalls in Gegenwart eines Säurebindemittels umsetzt;
oder
(Hα) mit Chlormonothioameisensäureestern oder Chlordithioameisensäureestem der Formel (XI) 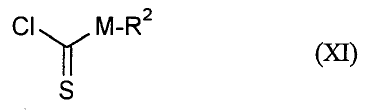 in welcher
M und R² die oben angegebenen Bedeutungen haben,
gegebenenfalls in Gegenwart eines Verdünnungsmittels und gegebenenfalls in Gegenwart eines Säurebindemittels umsetzt
oder
(Hβ) mit Schwefelkohlenstoff und anschließend mit Verbindungen der Formel (XII)
R²-Hal (XII)
in welcher
R² die oben angegebene Bedeutung hat und
Hal für Chlor, Brom oder Iod steht,
gegebenenfalls in Gegenwart eines Verdünnungsmittels und gegebenenfalls in Gegenwart einer Base umsetzt,
oder
(I) mit Sulfonsäurechloriden der Formel (XIII)
R³-SO₂-Cl (XIII)
in welcher
R³ die oben angegebene Bedeutung hat,
gegebenenfalls in Gegenwart eines Verdünnungsmittels und gegebenenfalls in Gegenwart eines Säurebindemittels umsetzt,
oder
(J) mit Phosphorverbindungen der Formel (XIV) 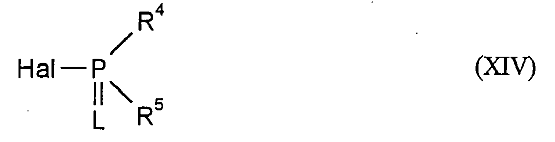 in welcher
L, R⁴ und R⁵ die oben angegebenen Bedeutungen haben und
Hal für Halogen (insbesondere Chlor oder Brom) steht,
gegebenenfalls in Gegenwart eines Verdünnungsmittels und gegebenenfalls in Gegenwart eines Säurebindemittels umsetzt,
oder
(K) mit Metallverbindungen oder Aminen der Formeln (XV) oder (XVI) 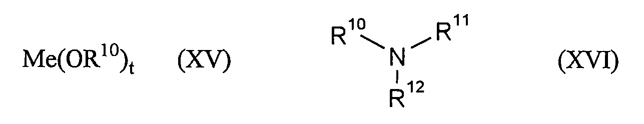 in welchen
Me für ein ein- oder zweiwertiges Metall (bevorzugt ein Alkali- oder Erdalkalimetall wie Lithium, Natrium, Kalium, Magnesium oder Calcium),
t für die Zahl 1 oder 2 und
R¹⁰, R¹¹, R¹² unabhängig voneinander für Wasserstoff oder Alkyl (bevorzugt C₁-C₈-Alkyl) stehen,
gegebenenfalls in Gegenwart eines Verdünnungsmittels umsetzt,
oder
(Lα) mit Isocyanaten oder Isothiocyanaten der Formel (XVII)
R⁶-N=C=L (XVII)
in welcher
R⁶ und L die oben angegebenen Bedeutungen haben,
gegebenenfalls in Gegenwart eines Verdünnungsmittels und gegebenenfalls in Gegenwart eines Katalysators umsetzt oder
(Lβ) mit Carbamidsäurechloriden oder Thiocarbamidsäurechloriden der Formel (XVIII) 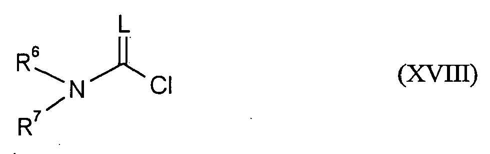 in welcher
L, R⁶ und R⁷ die oben angegebenen Bedeutungen haben,
gegebenenfalls in Gegenwart eines Verdünnungsmittels und gegebenenfalls in Gegenwart eines Säurebindemittels, umsetzt.

3. Verbindungen der Formel (I) gemäß Anspruch 1, worin
V ür Wasserstoff, Halogen, C₁-C₆-Alkyl oder C₁-C₆-Alkoxy steht,
W für Cyano, Nitro, Halogen, C₁-C₆-Alkyl, C₂-C₆-Alkenyl, C₂-C₆₋Alkinyl, C₁-C₆-Alkoxy, C₁-C₄-Halogenalkyl, C₁-C₄-Halogenalkoxy oder jeweils gegebenenfalls durch Halogen, C₁-C₆-Alkyl, C₁-C₆₋Alkoxy, C₁-C₄-Halogenalkyl, C₁-C₄-Halogenalkoxy, Nitro oder Cyano substituiertes Phenyl, Phenoxy, Phenylthio, Phenyl-C₁-C₄₋alkoxy oder Phenyl-C₁-C₄-alkylthio, steht
X für Wasserstoff, Halogen, C₁-C₆-Alkyl, C₂-C₆-Alkenyl, C₂-C₆₋Alkinyl, C₁-C₆-Alkoxy, C₁-C₄-Halogenalkyl, C₁-C₄-Halogenalkoxy, Cyano, Nitro oder jeweils gegebenenfalls durch Halogen, C₁-C₆₋Alkyl, C₁-C₆-Alkoxy, C₁-C₄-Halogenalkyl, C₁-C₄-Halogenalkoxy, Nitro oder Cyano substituiertes Phenyl, Phenoxy, Phenylthio, Phenyl-C₁-C₄-alkoxy oder Phenyl-C₁-C₄-alkylthio, steht
Y für Wasserstoff, Halogen, C₁-C₆-Alkyl, C₁-C₆-Alkoxy, C₁-C₄-Halogenalkyl, C₁-C₄-Halogenalkoxy, Cyano oder Nitro, steht
Z für Halogen, C₁-C₆-Alkyl, C₁-C₆-Alkoxy, C₁-C₄-Halogenalkyl, C₁-C₄-Halogenalkoxy, Hydroxy, Cyano, Nitro oder jeweils gegebenenfalls durch Halogen, C₁-C₄-Alkyl, C₁-C₄-Alkoxy, C₁-C₄-Halogenalkyl, C₁-C₄-Halogenalkoxy, Nitro oder Cyano substituiertes Phenoxy, Phenylthio, Thiazolyloxy, Pyridinyloxy, Pyrimidyloxy, Pyrazolyloxy, Phenyl-C₁-C₄-alkyloxy oder Phenyl-C₁-C₄-alkylthio steht, oder
Y und Z gemeinsam für jeweils gegebenenfalls durch Halogen, C₁-C₆₋Alkyl, C₁-C₆-Alkoxy oder C₁-C₄-Halogenalkyl substituiertes C₃-C₅₋Alkandiyl oder C₃-C₅-Alkendiyl stehen, in welchen gegebenenfalls ein bis drei Glieder durch Sauerstoff, Schwefel, Stickstoff oder eine Carbonylgruppe unabhängig voneinander ersetzt sein können, oder
W und Z gemeinsam für jeweils gegebenenfalls durch Halogen, C₁-C₆₋Alkyl, C₁-C₆-Alkoxy oder C₁-C₄-Halogenalkyl substituiertes C₃-C₅₋Alkandiyl oder C₃-C₅-Alkendiyl stehen, in welchen gegebenenfalls ein bis drei Glieder durch Sauerstoff, Schwefel, Stickstoff oder eine Carbonylgruppe unabhängig voneinander ersetzt sein können,
Het für die Gruppe 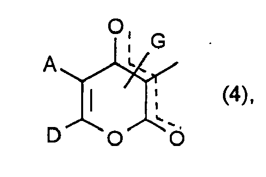 steht,
A für Wasserstoff, für jeweils gegebenenfalls durch Halogen substituiertes C₁-C₁₂-Alkyl, C₂-C₈-Alkenyl, C₁-C₁₀-Alkoxy-C₁-C₈-alkyl, Poly-C₁-C₈-alkoxy-C₁-C₈-alkyl oder C₁-C₁₀-Alkylthio-C₁-C₆-alkyl, für gegebenenfalls durch Halogen, C₁-C₆-Alkyl oder C₁-C₆-Alkoxy substituiertes C₃-C₈-Cycloalkyl, in welchem gegebenenfalls eine oder zwei nicht direkt benachbarte Methylengruppen durch Sauerstoff und/oder Schwefel ersetzt sind oder für jeweils gegebenenfalls durch Halogen, C₁-C₆-Alkyl, C₁-C₆-Halogenalkyl, C₁-C₆₋Alkoxy, C₁-C₆-Halogenalkoxy, Cyano oder Nitro substituiertes Phenyl, Naphthyl, Phenyl-C₁-C₆-alkyl, Naphthyl-C₁-C₆-alkyl oder Hetaryl mit 5 oder 6 Ringatomen und ein bis drei Heteroatomen aus der Reihe Sauerstoff, Schwefel und Stickstoff steht,
D für Wasserstoff, für jeweils gegebenenfalls durch Halogen substituiertes C₁-C₁₂-Alkyl, C₃-C₈-Alkenyl, C₃-C₈-Alkinyl, C₁-C₁₀-Alkoxy-C₂-C₈-alkyl, Poly-C₁-C₈-alkoxy-C₂-C₈-alkyl oder C₁-C₁₀-Alkylthio-C₂-C₈-alkyl, für gegebenenfalls durch Halogen, C₁-C₄-Alkyl, C₁-C₄₋Alkoxy oder C₁-C₄-Halogenalkyl substituiertes C₃-C₈-Cycloalkyl, in welchem gegebenenfalls eine oder zwei nicht direkt benachbarte Methylengruppen durch Sauerstoff und/oder Schwefel ersetzt sind oder für jeweils gegebenenfalls durch Halogen, C₁-C₆-Alkyl, C₁-C₆₋Halogenalkyl, C₁-C₆-Alkoxy, C₁-C₆-Halogenalkoxy, Cyano oder Nitro substituiertes Phenyl, Hetaryl mit 5 bis 6 Ringatomen und ein oder zwei Heteroatomen aus der Reihe Sauerstoff, Schwefel und Stickstoff, Phenyl-C₁-C₆-alkyl oder Hetaryl-C₁-C₆-alkyl mit 5 bis 6 Ringatomen und ein oder zwei Heteroatomen aus der Reihe Sauerstoff, Schwefel und Stickstoff steht, oder
A und D gemeinsam für eine C₃-C₆-Alkandiyl-, C₃-C₆-Alkendiyl- oder C₄-C₆-Alkadiendiylgruppe stehen, in welchen jeweils gegebenenfalls eine Methylengruppe durch Sauerstoff oder Schwefel ersetzt ist und welche jeweils gegebenenfalls substituiert sind durch Halogen oder durch jeweils gegebenenfalls durch Halogen substituiertes C₁-C₁₀₋Alkyl, C₁-C₆-Alkoxy, C₁-C₆-Alkylthio, C₃-C₇-Cycloalkyl, Phenyl oder Benzyloxy oder durch eine weitere, einen ankondensierten Ring bildende C₃-C₆-Alkandiyl-, C₃-C₆-Alkendiyl- oder C₄-C₆₋Alkadiendiylgruppe, in welchen gegebenenfalls jeweils eine Methylengruppe durch Sauerstoff oder Schwefel ersetzt ist und welche gegebenenfalls durch C₁-C₆-Alkyl substituiert sind, oder
A und D gemeinsam für eine C₃-C₆-Alkandiyl- oder C₃-C₆-Alkendiylgruppe stehen, worin jeweils gegebenenfalls eine der folgenden Gruppen 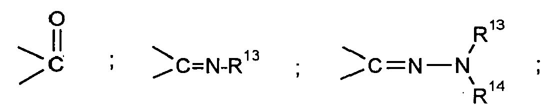 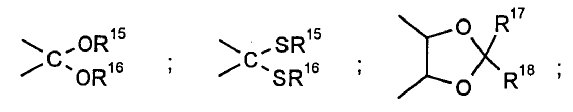 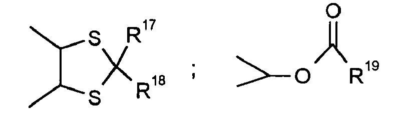 oder 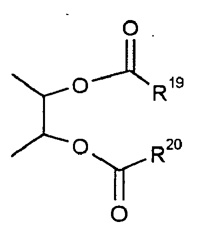 enthalten ist,
G für Wasserstoff (a) oder für eine der Gruppen 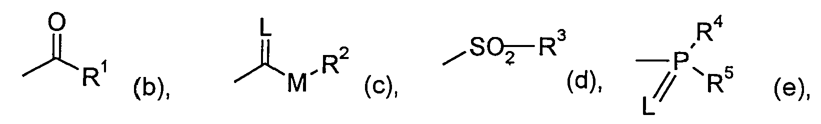 E (f) oder 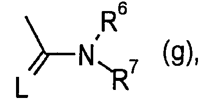 steht,
in welchen
E für ein Metallionäquivalent oder ein Ammoniumion steht,
L für Sauerstoff oder Schwefel steht und
M für Sauerstoff oder Schwefel steht,
R¹ für jeweils gegebenenfalls durch Halogen substituiertes C₁-C₂₀₋Alkyl, C₂-C₂₀-Alkenyl, C₁-C₈-Alkoxy-C₁-C₈-alkyl, C₁-C₈-Alkylthio-C₁-C₈-alkyl oder Poly-C₁-C₈-alkoxy-C₁-C₈-alkyl oder für gegebenenfalls durch Halogen, C₁-C₆-Alkyl oder C₁-C₆-Alkoxy substituiertes C₃-C₈-Cycloalkyl, in welchem gegebenenfalls eine oder zwei nicht direkt benachbarte Methylengruppen durch Sauerstoff und/oder Schwefel ersetzt sind,
für gegebenenfalls durch Halogen, Cyano, Nitro, C₁-C₆-Alkyl, C₁-C₆-Alkoxy, C₁-C₆-Halogenalkyl, C₁-C₆-Halogenalkoxy, C₁-C₆₋Alkylthio oder C₁-C₆-Alkylsulfonyl substituiertes Phenyl,
für gegebenenfalls durch Halogen, Nitro, Cyano, C₁-C₆-Alkyl, C₁-C₆-Alkoxy, C₁-C₆-Halogenalkyl oder C₁-C₆-Halogenalkoxy substituiertes Phenyl-C₁-C₆-alkyl,
für gegebenenfalls durch Halogen oder C₁-C₆-Alkyl substituiertes 5- oder 6-gliedriges Hetaryl mit ein oder zwei Heteroatomen aus der Reihe Sauerstoff, Schwefel und Stickstoff,
für gegebenenfalls durch Halogen oder C₁-C₆-Alkyl substituiertes Phenoxy-C₁-C₆-alkyl oder
für gegebenenfalls durch Halogen, Amino oder C₁-C₆-Alkyl substituiertes 5- oder 6-gliedriges Hetaryloxy-C₁-C₆-alkyl mit ein oder zwei Heteroatomen aus der Reihe Sauerstoff, Schwefel und Stickstoff steht,
R² für jeweils gegebenenfalls durch Halogen substituiertes C₁-C₂₀-Alkyl, C₂-C₂₀-Alkenyl, C₁-C₈-Alkoxy-C₂-C₈-alkyl oder Poly-C₁-C₈₋alkoxy-C₂-C₈-alkyl,
für gegebenenfalls durch Halogen, C₁-C₆-Alkyl oder C₁-C₆-Alkoxy substituiertes C₃-C₈-Cycloalkyl oder
für jeweils gegebenenfalls durch Halogen, Cyano, Nitro, C₁-C₆₋Alkyl, C₁-C₆-Alkoxy, C₁-C₆-Halogenalkyl oder C₁-C₆-Halogenalkoxy substituiertes Phenyl oder Benzyl steht,
R³ für gegebenenfalls durch Halogen substituiertes C₁-C₈-Alkyl oder jeweils gegebenenfalls durch Halogen, C₁-C₆-Alkyl, C₁-C₆-Alkoxy, C₁-C₄-Halogenalkyl, C₁-C₄-Halogenalkoxy, Cyano oder Nitro substituiertes Phenyl oder Benzyl steht,
R⁴ und R⁵ unabhängig voneinander für jeweils gegebenenfalls durch Halogen substituiertes C₁-C₈-Alkyl, C₁-C₈-Alkoxy, C₁-C₈-Alkylamino, Di-(C₁-C₈-alkyl)amino, C₁-C₈-Alkylthio oder C₃-C₈-Alkenylthio oder für jeweils gegebenenfalls durch Halogen, Nitro, Cyano, C₁-C₄-Alkoxy, C₁-C₄-Halogenalkoxy, C₁-C₄-Alkylthio, C₁-C₄-Halogenalkylthio, C₁-C₄-Alkyl oder C₁-C₄-Halogenalkyl substituiertes Phenyl, Phenoxy oder Phenylthio stehen,
R⁶ und R⁷ unabhängig voneinander für Wasserstoff, für jeweils gegebenenfalls durch Halogen substituiertes C₁-C₈-Alkyl, C₃-C₈-Cycloalkyl, C₁-C₈-Alkoxy, C₃-C₈-Alkenyl oder C₁-C₈-Alkoxy-C₂-C₈-alkyl, für jeweils gegebenenfalls durch Halogen, C₁-C₈-Alkyl, C₁-C₈-Halogenalkyl oder C₁-C₈-Alkoxy substituiertes Phenyl oder Benzyl oder zusammen für einen gegebenenfalls durch C₁-C₆-Alkyl substituierten C₃-C₆-Alkylenrest stehen, in welchem gegebenenfalls eine Methylengruppe durch Sauerstoff oder Schwefel ersetzt ist,
R¹³ für Wasserstoff oder jeweils gegebenenfalls durch Halogen substituiertes C₁-C₈-Alkyl oder C₁-C₈-Alkoxy, für gegebenenfalls durch Halogen, C₁-C₄-Alkyl oder C₁-C₄-Alkoxy substituiertes C₃-C₈-Cycloalkyl, in welchem gegebenenfalls eine Methylengruppe durch Sauerstoff oder Schwefel ersetzt ist, oder für jeweils gegebenenfalls durch Halogen, C₁-C₆-Alkyl, C₁-C₆-Alkoxy, C₁-C₄₋Halogenalkyl, C₁-C₄-Halogenalko-xy, Nitro oder Cyano substituiertes Phenyl, Phenyl-C₁-C₄-alkyl oder Phenyl-C₁-C₄-alkoxy steht,
R¹⁴ für Wasserstoff oder C₁-C₈-Alkyl steht oder
R¹³ und R¹⁴ gemeinsam für C₄-C₆-Alkandiyl stehen,
R¹⁵ und R¹⁶ gleich oder verschieden sind und für C₁-C₆-Alkyl stehen oder
R¹⁵ und R¹⁶ gemeinsam für einen C₂-C₄-Alkandiylrest stehen, der gegebenenfalls durch C₁-C₆-Alkyl oder durch gegebenenfalls durch Halogen, C₁-C₄-Alkyl, C₁-C₄-Halogenalkyl, C₁-C₄-Alkoxy, C₁-C₄-Halogenalkoxy, Nitro oder Cyano substituiertes Phenyl substituiert ist,
R¹⁷ und R¹⁸ unabhängig voneinander für Wasserstoff, für gegebenenfalls durch Halogen substituiertes C₁-C₈-Alkyl oder für gegebenenfalls durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl stehen oder
R¹⁷ und R¹⁸ gemeinsam mit dem Kohlenstoffatom, an das sie gebunden sind, für gegebenenfalls durch C₁-C₄-Alkyl substituiertes C₅-C₇₋Cycloalkyl stehen, in dem gegebenenfalls eine Methylengruppe durch Sauerstoff oder Schwefel ersetzt ist und
R¹⁹ und R²⁰ unabhängig voneinander für C₁-C₁₀-Alkyl, C₂-C₁₀-Alkenyl, C₁-C₁₀-Alkoxy, C₁-C₁₀-Alkylamino, C₃-C₁₀-Alkenylamino, Di-(C₁-C₁₀-alkyl)amino oder Di-(C₃-C₁₀-alkenyl)amino stehen,

4. Verbindungen der Formel (I) gemäß Anspruch 1, worin
V für Wasserstoff, Fluor, Chlor, Brom, C₁-C₄-Alkyl oder C₁-C₄₋Alkoxy steht,
W für Cyano, Nitro, Fluor, Chlor, Brom, C₁-C₄-Alkyl, C₁-C₄-Alkoxy, C₁-C₂-Halogenalkyl, C₁-C₂-Halogenalkoxy oder jeweils gegebenenfalls durch Fluor, Chlor, Brom, C₁-C₄-Alkyl, C₁-C₄-Alkoxy, C₁-C₂-Halogenalkyl, C₁-C₂-Halogenalkoxy, Nitro oder Cyano substituiertes Phenyl, Phenoxy, Benzyl oder Benzyloxy steht,
X für Wasserstoff, Fluor, Chlor, Brom, C₁-C₄-Alkyl, C₁-C₄-Alkoxy, C₁-C₂-Halogenalkyl, C₁-C₂-Halogenalkoxy, Cyano, Nitro oder jeweils gegebenenfalls durch Fluor, Chlor, Brom, C₁-C₄-Alkyl, C₁-C₄₋Alkoxy, C₁-C₂-Halogenalkyl, C₁-C₂-Halogenalkoxy, Nitro oder Cyano substituiertes Phenyl, Phenoxy, Benzyl oder Benzyloxy steht,
Y für Wasserstoff, Fluor, Chlor, Brom, C₁-C₄-Alkyl, C₁-C₄-Alkoxy, C₁-C₂-Halogenalkyl, C₁-C₂-Halogenalkoxy, Cyano oder Nitro steht,
Z für Fluor, Chlor, Brom, C₁-C₄-Alkyl, C₁-C₄-Alkoxy, C₁-C₂-Halogenalkyl, C₁-C₂-Halogenalkoxy, Hydroxy, Cyano, Nitro oder jeweils gegebenenfalls durch Fluor, Chlor, Brom, C₁-C₄-Alkyl, C₁-C₄₋Alkoxy, C₁-C₂-Halogenalkyl, C₁-C₂-Halogenalkoxy, Nitro oder Cyano substituiertes Phenoxy oder Benzyloxy steht, oder
Y und Z gemeinsam für jeweils gegebenenfalls durch Fluor, Chlor, Brom, C₁-C₄-Alkyl, C₁-C₄-Alkoxy oder C₁-C₂-Halogenalkyl substituiertes C₃-C₄-Alkandiyl oder C₃-C₄-Alkendiyl stehen, worin gegebenenfalls ein oder zwei nicht direkt benachbarte Glieder durch Sauerstoff, Schwefel oder Stickstoff unabhängig voneinander ersetzt sind, oder
W und Z gemeinsam für jeweils gegebenenfalls durch Fluor, Chlor, Brom, C₁-C₄-Alkyl, C₁-C₄-Alkoxy oder C₁-C₂-Halogenalkyl substituiertes C₃-C₄-Alkandiyl oder C₃-C₄-Alkenyldiyl stehen, worin gegebenenfalls ein oder zwei nicht direkt benachbarte Glieder durch Sauerstoff, Schwefel oder Stickstoff unabhängig voneinander ersetzt sind,
Het für die Gruppe 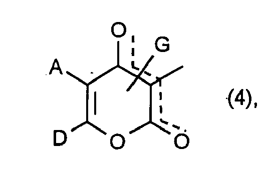 steht,
A für Wasserstoff, für jeweils gegebenenfalls durch Fluor oder Chlor substituiertes C₁-C₁₀-Alkyl, C₂-C₆-Alkenyl, C₁-C₈-Alkoxy-C₁-C₆-alkyl, Poly-C₁-C₆-alkoxy-C₁-C₆-alkyl oder C₁-C₈-Alkylthio-C₁-C₆₋alkyl oder für gegebenenfalls durch Fluor, Chlor, C₁-C₄-Alkyl oder C₁-C₄-Alkoxy substituiertes C₃-C₇-Cycloalkyl, in welchem gegebenenfalls eine oder zwei nicht direkt benachbarte Methylengruppen durch Sauerstoff und/oder Schwefel ersetzt sind oder für jeweils gegebenenfalls durch Fluor, Chlor, Brom, C₁-C₄-Alkyl, C₁-C₄₋Halogenalkyl, C₁-C₄-Alkoxy, C₁-C₄-Halogenalkoxy, Cyano oder Nitro substituiertes Phenyl, Furanyl, Pyridyl, Imidazolyl, Triazolyl, Pyrazolyl, Indolyl, Thiazolyl, Thienyl oder Phenyl-C₁-C₄-alkyl steht,
D für Wasserstoff, für jeweils gegebenenfalls durch Fluor oder Chlor substituiertes C₁-C₁₀-Alkyl, C₃-C₆-Alkenyl, C₃-C₆-Alkinyl, C₁-C₈₋Alkoxy-C₂-C₆-alkyl, Poly-C₁-C₆-alkoxy-C₂-C₆-alkyl oder C₁-C₈₋Alkylthio-C₂-C₆-alkyl, für gegebenenfalls durch Fluor, Chlor, C₁-C₄-Alkyl, C₁-C₄-Alkoxy oder C₁-C₂-Halogenalkyl substituiertes C₃-C₇-Cycloalkyl, in welchem gegebenenfalls eine oder zwei nicht direkt benachbarte Methylengruppen durch Sauerstoff und/oder Schwefel ersetzt sind oder für jeweils gegebenenfalls durch Fluor, Chlor, Brom, C₁-C₄-Alkyl, C₁-C₄-Halogenalkyl, C₁-C₄-Alkoxy, C₁-C₄-Halogenalkoxy, Cyano oder Nitro substituiertes Phenyl, Furanyl, Imidazolyl, Pyridyl, Thiazolyl, Pyrazolyl, Pyrimidyl, Pyrrolyl, Thienyl, Triazolyl oder Phenyl-C₁-C₄-alkyl steht oder
A und D für eine C₃-C₅-Alkandiyl- oder C₃-C₅-Alkendiylgruppe stehen, worin jeweils gegebenenfalls ein Kohlenstoffatom durch Sauerstoff oder Schwefel ersetzt ist und welche gegebenenfalls substituiert sind durch Fluor, Chlor oder durch jeweils gegebenenfalls durch Fluor oder Chlor substituiertes C₁-C₆-Alkyl, C₁-C₄-Alkoxy, C₁-C₄₋Alkylthio, C₃-C₆-Cycloalkyl, Phenyl oder Benzyloxy oder
worin jeweils gegebenenfalls eine der folgenden Gruppen: 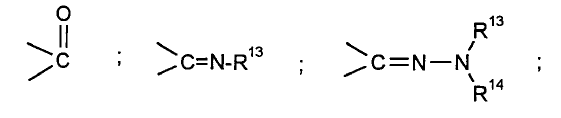 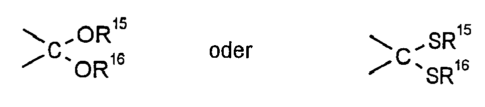 oder enthalten ist;
G für Wasserstoff (a) oder für eine der Gruppen 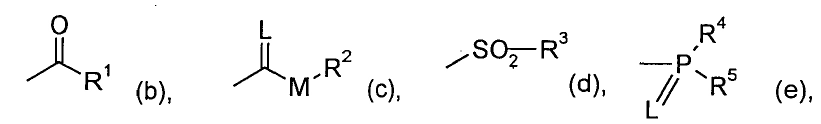 E (f) oder 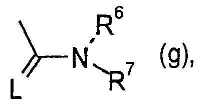 steht, in welchen
E für ein Metallionäquivalent oder ein Ammoniumion steht,
L für Sauerstoff oder Schwefel steht und
M für Sauerstoff oder Schwefel steht.
R¹ für jeweils gegebenenfalls durch Fluor oder Chlor substituiertes C₁₋C₁₆-Alkyl, C₂-C₁₆-Alkenyl, C₁-C₆-Alkoxy-C₁-C₆-alkyl, C₁-C₆₋Alkylthio-C₁-C₆-alkyl oder Poly-C₁-C₆-alkoxy-C₁-C₆-alkyl oder für gegebenenfalls durch Fluor, Chlor, C₁-C₅-Alkyl oder C₁-C₅-Alkoxy substituiertes C₃-C₇-Cycloalkyl, in welchem gegebenenfalls eine oder zwei nicht direkt benachbarte Methylengruppen durch Sauerstoff und/oder Schwefel ersetzt sind,
für gegebenenfalls durch Fluor, Chlor, Brom, Cyano, Nitro, C₁-C₄₋Alkyl, C₁-C₄-Alkoxy, C₁-C₃-Halogenalkyl, C₁-C₃-Halogenalkoxy, C₁-C₄-Alkylthio oder C₁-C₄-Alkylsulfonyl substituiertes Phenyl,
für gegebenenfalls durch Fluor, Chlor, Brom, C₁-C₄-Alkyl, C₁-C₄₋Alkoxy, C₁-C₃-Halogenalkyl oder C₁-C₃-Halogenalkoxy substituiertes Phenyl-C₁-C₄-alkyl,
für jeweils gegebenenfalls durch Fluor, Chlor, Brom oder C₁-C₄₋Alkyl substituiertes Pyrazolyl, Thiazolyl, Pyridyl, Pyrimidyl, Furanyl oder Thienyl,
für gegebenenfalls durch Fluor, Chlor, Brom oder C₁-C₄-Alkyl substituiertes Phenoxy-C₁-C₅-alkyl oder
für jeweils gegebenenfalls durch Fluor, Chlor, Brom, Amino oder C₁-C₄-Alkyl substituiertes Pyridyloxy-C₁-C₅-alkyl, Pyrimidyloxy-C₁-C₅-alkyl oder Thiazolyloxy-C₁-C₅-alkyl steht,
R² für jeweils gegebenenfalls durch Fluor oder Chlor substituiertes C₁-C₁₆-Alkyl, C₂-C₁₆-Alkenyl, C₁-C₆-Alkoxy-C₂-C₆-alkyl oder Poly-C₁-C₆-alkoxy-C₂-C₆-alkyl,
für gegebenenfalls durch Fluor, Chlor, C₁-C₄-Alkyl oder C₁-C₄₋Alkoxy substituiertes C₃-C₇-Cycloalkyl oder
für jeweils gegebenenfalls durch Fluor, Chlor, Brom, Cyano, Nitro, C₁-C₄-Alkyl, C₁-C₃-Alkoxy, C₁-C₃-Halogenalkyl oder C₁-C₃₋Halogenalkoxy substituiertes Phenyl oder Benzyl steht,
R³ für gegebenenfalls durch Fluor oder Chlor substituiertes C₁-C₆₋Alkyl oder jeweils gegebenenfalls durch Fluor, Chlor, Brom, C₁-C₄₋Alkyl, C₁-C₄-Alkoxy, C₁-C₂-Halogenalkoxy, C₁-C₂-Halogenalkyl, Cyano oder Nitro substituiertes Phenyl oder Benzyl steht,
R⁴ und R⁵ unabhängig voneinander für jeweils gegebenenfalls durch Fluor oder Chlor substituiertes C₁-C₆-Alkyl, C₁-C₆-Alkoxy, C₁-C₆₋Alkylamino, Di-(C₁-C₆-alkyl)amino, C₁-C₆-Alkylthio oder C₃-C₄₋Alkenylthio oder für jeweils gegebenenfalls durch Fluor, Chlor, Brom, Nitro, Cyano, C₁-C₃-Alkoxy, C₁-C₃-Halogenalkoxy, C₁-C₃₋Alkylthio, C₁-C₃-Halogenalkylthio, C₁-C₃-Alkyl oder C₁-C₃-Halogenalkyl substituiertes Phenyl, Phenoxy oder Phenylthio stehen,
R⁶ und R⁷ unabhängig voneinander für Wasserstoff, für jeweils gegebenenfalls durch Fluor oder Chlor substituiertes C₁-C₆-Alkyl, C₃-C₆-Cycloalkyl, C₁-C₆-Alkoxy, C₃-C₆-Alkenyl oder C₁-C₆-Alkoxy-C₂-C₆-alkyl, für jeweils gegebenenfalls durch Fluor, Chlor, Brom, C₁-C₅-Halogenalkyl, C₁-C₅-Alkyl oder C₁-C₅-Alkoxy substituiertes Phenyl oder Benzyl, oder zusammen für einen gegebenenfalls durch C₁-C₄-Alkyl substituierten C₃-C₆-Alkylenrest stehen, in welchem gegebenenfalls eine Methylengruppe durch Sauerstoff oder Schwefel ersetzt ist,
R¹³ für Wasserstoff oder jeweils gegebenenfalls durch Fluor oder Chlor substituiertes C₁-C₆-Alkyl oder C₁-C₆-Alkoxy, für gegebenenfalls durch Fluor, C₁-C₂-Alkyl oder C₁-C₂-Alkoxy substituiertes C₃-C₇₋Cycloalkyl, in welchem gegebenenfalls eine Methylengruppe durch Sauerstoff oder Schwefel ersetzt ist oder für jeweils gegebenenfalls durch Fluor, Chlor, Brom, C₁-C₅-Alkyl, C₁-C₅-Alkoxy, C₁-C₂₋Halogenalkyl, C₁-C₂-Halogenalkoxy, Nitro oder Cyano substituiertes Phenyl, Phenyl-C₁-C₃-alkyl oder Phenyl-C₁-C₂-alkyloxy steht,
R¹⁴ Für Wasserstoff oder C₁-C₆-alkyl steht oder
R¹³ und R¹⁴ gemeinsam für C₄-C₆-Alkandiyl stehen,
R¹⁵ und R¹⁶ gleich oder verschieden sind und für C₁-C₄-Alkyl stehen oder
R¹⁵ und R¹⁶ zusammen für einen C₂-C₃-Alkandiylrest stehen, der gegebenenfalls durch C₁-C₄-Alkyl oder durch gegebenenfalls durch Fluor, Chlor, Brom, C₁-C₂-Alkyl, C₁-C₂-Halogenalkyl, C₁-C₂-Alkoxy, C₁-C₂-Halogenalkoxy, Nitro oder Cyano substituiertes Phenyl substituiert ist,

5. Verbindungen der Formel (I) gemäß Anspruch 1, worin
V für Wasserstoff, Fluor, Chlor, Brom, Methyl, Ethyl, Propyl, iso-Propyl, tert.-Butyl, Methoxy, Ethoxy, Pröpoxy oder iso-Propoxy steht,
w für Cyano, Nitro, Fluor, Chlor, Brom, Methyl, Ethyl, Propyl, Butyl, iso-Propyl, iso-Butyl, tert.-Butyl, Methoxy, Ethoxy, Propoxy, iso-Propoxy, Trifluormethyl, Difluormethoxy, Trifluormethoxy, Phenyl oder Benzyloxy steht,
X für Wasserstoff, Fluor, Chlor, Brom, Methyl, Ethyl, Propyl, Butyl, iso-Butyl, iso-Propyl, tert.-Butyl, Methoxy, Ethoxy, Propoxy, iso-Propoxy, Trifluormethyl, Trifluormethoxy, Difluormethoxy, Cyano, Nitro, Phenyl oder Benzyloxy steht,
Y für Wasserstoff, Fluor, Chlor, Brom, Methyl, Ethyl, Propyl, iso-Propyl, Butyl, iso-Butyl, tert.-Butyl, Methoxy, Ethoxy, Propoxy, iso-Propoxy, Trifluormethyl, Trifluormethoxy, Difluormethoxy, Cyano oder Nitro steht,
Z für Fluor, Chlor, Brom, Methyl, Ethyl, Propyl, Butyl, iso-Butyl, iso-Propyl, tert.-Butyl, Methoxy, Ethoxy, Propoxy, iso-Propoxy, Trifluormethyl, Trifluormethoxy, Difluormethoxy, Cyano oder Nitro steht, oder
Y und Z gemeinsam für gegebenenfalls durch Fluor, Chlor, Methyl, Ethyl, Propyl, iso-Propyl, Methoxy, Ethoxy, Propoxy, iso-Propoxy oder Trifluormethyl substituiertes C₃-C₄-Alkandiyl stehen, in welchem gegebenenfalls zwei nicht direkt benachbarte Glieder durch Sauerstoff ersetzt sind, oder
W und Z gemeinsam für gegebenenfalls durch Fluor, Chlor, Methyl, Ethyl, Propyl, iso-Propyl, Methoxy, Ethoxy, Propoxy, iso-Propoxy oder Trifluormethyl substituiertes C₃-C₄-Alkandiyl stehen, in welchem gegebenenfalls zwei nicht direkt benachbarte Glieder durch Sauerstoff ersetzt sind,
Het für die Gruppe 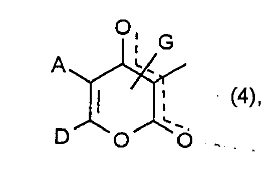 steht,
A für Wasserstoff, für jeweils gegebenenfalls durch Fluor oder Chlor substituiertes C₁-C₈-Alkyl, C₂-C₄-Alkenyl, C₁-C₆-Alkoxy-C₁-C₄₋alkyl, Poly-C₁-C₄-alkoxy-C₁-C₄-alkyl oder C₁-C₆-Alkylthio-C₁-C₄₋alkyl oder für gegebenenfalls durch Fluor, Chlor, Methyl oder Methoxy substituiertes C₃-C₆-Cycloalkyl, in welchem gegebenenfalls eine oder zwei nicht direkt benachbarte Methylengruppen durch Sauerstoff und/oder Schwefel ersetzt sind oder für jeweils gegebenenfalls durch Fluor, Chlor, Brom, Methyl, Ethyl, n-Propyl, iso-Propyl, Methoxy, Ethoxy, Trifluormethyl, Trifluormethoxy, Cyano oder Nitro substituiertes Phenyl, Pyridyl oder Benzyl steht,
D für Wasserstoff, für jeweils gegebenenfalls durch Fluor oder Chlor substituiertes C₁-C₈-Alkyl, C₃-C₄-Alkenyl, C₃-C₄-Alkinyl, C₁-C₆₋Alkoxy-C₂-C₄-alkyl, Poly-C₁-C₄-alkoxy-C₂-C₄-alkyl, C₁-C₄-Alkylthio-C₂-C₄-alkyl oder C₃-C₆-Cycloalkyl, in welchem gegebenenfalls eine oder zwei nicht direkt benachbarte Methylengruppen durch Sauerstoff und/oder Schwefel ersetzt sind oder für jeweils gegebenenfalls durch Fluor, Chlor, Brom, Methyl, Ethyl, n-Propyl, iso-Propyl, Methoxy, Ethoxy, Trifluormethyl, Trifluormethoxy, Cyano oder Nitro substituiertes Phenyl, Furanyl, Pyridyl, Thienyl oder Benzyl steht,
oder
A und D gemeinsam für eine C₃-C₅-Alkandiyl- oder C₃-C₅-Alkendiylgruppe stehen, worin jeweils gegebenenfalls eine Methylengruppe durch Sauerstoff oder Schwefel ersetzt ist und welche gegebenenfalls durch Fluor, Chlor oder durch jeweils gegebenenfalls durch Fluor oder Chlor substituiertes C₁-C₆-Alkyl oder C₁-C₄-Alkoxy substituiert sind,
G für Wasserstoff (a) oder für eine der Gruppen 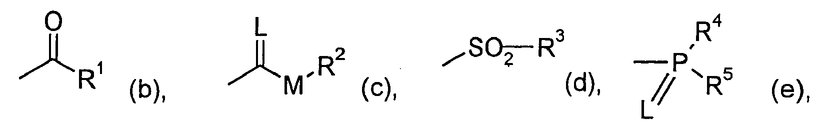 E (f) oder 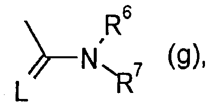 steht, in welchen
E für ein Metallionäquivalent oder ein Ammoniumion steht,
L für Sauerstoff oder Schwefel steht und
M für Sauerstoff oder Schwefel steht.
R¹ für jeweils gegebenenfalls durch Fluor oder Chlor substituiertes C₁-C₁₄-Alkyl, C₂-C₁₄-Alkenyl, C₁-C₄-Alkoxy-C₁-C₆-alkyl, C₁-C₄₋Alkylthio-C₁-C₆-alkyl, Poly-C₁-C₄-alkoxy-C₁-C₄-alkyl oder für gegebenenfalls durch Fluor, Chlor, Methyl, Ethyl, n-Propyl, i-Propyl, n-Butyl, i-Butyl, tert.-Butyl, Methoxy, Ethoxy, n-Propoxy oder iso-Propoxy substituiertes C₃-C₆-Cycloalkyl, in welchem gegebenenfalls eine oder zwei nicht direkt benachbarte Methylengruppen durch Sauerstoff und/oder Schwefel ersetzt sind,
für gegebenenfalls durch Fluor, Chlor, Brom, Cyano, Nitro, Methyl, Ethyl, n-Propyl, i-Propyl, Methoxy, Ethoxy, Trifluormethyl, Trifluormethoxy, Methylthio, Ethylthio, Methylsulfonyl oder Ethylsulfonyl substituiertes Phenyl,
für gegebenenfalls durch Fluor, Chlor, Brom, Methyl, Ethyl, n-Propyl, i-Propyl, Methoxy, Ethoxy, Trifluormethyl oder Trifluormethoxy substituiertes Benzyl,
für jeweils gegebenenfalls durch Fluor, Chlor, Brom, Methyl oder Ethyl substituiertes Furanyl, Thienyl oder Pyridyl,
für gegebenenfalls durch Fluor, Chlor, Methyl oder Ethyl substituiertes Phenoxy-C₁-C₄-alkyl oder
für jeweils gegebenenfalls durch Fluor, Chlor, Amino, Methyl oder Ethyl substituiertes Pyridyloxy-C₁-C₄-alkyl, Pyrimidyloxy-C₁-C₄₋alkyl oder Thiazolyloxy-C₁-C₄-alkyl steht
R² für jeweils gegebenenfalls durch Fluor oder Chlor substituiertes C₁-C₁₄-Alkyl, C₂-C₁₄-Alkenyl, C₁-C₄-Alkoxy-C₂-C₆-alkyl oder Poly-C₁-C₄-alkoxy-C₂-C₆-alkyl,
für gegebenenfalls durch Fluor, Chlor, Methyl, Ethyl, n-Propyl, iso-Propyl oder Methoxy substituiertes C₃-C₆-Cycloalkyl,
oder für jeweils gegebenenfalls durch Fluor, Chlor, Cyano, Nitro, Methyl, Ethyl, n-Propyl, i-Propyl, Methoxy, Ethoxy, Trifluormethyl oder Trifluormethoxy substituiertes Phenyl oder Benzyl steht,
R³ für gegebenenfalls durch Fluor oder Chlor substituiertes Methyl, Ethyl, Propyl, iso-Propyl, Butyl, tert.-Butyl, oder jeweils gegebenenfalls durch Fluor, Chlor, Brom, Methyl, Ethyl, iso-Propyl, tert.-Butyl, Methoxy, Ethoxy, iso-Propoxy, Trifluormethyl, Trifluormethoxy, Cyano oder Nitro substituiertes Phenyl oder Benzyl steht,
R⁴ und R⁵ unabhängig voneinander für jeweils gegebenenfalls durch Fluor oder Chlor substituiertes C₁-C₄-Alkyl, C₁-C₄-Alkoxy, C₁-C₄-Alkylamino, Di-(C₁-C₄-alkyl)amino oder C₁-C₄-Alkylthio oder für jeweils gegebenenfalls durch Fluor, Chlor, Brom, Nitro, Cyano, Methyl, Methoxy, Trifluormethyl oder Trifluormethoxy substituiertes Phenyl, Phenoxy oder Phenylthio stehen,
R⁶ und R⁷ unabhängig voneinander für Wasserstoff, für jeweils gegebenenfalls durch Fluor oder Chlor substituiertes C₁-C₄-Alkyl, C₃-C₆₋Cycloalkyl, C₁-C₄-Alkoxy, C₃-C₄-Alkenyl oder C₁-C₄-Alkoxy-C₂₋C₄-alkyl, für jeweils gegebenenfalls durch Fluor, Chlor, Brom, Methyl, Methoxy oder Trifluormethyl substituiertes Phenyl oder Benzyl, oder zusammen für einen gegebenenfalls durch Methyl oder Ethyl substituierten C₅-C₆-Alkylenrest stehen, in welchem gegebenenfalls eine Methylengruppe durch Sauerstoff oder Schwefel ersetzt ist, wobei

6. Verbindungen der Formel (VI) 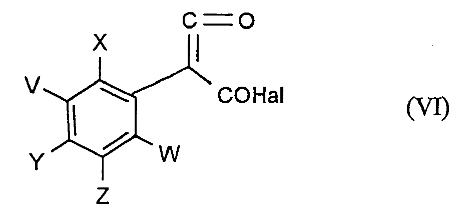 in welcher
V, W, X, Y und Z die in Anspruch 1 angegebenen Bedeutungen haben und
Hal für Chlor oder Brom steht.

7. Verbindungen der Formel (XXXIII) 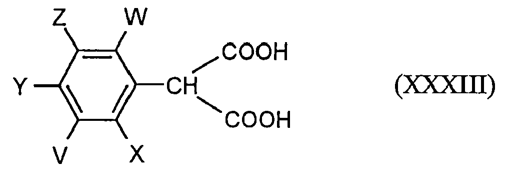 in welcher
V, W, X, Y und Z die in Anspruch 1 angegebenen Bedeutungen haben.

8. Verbindungen der Formel (XXXIV) 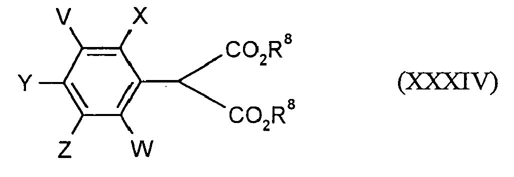 in welcher
V, W, X, Y und Z die in Anspruch 1 angegebenen Bedeutungen haben und
R⁸ für Alkyl steht.

9. Schädlingsbekämpfungsmittel oder herbizide Mittel, **gekennzeichnet durch** einen Gehalt an einer Verbindung der Formel (I) gemäß Anspruch 1.

10. Verwendung von Verbindungen der Formel (I) gemäß Anspruch 1 zur Bekämpfung von Schädlingen und Unkräutern.

11. Verfahren zur Bekämpfung von Schädlingen und Unkräutern, **dadurch gekennzeichnet, daß** man Verbindungen der Formel (I) gemäß Anspruch 1 auf Schädlinge bzw. Unkräuter und/oder ihren Lebensraum einwirken läßt.

12. Verfahren zur Herstellung von Schädlingsbekämpfungsmitteln und herbiziden Mitteln, **dadurch gekennzeichnet, daß** man Verbindungen der Formel (I) gemäß Anspruch 1 mit Streckmitteln und/oder oberflächenaktiven Mitteln vermischt.
